# EUROPEAN PATENT SPECIFICATION

(11) **EP 4 326 262 B1**
(45) Date of publication and mention of the grant of the patent: **14.10.2026**
(21) Application number: 22723682.5
(22) Date of filing: 21.04.2022
(51) Int. Cl.: A61K 31/4178, A61K 31/426, A61K 31/429, A61P 35/00

(54) **COMPOUNDS FOR USE IN THE TREATMENT OF HYPERPROLIFERATIVE DISORDERS**
VERBINDUNGEN ZUR VERWENDUNG BEI DER BEHANDLUNG VON HYPERPROLIFERATIVEN STÖRUNGEN
COMPOSÉS DESTINÉS À ÊTRE UTILISÉS DANS LE TRAITEMENT DE TROUBLES HYPERPROLIFÉRANTS

(30) Priority: 21.04.2021 EP 21382343
(43) Date of publication of application: 28.02.2024
(73) Proprietor: Fundació Privada Institut d'Investigació Oncològica de Vall Hebron, 08035 Barcelona (ES); Universitat de Barcelona, 08028 Barcelona (ES); Institució Catalana de Recerca i Estudis Avançats, 08010 Barcelona (ES)
(72) Inventor: GARCÍA PALMER, Héctor, 08035 Barcelona (ES); PUIG BORREIL, Isabel, 08035 Barcelona (ES); TABERNERO CATURLA, Josep, 08035 Barcelona (ES); GALDEANO CANTADOR, Carlos, 08028 Barcelona (ES); MUÑOZ-TORRERO LÓPEZ-IBARRA, Diego, 08028 Barcelona (ES); BARRIL ALONSO, Xavier, 08028 Barcelona (ES); RUIZ CARMONA, Sergio, 08028 Barcelona (ES)
(74) Representative: ABG Intellectual Property Law, S.L.
(86) International application number: PCT/EP2022/060579
(87) International publication number: WO 2022/223704

(56) References cited:
- WO-A1-2009/002933
- CASCIUC IURI ET AL: "Pros and cons of virtual screening based on public "Big Data": In silico mining for new bromodomain inhibitors", EUROPEAN JOURNAL OF MEDICINAL CHEMISTRY, vol. 165, 1 March 2019 (2019-03-01), AMSTERDAM, NL, pages 258 - 272, XP055841354, ISSN: 0223-5234, DOI: 10.1016/j.ejmech.2019.01.010
- CASCIUC I ET AL: "Supporting Information for the paper by I. Casciuc et al. "Pros and Cons of Virtual Screening based on Public "Big Data": In Silico mining for new Bromodomain inhibitors"", EUROPEAN JOURNAL OF MEDICINAL CHEMISTRY VOLUME 165, 1 MARCH 2019, 1 March 2019 (2019-03-01), pages 1 - 10, XP055841293, Retrieved from the Internet <URL:https://www.sciencedirect.com/science/article/pii/S0223523419300169?via%3Dihub#appsec1> [retrieved on 20210915]
- DUAN YINGCHAO ET AL: "Targeting Brd4 for cancer therapy: inhibitors and degraders", MEDCHEMCOMM, vol. 9, no. 11, 1 January 2018 (2018-01-01), United Kingdom, pages 1779 - 1802, XP055842447, ISSN: 2040-2503, DOI: 10.1039/C8MD00198G

## Description

### FIELD OF THE INVENTION

The present invention relates to compounds for use in the treatment and/or prevention of hyperproliferative disorders and to a subset of said compounds as such.

### BACKGROUND OF THE INVENTION

Undesired and uncontrolled cell proliferation underlies the so-called hyperproliferative disorders and pharmacological treatment of said uncontrolled cell proliferation is desirable to prevent or treat these hyperproliferative disorders.

TET2 enzyme oxidizes 5-methylcytosine to 5-hydroxymethylcytosine and other oxidized methylcytosines in DNA. The pattern of DNA methylation at cytosine bases in the genome is tightly linked to gene expression, and DNA methylation abnormalities are often observed in hyperproliferative disorders. TET2 protein deregulation promotes various hyperproliferative disorders and cancer phenotype (cf. e.g. Rasmussen and Helin, "Role of the TET enzymes in DNA methylation, development and cancer" Genes & Dev. 2016, 30, 733, a review with 149 references).

TET2 direct or indirect modulation has been associated to various beneficial biological effects, useful in the treatment and/or prevention of hyperproliferative diseases (c.f. e.g. Bates, "Epigenetic Therapies for Cancer", N. Eng. J. Med. 2020, 383, 651) (c.f. e.g. Yue and Rao, "TET family dioxygenases and the TET activator vitamin C in immune response and cancer" Blood 2020, 136, 1394).

More specifically, the documents cited below have described the involvement of TET2 mutations in different cancers: leukaemia (Leukaemia 2012, 26, 934), lymphoma (Genome Med. 2015, 7:9), haematological cancers (Int. J. Hematol. 2017, 105, 17), myelodysplastic syndrome (Front. Oncol. 2019, 9, 210), endometrial cancer (Carcinogenesis 2014, 35, 2068), blastic plasmacytoid dentritic cell neoplasm (Br. J. Haematol. 2011, 153, 402), essential thrombocythemia (Leukaemia 2009, 23, 905), myelofibrosis (Leukaemia 2009, 23, 905), Sezary syndrome (Nat. Genet. 2015, 47, 1465), thymic carcinoma (Sci. Rep. 2014, 4:7336), pancreatic squamous cell carcinoma (Oncotarget 2017, 8, 14620).

The reduced activity of TET2 has been also described in other several cancers: sarcoma (Nat. Commun. 2013, 4, 2166), parathyroid carcinoma (Endocr. Relat. Cancer 2017, 24, 319), oral squamous carcinoma (Anticancer Res. 2013, 33, 4325), esophageal squamous carcinoma (Oncotarget 2015, 6, 23372), small intestinal neuroendocrine tumours (BMC Cancer 2018, 18:764), refractory cytopenia of childhood (Leukemia Res. 2015, 39, 1103), hepatocellular carcinoma (Clin. Epigenetics 2015, 7:98), glioblastoma (Oncotarget 2018, 9, 25922), head and neck cancer (Oncotarget 2018, 9, 24480), cervical squamous cell carcinoma (Jpn. J. Clin. Oncol. 2016, 46, 427), urothelial cancer (Epigenetics 2014, 9, 760), solid papillary breast carcinoma (Histopathology 2018, 73, 339), IDH1 and IDH2 mutant cancer (Genes & Dev. 2016, 30, 733) and melanoma (Cell 2012, 150, 1135).

The role of TET2 has also been described in other haematological hyperproliferative disorders: systemic mastocytosis *(*Leukemia 2009, 23, 900), erythropoiesis (Mol. Cel. Biol. 2014, 34, 989), granulocyte differentiation (Blood 2011, 118, 2551), clonal haematopoiesis (Blood 2017, 130, 753), polycythemia vera *(*Leukemia 2009, 23, 905), thrombocytopenia *(*Leukemia 2009, 23, 905), myelofibrosis *(*Leukemia 2009, 23, 905), aplastic anaemia (Haematologica 2015, 100:e172), refractory anaemia (Haematologica 2010, 95, 518), beta-thalassemia (Chin. J. Intern. Med. 2018, 57, 206) and idiopathic hypereosinophilia (PLoS ONE 2017, 12:e0185602).

TET2 has also a pleiotropic role in haematopoiesis, and its mutation has been described in several associated pathological processes: atherosclerosis (Science 2017, 35, 842), restenosis (Circulation 2013, 128, 2047), aneurysms (PLoS ONE 2015, 10, e0121104, hearth failure (J. Am. Coll. Cardiol. 2018, 71, 875), coronary artery disease (Nat. Commun. 2019,10, 1251) and pulmonary arterial hypertension (Circulation 2020, 141, 1986).

WO 2009/002933 A1 discloses compound of formula: 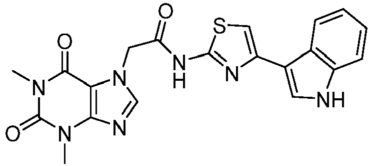 in the treatment of TRPAI mediated disorder including cancer, psoriasis and epidermal hyperplasia. Said compound does not fall within the scope of the present claims due to the presence of the 1,2,3,6-tetrahydro-7H-purine core.

Despite the importance of TET2 in hyperproliferative diseases, until now no potent compounds targeting directly TET2 have reached the market.

### SUMMARY OF THE INVENTION

The inventors have surprisingly found that certain 2-aminothiazol or 2-aminooxazol derivatives reduce undesired cell proliferation which makes these compounds suitable for the prevention and/or treatment of hyperproliferative disorders. Thus, in a first aspect, the present invention relates to compounds of formula (I) 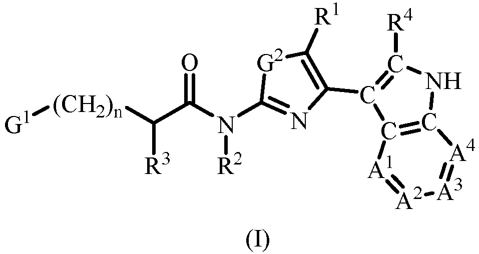 wherein
- n is 0 or 1;
- G¹ is an heteroaromatic ring system comprising a five membered ring attached to the (CH₂)ₙ group, in which the five membered ring is optionally condensed with other rings, wherein said heteroaromatic ring system may comprise 1 to 3 atoms selected from O, S and N in the ring system and said ring system is:
   - a) unsubstituted,
   - b) C-substituted with 1-3 substituents selected from the group consisting of halogen atoms, C₁₋₄-alkyl, C₁₋₄-alkoxy, C₁₋₄-alkyl-NH-, C₁₋₄-alkyl-S-, C₁₋₄-alkyl-SO-, C₁₋₄-alkyl-SO₂-, C₂₋₄-alkynyl, C₃₋₄-alkynyl-O-, C₃₋₄-alkynyl-NH-, C₃₋₄-alkynyl-S-, -OH, HO-C₁₋₄-alkyl, C₁₋₂-alkyl-O-C₁₋₄-alkyl-, -NH₂, H₂N-C₁₋₄-alkyl, C₁₋₂-alkyl-NH-C₁₋₄-alkyl-, -SH, HS-C₁₋₄-alkyl, C₁₋₂-alkyl-S-C₁₋₄-alkyl-, -CN, NC-C₁₋₄-alkyl, -COOH, HOOC-C₁₋₄-alkyl, -COO-C₁₋₂-alkyl, C₁₋₂-alkyl-OCO-C₁₋₄-alkyl-, -CONH₂, H₂NCO-C₁₋₄-alkyl, -CONH-C₁₋₂-alkyl, C₁₋₂-alkyl-NHCO-C₁₋₄-alkyl-, azido, azido-C₁₋₄-alkyl and -NO₂, or
   - c) when the ring system contains nitrogen atoms, said ring system is optionally N-substituted with 1-3 substituents selected from the group consisting of C₁₋₄-alkyl, C₁₋₄-alkyl-SO-, C₁₋₄-alkyl-SO₂-, C₃₋₄-alkynyl, HO-C₂₋₄-alkyl, C₁₋₂-alkyl-O-C₂₋₄-alkyl-, H₂N-C₂₋₄-alkyl, C₁₋₂-alkyl-NH-C₂₋₄-alkyl-, HS-C₂₋₄-alkyl, C₁₋₂-alkyl-S-C₂₋₄-alkyl-, NC-C₁₋₄-alkyl, HOOC-C₁₋₄-alkyl, C₁₋₂-alkyl-OCO-C₁₋₄-alkyl-, H₂NCO-C₁₋₄-alkyl, C₁₋₂-alkyl-NHCO-C₁₋₄-alkyl- and azido-C₂₋₄-alkyl;
      wherein when -OH, -NH₂ or -SH substituents are present on the ring system they are not vicinal to a single or double carbon-heteroatom bond;
   - G² is selected from the group consisting of O and S;
   - R¹ is selected from the group consisting of hydrogen, fluorine and C₁₋₄-alkyl groups,
   - R² and R³ are independently selected from the group consisting of hydrogen and C₁₋₄-alkyl groups,
   - R⁴ is independently selected from the group consisting of hydrogen and methyl
   - each one of A¹, A², A³ and A⁴ independently represents any one of N and CR, wherein the ring comprising A¹, A², A³ and A⁴ is aromatic,
   - R is selected from the group consisting of hydrogen, halogen atoms, C₁₋₄-alkyl, C₁₋₄-alkoxy, C₁₋₄-alkyl-NH-, C₁₋₄-alkyl-S-, C₁₋₄-alkyl-SO-, C₁₋₄-alkyl-SO₂-, C₂₋₄-alkynyl, C₃₋₄-alkynyl-O-, C₃₋₄-alkynyl-NH-, C₃₋₄-alkynyl-S-, -OH, HO-C₁₋₄-alkyl, C₁₋₂-alkyl-O-C₁₋₄-alkyl-, -NH₂, H₂N-C₁₋₄-alkyl, C₁₋₂-alkyl-NH-C₁₋₄-alkyl-, -SH, HS-C₁₋₄-alkyl, C₁₋₂-alkyl-S-C₁₋₄-alkyl-, -CN, NC-C₁₋₄-alkyl, -COOH, HOOC-C₁₋₄-alkyl, -COO-C₁₋₂-alkyl, C₁₋₂-alkyl-OCO-C₁₋₄-alkyl-, -CONH₂, H₂NCO-C₁₋₄-alkyl, -CONH-C₁₋₂-alkyl, C₁₋₂-alkyl-NHCO-C₁₋₄-alkyl-, azido, azido-C₁₋₄-alkyl and -NO₂,
   and pharmaceutically acceptable salts thereof for use in the treatment and/or prevention of hyperproliferative disorders.

In a second aspect, the present invention relates to a subgroup of new compounds selected from the group of compounds defined in the first aspect of the invention. Said subgroup is defined by formula I' 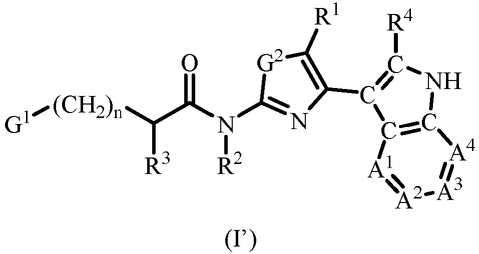 wherein
- n is 0 or 1;
- G¹ is an heteroaromatic ring system selected from the group consisting of benzo[*d*]oxazolyl, imidazo[1,2-*a*]pyridinyl, benzo[*b*]thiophenyl, benzo[*d*]imidazo[2,1-*b*]thiazolyl, 1*H*-imidazol-4-yl, indol-2-yl and benzofuran-2-yl and said ring system is:
   - a) unsubstituted,
   - b) C-substituted with 1-3 substituents selected from the group consisting of halogen atoms, C₁₋₄-alkyl, C₁₋₄-alkoxy, C₁₋₄-alkyl-NH-, C₁₋₄-alkyl-S-, C₁₋₄-alkyl-SO-, C₁₋₄-alkyl-SO₂-, C₂₋₄-alkynyl, C₃₋₄-alkynyl-O-, C₃₋₄-alkynyl-NH-, C₃₋₄-alkynyl-S-, -OH, HO-C₁₋₄-alkyl, C₁₋₂-alkyl-O-C₁₋₄-alkyl-, -NH₂, H₂N-C₁₋₄-alkyl, C₁₋₂-alkyl-NH-C₁₋₄-alkyl-, -SH, HS-C₁₋₄-alkyl, C₁₋₂-alkyl-S-C₁₋₄-alkyl-, -CN, NC-C₁₋₄-alkyl, -COOH, HOOC-C₁₋₄-alkyl, -COO-C₁₋₂-alkyl, C₁₋₂-alkyl-OCO-C₁₋₄-alkyl-, -CONH₂, H₂NCO-C₁₋₄-alkyl, -CONH-C₁₋₂-alkyl, C₁₋₂-alkyl-NHCO-C₁₋₄-alkyl-, azido, azido-C₁₋₄-alkyl and -NO₂;
   - when the ring system contains nitrogen atoms, said ring system is optionally N-substituted with 1 substituent selected from the group consisting of C₁₋₄-alkyl, C₁₋₄-alkyl-SO-, C₁₋₄-alkyl-SO₂-, C₃₋₄-alkynyl, HO-C₂₋₄-alkyl, C₁₋₂-alkyl-O-C₂₋₄-alkyl-, H₂N-C₂₋₄-alkyl, C₁₋₂-alkyl-NH-C₂₋₄-alkyl-, HS-C₂₋₄-alkyl, C₁₋₂-alkyl-S-C₂₋₄-alkyl-, NC-C₁₋₄-alkyl, HOOC-C₁₋₄-alkyl, C₁₋₂-alkyl-OCO-C₁₋₄-alkyl-, H₂NCO-C₁₋₄-alkyl, C₁₋₂-alkyl-NHCO-C₁₋₄-alkyl- and azido-C₂₋₄-alkyl;

wherein when -OH, -NH₂ or -SH substituents are present on the ring system they are not vicinal to a single or double carbon-heteroatom bond;
   - G² is selected from the group consisting of O and S;
   - R¹ is selected from the group consisting of hydrogen, fluorine and C₁₋₄-alkyl groups,
   - R² and R³ are independently selected from the group consisting of hydrogen and C₁₋₄ alkyl groups,
   - R⁴ is independently selected from the group consisting of hydrogen and methyl
   - each one of A¹, A², A³ and A⁴ independently represents any one of N and CR, wherein the ring comprising A¹, A², A³ and A⁴ is aromatic,
   - R is selected from the group consisting of hydrogen, halogen atoms, C₁₋₄-alkyl, C₁₋₄-alkoxy, C₁₋₄-alkyl-NH-, C₁₋₄-alkyl-S-, C₁₋₄-alkyl-SO-, C₁₋₄-alkyl-SO₂-, C₂₋₄-alkynyl, C₃₋₄-alkynyl-O-, C₃₋₄-alkynyl-NH-, C₃₋₄-alkynyl-S-, -OH, HO-C₁₋₄-alkyl, C₁₋₂-alkyl-O-C₁₋₄-alkyl-, -NH₂, H₂N-C₁₋₄-alkyl, C₁₋₂-alkyl-NH-C₁₋₄-alkyl-, -SH, HS-C₁₋₄-alkyl, C₁₋₂-alkyl-S-C₁₋₄-alkyl-, -CN, NC-C₁₋₄-alkyl, -COOH, HOOC-C₁₋₄-alkyl, -COO-C₁₋₂-alkyl, C₁₋₂-alkyl-OCO-C₁₋₄-alkyl-, -CONH₂, H₂NCO-C₁₋₄-alkyl, -CONH-C₁₋₂-alkyl, C₁₋₂-alkyl-NHCO-C₁₋₄-alkyl-, azido, azido-C₁₋₄-alkyl and -NO₂,
and pharmaceutically acceptable salts thereof.

In a third aspect, the present invention relates to a pharmaceutical composition comprising a compound of formula (I') and at least one pharmaceutically acceptable excipient for use in the treatment and/or prevention of hyperproliferative disorders.

### DESCRIPTION OF THE INVENTION

In a first aspect, the present invention relates to compounds of formula (I) 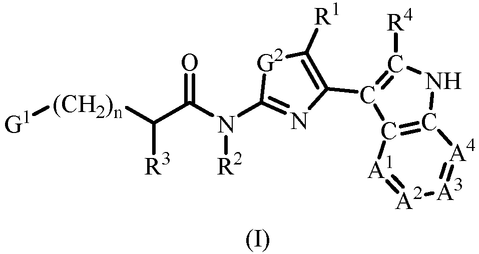 wherein
- n is 0 or 1;
- G¹ is an heteroaromatic ring system comprising a five membered ring attached to the (CH₂)ₙ group, in which the five membered ring is optionally condensed with other rings, wherein said heteroaromatic ring system may comprise 1 to 3 atoms selected from O, S and N in the ring system and said ring system is:
   - a) unsubstituted,
   - b) C-substituted with 1-3 substituents selected from the group consisting of halogen atoms, C₁₋₄-alkyl, C₁₋₄-alkoxy, C₁₋₄-alkyl-NH-, C₁₋₄-alkyl-S-, C₁₋₄-alkyl-SO-, C₁₋₄-alkyl-SO₂-, C₂₋₄-alkynyl, C₃₋₄-alkynyl-O-, C₃₋₄-alkynyl-NH-, C₃₋₄-alkynyl-S-, -OH, HO-C₁₋₄-alkyl, C₁₋₂-alkyl-O-C₁₋₄-alkyl-, -NH₂, H₂N-C₁₋₄-alkyl, C₁₋₂-alkyl-NH-C₁₋₄-alkyl-, -SH, HS-C₁₋₄-alkyl, C₁₋₂-alkyl-S-C₁₋₄-alkyl-, -CN, NC-C₁₋₄-alkyl, -COOH, HOOC-C₁₋₄-alkyl, -COO-C₁₋₂-alkyl, C₁₋₂-alkyl-OCO-C₁₋₄-alkyl-, -CONH₂, H₂NCO-C₁₋₄-alkyl, -CONH-C₁₋₂-alkyl, C₁₋₂-alkyl-NHCO-C₁₋₄-alkyl-, azido, azido-C₁₋₄-alkyl and -NO₂, or
   - c) when the ring system contains nitrogen atoms, said ring system is optionally N-substituted with 1-3 substituents selected from the group consisting of C₁₋₄-alkyl, C₁₋₄-alkyl-SO-, C₁₋₄-alkyl-SO₂-, C₃₋₄-alkynyl, HO-C₂₋₄-alkyl, C₁₋₂-alkyl-O-C₂₋₄-alkyl-, H₂N-C₂₋₄-alkyl, C₁₋₂-alkyl-NH-C₂₋₄-alkyl-, HS-C₂₋₄-alkyl, C₁₋₂-alkyl-S-C₂₋₄-alkyl-, NC-C₁₋₄-alkyl, HOOC-C₁₋₄-alkyl, C₁₋₂-alkyl-OCO-C₁₋₄-alkyl-, H₂NCO-C₁₋₄-alkyl, C₁₋₂-alkyl-NHCO-C₁₋₄-alkyl- and azido-C₂₋₄-alkyl;
      wherein when -OH, -NH₂ or -SH substituents are present on the ring system they are not vicinal to a single or double carbon-heteroatom bond;
   - G² is selected from the group consisting of O and S;
   - R¹ is selected from the group consisting of hydrogen, fluorine and C₁₋₄-alkyl groups,
   - R² and R³ are independently selected from the group consisting of hydrogen and C₁₋₄-alkyl groups,
   - R⁴ is independently selected from the group consisting of hydrogen and methyl
   - each one of A¹, A², A³ and A⁴ independently represents any one of N and CR, wherein the ring comprising A¹, A², A³ and A⁴ is aromatic,
   - R is selected from the group consisting of hydrogen, halogen atoms, C₁₋₄-alkyl, C₁₋₄-alkoxy, C₁₋₄-alkyl-NH-, C₁₋₄-alkyl-S-, C₁₋₄-alkyl-SO-, C₁₋₄-alkyl-SO₂-, C₂₋₄-alkynyl, C₃₋₄-alkynyl-O-, C₃₋₄-alkynyl-NH-, C₃₋₄-alkynyl-S-, -OH, HO-C₁₋₄-alkyl, C₁₋₂-alkyl-O-C₁₋₄-alkyl-, -NH₂, H₂N-C₁₋₄-alkyl, C₁₋₂-alkyl-NH-C₁₋₄-alkyl-, -SH, HS-C₁₋₄-alkyl, C₁₋₂-alkyl-S-C₁₋₄-alkyl-, -CN, NC-C₁₋₄-alkyl, -COOH, HOOC-C₁₋₄-alkyl, -COO-C₁₋₂-alkyl, C₁₋₂-alkyl-OCO-C₁₋₄-alkyl-, -CONH₂, H₂NCO-C₁₋₄-alkyl, -CONH-C₁₋₂-alkyl, C₁₋₂-alkyl-NHCO-C₁₋₄-alkyl-, azido, azido-C₁₋₄-alkyl and -NO₂,
and pharmaceutically acceptable salts thereof for use in the treatment and/or prevention of hyperproliferative disorders.

In a particular embodiment of the first, second and third aspects of the invention in the compound for use of formula (I) R¹ is selected from the group consisting of hydrogen, fluorine and C₁₋₄-alkyl groups, preferably selected from the group consisting of hydrogen and C₁₋₄-alkyl groups.

In a particular embodiment of the first aspect of the invention in the compound for use of formula (I) G¹ is selected to be an optionally substituted monocyclic, bicyclic or tricyclic heteroaromatic ring system, preferably a monocyclic or bicyclic ring system.

In a particular embodiment of the first aspect of the invention in the compound for use of formula (I) G¹ is an optionally substituted heteroaromatic ring system comprising a five membered ring containing 1 or 2 heteroatoms selected from N, S and O optionally fused to another ring. The heteroaromatic ring system may be monocyclic, bicyclic or tricyclic.

In a particular embodiment of the first aspect of the invention in the compound for use of formula (I) the five-membered ring of G¹ which is attached to the (CH₂)ₙ group contains 1 or 2 heteroatoms selected from N, S and O.

In another embodiment of the first aspect of the invention in the compound for use of formula (I) G¹ is selected from the group consisting of 1H-indol-3-yl, imidazo[2,1-b]thiazol-6-yl, imidazo[1,2-a]pyridin-2-yl, 1H-indol-2-yl, benzo[d]oxazol-2-yl, imidazo[2,1-b]thiazol-3-yl, benzo[b]thiophen-2-yl, thiophen-2-yl, benzo[b]furan-2-yl, 1H-imidazol-4-yl, all of which may be optionally substituted as defined in the first aspect.

In still another embodiment of the first aspect of the invention in the compound for use of formula (I) G² is S.

In still another embodiment of the first aspect of the invention in the compound for use of formula (I) R¹ is selected from hydrogen atom and methyl.

In still another embodiment of the first aspect of the invention in the compound for use of formula (I) R² and R³ are independently selected from hydrogen atom and methyl.

In still another embodiment of the first aspect of the invention in the compound for use of formula (I) A², A³ and A⁴ are CR.

In still another embodiment of the first aspect of the invention the compound for use of formula (I) is selected from the group consisting of:
- 2-imidazo[2,1-*b*]thiazol-6-yl-*N*-[4-(2-methyl-1*H*-indol-3-yl)thiazol-2-yl]acetamide
- 2-(imidazo[2,1-*b*]thiazol-3-yl)-*N*-[4-(2-methyl-1*H*-indol-3-yl)thiazol-2-yl]acetamide
- 2-(benzo[*d*]oxazol-2-yl)-*N*-[4-(2-methyl-1*H*-indol-3-yl)thiazol-2-yl]acetamide
- 2-(imidazo[2,1-*b*]thiazol-6-yl)-*N*-[5-methyl-4-(2-methyl-1*H*-indol-3-yl)thiazol-2-yl]acetamide
- 2-(imidazo[2,1-*b*]thiazol-6-yl)-*N*-[4-(2-methyl-1*H*-indol-3-yl)oxazol-2-yl]acetamide
- 2-(5-methoxy-1*H*-indol-3-yl)-*N*-[4-(2-methyl-1*H*-indol-3-yl)thiazol-2-yl]acetamide
- 2-(1*H*-indol-2-yl)-*N*-[4-(2-methyl-1*H*-indol-3-yl)thiazol-2-yl]acetamide
- 2-(6-fluoroimidazo[1,2-*a*]pyridin-2-yl)-N-[4-(2-methyl-1*H*-indol-3-yl)thiazol-2-yl]acetamide
- *N*-[4-(2-methyl-1*H*-indol-3-yl)thiazol-2-yl]-2-(2-methylimidazo[2,1-*b*]thiazol-6-yl)acetamide
- *N*-[4-(2-methyl-1*H*-indol-3-yl)thiazol-2-yl]-2-(3-methylimidazo[2,1-*b*]thiazol-6-yl)acetamide
- 2-(1-methyl-1*H*-indol-3-yl)-*N*-[4-(2-methyl-1*H*-indol-3-yl)thiazol-2-yl]acetamide
- 2-(5-fluoro-1*H*-indol-3-yl)-*N*-[4-(2-methyl-1*H*-indol-3-yl)thiazol-2-yl]acetamide
- 2-(1*H*-indol-3-yl)-*N*-[4-(2-methyl-1*H*-indol-3-yl)thiazol-2-yl]acetamide
- 3-(1*H*-indol-3-yl)-*N*-[4-(2-methyl-1*H*-indol-3-yl)thiazol-2-yl]propanamide
- 2-(imidazo[2,1-*b*]thiazol-6-yl)-*N*-methyl-*N*-[4-(2-methyl-1*H*-indol-3-yl)thiazol-2-yl]propanamide
- 2-(6-chloro-1*H*-indol-3-yl)-*N*-[4-(2-methyl-1*H*-indol-3-yl)thiazol-2-yl]acetamide
- 2-(1*H*-imidazol-4-yl)-*N*-[4-(2-methyl-1*H*-indol-3-yl)thiazol-2-yl]acetamide
- 2-(benzofuran-2-yl)-*N*-[4-(2-methyl-1*H*-indol-3-yl)thiazol-2-yl]acetamide
- 2-(4-bromothiophen-2-yl)-*N*-[4-(2-methyl-1*H*-indol-3-yl)thiazol-2-yl]acetamide
- *N*-[4-(2-methyl-1*H*-indol-3-yl)thiazol-2-yl]-2-(3-methylbenzo[*b*]thiophen-2-yl)acetamide
- 2-(5-hydroxy-1*H*-indol-3-yl)-*N*-[4-(2-methyl-1*H*-indol-3-yl)thiazol-2-yl]acetamide
- *N*-[4-(4-bromo-1*H*-indol-3-yl)thiazol-2-yl]-2-(imidazo[2,1-*b*]thiazol-6-yl)acetamide
- 2-(imidazo[2,1-*b*]thiazol-6-yl)-*N*-[4-(5-nitro-1*H*-indol-3-yl)thiazol-2-yl]acetamide
- 2-(imidazo[2,1-*b*]thiazol-6-yl)-*N*-[4-(5-methyl-1*H*-indol-3-yl)thiazol-2-yl]acetamide
- 2-(imidazo[2,1-*b*]thiazol-6-yl)-*N*-[4-(5-methoxy-2-methyl-1*H*-indol-3-yl)thiazol-2-yl]acetamide
- *N*-[4-(6-fluoro-2-methyl-1*H*-indol-3-yl)thiazol-2-yl]-2-(imidazo[2,1-*b*]thiazol-6-yl)acetamide
- 2-(imidazo[2,1-*b*]thiazol-6-yl)-*N*-[4-(2-methyl-5-nitro-1*H*-indol-3-yl)thiazol-2-yl]acetamide
- *N*-[4-(5-amino-2-methyl-1*H*-indol-3-yl)thiazol-2-yl]-2-(imidazo[2,1-*b*]thiazol-6-yl)acetamide
- *N*-[5-fluoro-4-(2-methyl-1*H*-indol-3-yl)thiazol-2-yl]-2-(imidazo[2,1-*b*]thiazol-6-yl)acetamide
and pharmaceutically acceptable salts thereof.

In a second aspect, the present invention relates to a subgroup of new compounds selected from the group of compounds defined in the first aspect of the invention. Said subgroup is defined by formula (I') 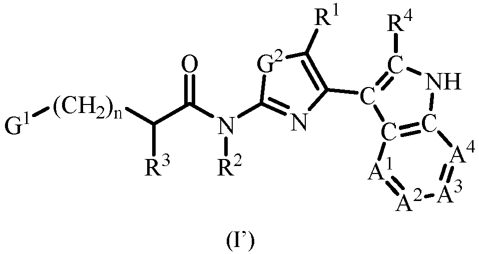 wherein
- n is 0 or 1;
- G¹ is an heteroaromatic ring system selected from the group consisting of benzo[*d*]oxazolyl, imidazo[1,2-*a*]pyridinyl, benzo[*b*]thiophenyl, benzo[*d*]imidazo[2,1-*b*]thiazolyl, 1*H*-imidazol-4-yl, indol-2-yl and benzofuran-2-yl and said ring system is:
   - a) unsubstituted,
   - b) C-substituted with 1-3 substituents selected from the group consisting of halogen atoms, C₁₋₄-alkyl, C₁₋₄-alkoxy, C₁₋₄-alkyl-NH-, C₁₋₄-alkyl-S-, C₁₋₄-alkyl-SO-, C₁₋₄-alkyl-SO₂-, C₂₋₄-alkynyl, C₃₋₄-alkynyl-O-, C₃₋₄-alkynyl-NH-, C₃₋₄-alkynyl-S-, -OH, HO-C₁₋₄-alkyl, C₁₋₂-alkyl-O-C₁₋₄-alkyl-, -NH₂, H₂N-C₁₋₄-alkyl, C₁₋₂-alkyl-NH-C₁₋₄-alkyl-, -SH, HS-C₁₋₄-alkyl, C₁₋₂-alkyl-S-C₁₋₄-alkyl-, -CN, NC-C₁₋₄-alkyl, -COOH, HOOC-C₁₋₄-alkyl, -COO-C₁₋₂-alkyl, C₁₋₂-alkyl-OCO-C₁₋₄-alkyl-, -CONH₂, H₂NCO-C₁₋₄-alkyl, -CONH-C₁₋₂-alkyl, C₁₋₂-alkyl-NHCO-C₁₋₄-alkyl-, azido, azido-C₁₋₄-alkyl and -NO₂;
   - c) when the ring system contains nitrogen atoms, said ring system is optionally N-substituted with 1 substituent selected from the group consisting of C₁₋₄-alkyl, C₁₋₄-alkyl-SO-, C₁₋₄-alkyl-SO₂-, C₃₋₄-alkynyl, HO-C₂₋₄-alkyl, C₁₋₂-alkyl-O-C₂₋₄-alkyl-, H₂N-C₂₋₄-alkyl, C₁₋₂-alkyl-NH-C₂₋₄-alkyl-, HS-C₂₋₄-alkyl, C₁₋₂-alkyl-S-C₂₋₄-alkyl-, NC-C₁₋₄-alkyl, HOOC-C₁₋₄-alkyl, C₁₋₂-alkyl-OCO-C₁₋₄-alkyl-, H₂NCO-C₁₋₄-alkyl, C₁₋₂-alkyl-NHCO-C₁₋₄-alkyl- and azido-C₂₋₄-alkyl;

wherein when -OH, -NH₂ or -SH substituents are present on the ring system they are not vicinal to a single or double carbon-heteroatom bond;
   - G² is selected from the group consisting of O and S;
   - R¹ is selected from the group consisting of hydrogen, fluorine and C₁₋₄ alkyl groups,
   - R² and R³ are independently selected from the group consisting of hydrogen and C₁₋₄ alkyl groups,
   - R⁴ is independently selected from the group consisting of hydrogen and methyl
   - each one of A¹, A², A³ and A⁴ independently represents any one of N and CR, wherein the ring comprising A¹, A², A³ and A⁴ is aromatic,
   - R is selected from the group consisting of hydrogen, halogen atoms, C₁₋₄-alkyl, C₁₋₄-alkoxy, C₁₋₄-alkyl-NH-, C₁₋₄-alkyl-S-, C₁₋₄-alkyl-SO-, C₁₋₄-alkyl-SO₂-, C₂₋₄-alkynyl, C₃₋₄-alkynyl-O-, C₃₋₄-alkynyl-NH-, C₃₋₄-alkynyl-S-, -OH, HO-C₁₋₄-alkyl, C₁₋₂-alkyl-O-C₁₋₄-alkyl-, -NH₂, H₂N-C₁₋₄-alkyl, C₁₋₂-alkyl-NH-C₁₋₄-alkyl-, -SH, HS-C₁₋₄-alkyl, C₁₋₂-alkyl-S-C₁₋₄-alkyl-, -CN, NC-C₁₋₄-alkyl, -COOH, HOOC-C₁₋₄-alkyl, -COO-C₁₋₂-alkyl, C₁₋₂-alkyl-OCO-C₁₋₄-alkyl-, -CONH₂, H₂NCO-C₁₋₄-alkyl, -CONH-C₁₋₂-alkyl, C₁₋₂-alkyl-NHCO-C₁₋₄-alkyl-, azido, azido-C₁₋₄-alkyl and -NO₂,
and pharmaceutically acceptable salts thereof.

As used herein the term heteroaromatic ring system is used to designate a ring system comprising one or more fused rings which ring system is aromatic and contains at least one heteroatom as part of at least one of the rings. The ring system may comprise one ring (monocyclic ring system), two rings (bicyclic ring system), three rings (tricyclic ring system) or more than three rings (polycyclic ring system). The carbon atoms of the rings may be unsubstituted or substituted. The heteroaromatic ring system of G¹ comprises at least one five membered ring which is attached to the (CH₂)ₙ group of the compounds of formulae (I) and (I'). The heteroaromatic ring system comprises C, O, S and/or N atoms as part of the rings.

As used herein the term alkyl is used to designate linear or branched hydrocarbon radicals (CₙH₂ₙ₊₁). Examples include methyl, ethyl, *n*-propyl, isopropyl, *n*-butyl, *sec-*butyl, *tert*-butyl, *n*-pentyl, 2-pentyl, 2-methylbutyl, isopentyl, 3-pentyl, 1-methyl-2-butyl, 3-methyl-2-butyl, *n*-hexyl, 3-hexyl, 2-ethylbutyl, 2-methyl-2-pentyl, 3-methyl-2-pentyl, 4-methyl-2-pentyl, 2,2-dimethylbutyl, 2,3-dimethylbutyl, 2-methyl-1-pentyl and 3-methyl-1-pentyl. In a preferred embodiment said alkyl groups have 1 to 4 carbon atoms, most preferably 1 to 2 carbons atoms, most preferably the alkyl group is methyl.

As used herein, the term C₁₋₄-alkoxy is used to designate radicals which contain a linear or branched C₁₋₄ alkyl group linked to an oxygen atom (CₙH₂ₙ₊₁-O-) wherein n ranges from 1 to 4. Preferred alkoxy radicals include methoxy, ethoxy, *n*-propoxy, *i-*propoxy, *n*-butoxy, *sec*-butoxy and *tert*-butoxy.

As used herein the term alkynyl is used to designate linear or branched hydrocarbon radicals comprising a triple bond (CₙH₂ₙ₋₃).

As used herein, the term azido is used to designate the group -N₃

As used herein, the term halogen atom is used to designate an atom selected from the group consisting of chlorine, fluorine, bromine or iodine atom, preferably bromine, fluorine or chlorine atom.

The term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

Further, the term "pharmaceutically acceptable salt" refers to any salt, which, upon administration to the recipient is capable of providing (directly or indirectly) a compound as described herein. For instance, a pharmaceutically acceptable salt of compounds provided herein may be acid addition salts, base addition salts or metallic salts, and they can be synthesized from the parent compound, which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and *p*-toluenesulphonate. Examples of the alkali addition salts include inorganic salts such as, for example, ammonium, and organic alkali salts such as, for example, ethylenediamine, ethanolamine, *N*,*N*-dialkylenethanolamine, triethanolamine, glucamine and basic amino acid salts. Examples of the metallic salts include, for example, sodium, potassium, calcium, magnesium, aluminium and lithium salts.

The term "hyperproliferative disorder" refers to a disorder involving undesired and uncontrolled cell proliferation. The hyperproliferative disorder may be benign or malignant (cancer). The term "cancer" thus refers to any malignant growth or tumour caused by abnormal and uncontrolled cell division; it may spread to other parts of the body through the lymphatic system or the blood stream and includes both solid tumours and blood-borne tumours.

Exemplary cancers include a) carcinomas (such as parathyroid carcinoma, thymic carcinoma, squamous-cell carcinoma, non-small-cell lung carcinoma, small-cell lung carcinoma, invasive ductal carcinoma, ductal carcinoma in situ, carcinoma of the prostate, adenocarcinoma, pancreatic carcinoma, colorectal carcinoma, urothelial cancer, endometrioid carcinoma, squamous carcinoma (i.e. head and neck cancer, oral squamous carcinoma (i.e. head and neck, thyroid, oesophagus, lungs, penis, prostate, vagina and cervix or bladder), hepatocellular carcinoma, solid papillary carcinoma, renal cell carcinoma and ovarian carcinoma); b) sarcomas such as bone sarcoma (i.e. osteosarcoma, chondrosarcoma, poorly differentiated round/spindle cell tumours such as Ewing sarcoma, hemangioendothelioma, angiosarcoma, fibrosarcoma, myofibrosarcoma, chordoma, adamantinoma, liposarcoma, leiomyosarcoma, malignant peripheral nerve sheath tumour, rhabdomyosarcoma, synovial sarcoma and malignant solitary fibrous tumour) and soft tissue sarcoma (i.e. liposarcoma, atypical lipomatous tumour, dermatofibrosarcoma protuberans, malignant solitary fibrous tumour, inflammatory myofibroblastic tumor, low-grade myofibroblastic sarcoma, fibrosarcoma, myxofibrosarcoma, low-grade fibromyxoid sarcoma, giant cell tumour of soft tissues, leiomyosarcoma, malignant glomus tumour, rhabdomyosarcoma, hemangioendothelioma, angiosarcoma of soft tissue, extraskeletal osteosarcoma, gastrointestinal stromal tumour, malignant (GIST), malignant peripheral nerve sheath tumour, malignant Triton tumour, malignant granular cell tumour, malignant ossifying fibromyxoid tumor, stromal sarcoma not otherwise specified, malignant phosphaturic mesenchymal tumour, synovial sarcoma, epithelioid sarcoma, alveolar soft part sarcoma, clear cell sarcoma of soft tissue, extraskeletal myxoid chondrosarcoma, extraskeletal Ewing sarcoma, desmoplastic small round cell tumour, extrarenal rhabdoid tumour, perivascular epithelioid cell tumour, not otherwise specified, intimal sarcoma, undifferentiated spindle cell sarcoma, undifferentiated pleomorphic sarcoma, undifferentiated round cell sarcoma, undifferentiated epithelioid sarcoma, and undifferentiated sarcoma, not otherwise specified); c) tumours of the hematopoietic and lymphoid tissues such as lymphoma (i.e. Hodgkin's lymphomas, non-Hodgkin's lymphomas (i.e. diffuse large B-cell lymphoma, mantle cell lymphoma, peripheral T-cell lymphoma such as Sézary disease, Burkitt lymphoma and lymphoblastic lymphoma), chronic lymphocytic leukaemia (CLL) and small lymphocytic lymphoma (SLL)), leukaemia (acute lymphoblastic leukaemia (ALL), acute myelogenous leukaemia (AML), chronic lymphocytic leukaemia (CLL), chronic neutrophilic leukaemia (CNL), chronic myeloid leukaemia (CML), primary polycythemia (such as polycythemia vera (PV)), primary myelofibrosis (PMF), essential thrombocythemia (ET), chronic eosinophilic leukaemia, blastic plasmacytoid dendritic cell neoplasm (BPDCN) and acute monocytic leukaemia (AMoL)) and myeloma; d) germ cell tumours such as germinoma (including dysgerminoma and seminoma), embryonal carcinoma, endodermal sinus tumour, also known as yolk sac tumour (EST, YST), choriocarcinoma, teratoma (including mature teratoma, dermoid cyst, immature teratoma and teratoma with malignant transformation), polyembryoma and gonadoblastoma), myelodysplastic syndrome (i.e. refractory cytopenia with unilineage dysplasia, refractory anaemia with ring sideroblasts, refractory cytopenia with multilineage dysplasia, refractory anaemias with excess blasts, refractory cytopenia of childhood and aplastic anaemia) and e) blastomas such as hepatoblastoma, medulloblastoma, nephroblastoma, neuroblastoma, pancreatoblastoma, pleuropulmonary blastoma, retinoblastoma and glioblastoma multiforme; f) melanoma and g) other various cancers such as IDH1-mutated cancers and IDH2-mutated cancers, uterine cancer such as gestational trophoblastic disease, endometrial cancer, cervical cancer and uterine sarcoma; neuroendocrine tumours such as small intestinal neuroendocrine tumour, typical pulmonary carcinoid tumour and goblet cell carcinoid; .

The term "benign hyperproliferative disorder" refers to disorders such as benign tumours, e.g. hemangiomas, hepatocellular adenoma, cavernous haemangioma, focal nodular hyperplasia, acoustic neuromas, neurofibroma, bile duct adenoma, bile duct cystanoma, fibroma, lipomas, leiomyomas, mesotheliomas, teratomas, myxomas, nodular regenerative hyperplasia, trachomas and pyogenic granulomas. Other types of non-malignant hyperproliferative disorders are abnormal cell proliferation due to insults to body tissue during surgery, proliferative responses associated with organ transplantation, abnormal angiogenesis, e.g. abnormal angiogenesis accompanying rheumatoid arthritis, ischemic-reperfusion related brain oedema and injury, cortical ischemia, ovarian hyperplasia and hypervascularity, polycystic ovary syndrome, endometriosis, psoriasis, diabetic retinopathy, and other ocular angiogenic diseases such as retinopathy of prematurity (retrolental fibroplastic), macular degeneration, corneal graft rejection, neovascular glaucoma and Oster Webber syndrome, etc.

In a preferred embodiment of the present invention, the hyperproliferative disorder is selected from the group consisting of carcinoma, sarcoma, lymphoma, leukaemia, germ cell tumour, blastoma and melanoma.

The terms "treating" and "treatment", as used herein, mean reversing, alleviating, inhibiting the progress of the disease or condition to which such term applies, or of one or more symptoms of such disease or condition, such as reducing the rate of tumoral growth.

The terms "preventing" and "prevention", as used herein, mean avoiding or inhibiting the onset of hyperproliferation.

In particular, the compounds for use according to the invention are administered as a pharmaceutical composition, which comprises the corresponding (active) compound and one or more pharmaceutically acceptable excipients.

The term "pharmaceutically acceptable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similars. Water or saline aqueous solutions and aqueous dextrose and glycerol solutions, particularly for injectable solutions, are preferably used as vehicles. Suitable pharmaceutical vehicles are known by the skilled person.

The pharmaceutically acceptable excipient necessary to manufacture the desired pharmaceutical composition of the invention will depend, among other factors, on the elected administration route. Said pharmaceutical compositions may be manufactured according to conventional methods known by the skilled person in the art.

The compounds for use according to the invention may be administered in a "therapeutically effective amount", i.e. a nontoxic but sufficient amount of the corresponding compound to provide the desired effect. The amount that is "effective" will vary from subject to subject, depending on the age and general condition of the individual, the particular compound administered, and the like. Thus, it is not always possible to specify an exact "therapeutically effective amount". However, an appropriate amount in any individual case may be determined by one of ordinary skill in the art using routine experimentation.

The compounds for use according to the invention will typically be administered once or more times a day, for example 1, 2, 3 or 4 times daily, with typical total daily doses depending on the particular compound and severity of the disease, and may be easily determined by the skilled practitioner.

By way of example, typical total daily doses of the compound of the invention are in the range from 0.1 to 2000 mg/day, preferably from 1 to 600 mg/day, even more preferably from 1 to 100 mg/day.

The pharmaceutical compositions may be prepared using standard methods such as those described or referred to in the Spanish and US Pharmacopoeias and similar reference texts.

The term "subject" refers to a mammal, e.g., a human.

The compounds for use according to the invention may be administered as the sole active ingredient or in combination with other active ingredients. In a particular embodiment, the compounds are used as the sole active ingredient. In another particular embodiment, the compounds are used in combination with other active ingredients.

### Synthesis of the compounds.

The compounds of general formula (I) of the present invention can be synthesized as described in detail below:

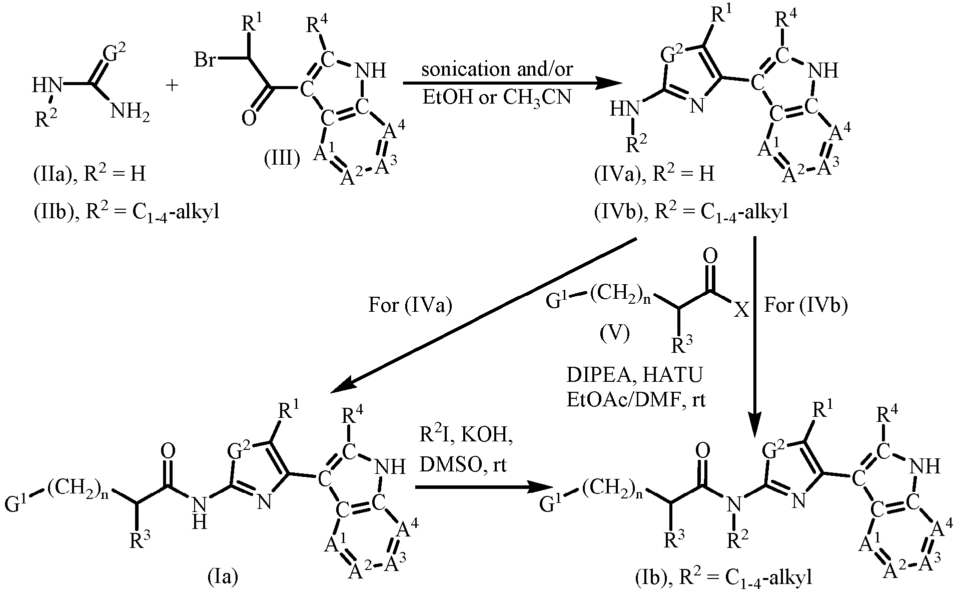

The compounds of general formula (I) can be prepared in two or three steps, starting with the reaction of compounds of formula (III) with compounds of formula (II) which can be carried out in an organic solvent, such as EtOH or acetonitrile, under ultrasound irradiation and/or by heating. Compounds of formula (I) are obtained by amide coupling of the amine of formula (IV) with a carboxylic acid of formula (V), wherein X is OH, in the presence of a base, such as DIPEA, and a coupling reagent, such as HATU, to convert the carboxylic acid into a more reactive intermediate. Alternatively, other bases and coupling reagents or derivatives of carboxylic acids such as acyl halides (formula V, with X being a halogen atom), esters (formula V, with X being an alkyloxy or aryloxy group) or acid anhydrides (formula V, with X being an OCO-alkyl, OCO-aryl, OCOO-alkyl or OCOO-aryl) can be used.

Compounds of formula (Ib) can be also prepared by reaction of compounds of formula (Ia) with a base, such as KOH, and a suitable alkylating reagent, for example an alkyl iodide. This reaction can be also used to prepare compounds of formula (Ib), wherein R² and R³ are the same C₁₋₄-alkyl, from compounds of formula (Ia), wherein R³ = H.

The compounds of formula (III) may be prepared by reaction of a compound of formula (VI), wherein G³ is CN, with a compound of formula (VII) in the presence of a Lewis acid, such as PhBCl₂, followed by an alkaline aqueous work-up, as described in the Scheme below:

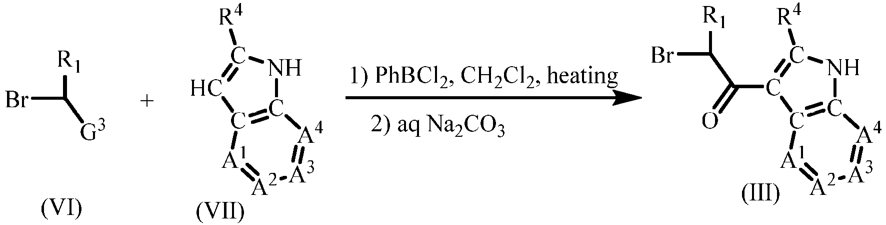

The compounds of formula (III) may be also prepared under standard Friedel-Crafts acylation conditions, i.e., by reacting compounds of formula (VII) with suitable activated α-bromo-substituted carboxylic acid derivatives of formula (VI) wherein G³ is selected from the group consisting of -COX wherein X is a halogen atom, preferably a chlorine or bromine atom, -COOCOR and -COOR wherein R is an alkyl or aryl group, in the presence of a protic or a Lewis acid catalyst.

Alternatively, compounds of formula (III) may be prepared under standard Friedel-Crafts acylation conditions, i.e., by reacting compounds of formula (VII) with suitable activated carboxylic acid derivatives of formula (VIII) wherein G³ is selected from the group consisting of -COX wherein X is a halogen atom, preferably a chlorine or bromine atom, -COOCOR and -COOR wherein R is an alkyl or aryl group, in the presence of a protic or a Lewis acid catalyst, followed by bromination at the α-carbonyl position with bromine or a suitable brominating reagent, such as copper (II) bromide or N-bromosuccinimide.

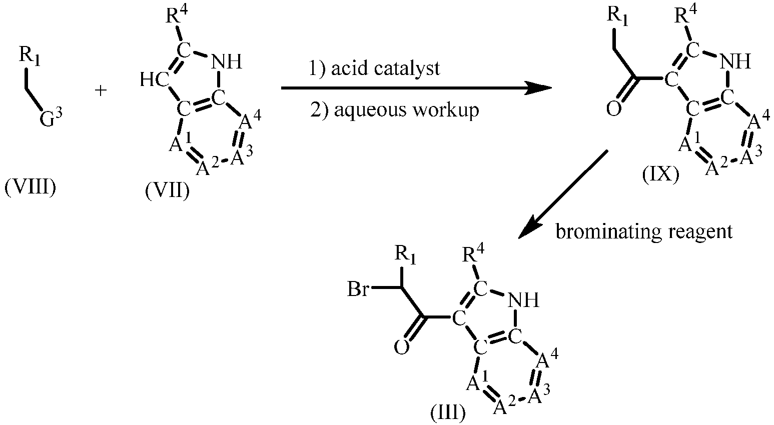

Compounds of formulae (II), (V), (VI), (VII) and (VIII) are either commercially available or can be prepared by methods known to the person skilled in the art.

It is sometimes possible to obtain compounds of formula (I) wherein one or more of A¹, A², A³ and A⁴ represents CR as defined above from another compound of formula (I) wherein R is a different group, by using standard protocols known to the person skilled in the art. For example, it is possible to convert a compound wherein one or more of A¹, A², A³ and A⁴ is C-NO₂ to a compound wherein the group C-NO₂ has been converted into a group C-NH₂. Other examples could be the conversion of a group C-COOH into a group C-COO-C₁₋₄-alkyl by esterification, the conversion of a group C-COO-C₁₋₄-alkyl into a C-COOH group by hydrolysis, the conversion of C-COO-C₁₋₄-alkyl into a different C-COO-C₁₋₄-alkyl by transesterification, the conversion of a C₁₋₄-alkoxy group into a OH group by ether cleavage, the conversion of -COOH, -COO-C₁₋₂-alkyl, -CONH₂, -CONH-C₁₋₂-alkyl, azido or azido-C₁₋₄-alkyl groups into -CH₂OH, -CH₂OH, -CH₂NH₂, -CH₂NH-C₁₋₂-alkyl, -NH₂ or H₂N-C₁₋₄-alkyl groups, respectively, by reduction.

### ABREVIATIONS:

The following abbreviations have been used along the present application:
ATR: attenuated total reflectance
br: broad
calcd: calculated
d: doublet
dd: doublet of doublets
ddd: doublet of doublet of doublets
DIPEA: *N*,*N*-diisopropylethylamine
DMF: *N*,*N*-dimethylformamide
DMSO: dimethylsulfoxide
EDTA: ethylenediaminetetraacetic acid
ESI: electrospray ionization
Et₂O: diethyl ether
EtOAc: ethyl acetate
EtOH: ethanol
FBS: fetal bovine serum
GS-linker: glycine-serine linker
HATU: 1-[bis(dimethylamino)methylene]-1*H*-1,2,3-triazolo[4,5-*b*]pyridinium 3-oxid hexafluorophosphate
HEPES: 4-(2-hydroxyethyl)-1-piperazineethanesulfonic acid
HRMS: high resolution mass spectrometry
HRP: horseradish peroxidase
IDH: isocitrate dehydrogenase
IR: infrared
LB: lysogeny broth
LC/MSD TOF: liquid chromatography-mass spectrometry with time-of-flight mass analyser
m: multiplet
MeOH: methanol
mp: melting point
NMR: nuclear magnetic resonance
PBS: phosphate-buffered saline
PhBCl₂: phenylboron dichloride
rt: room temperature
s: singlet
SDS: sodium dodecyl sulfate
t: triplet
TET2: methylcytosine dioxygenase 2
TLC: thin layer chromatography
UV: ultraviolet

The following examples represent specific embodiments of the present invention.

### EXAMPLES

### Purification of the compounds and analytical methods

Analytical grade solvents were used for crystallization, while pure for synthesis solvents were used in the reactions, extractions and column chromatography. Preparative normal phase chromatography was performed on silica gel 60 AC.C (35-70 mesh, SDS, ref 2000027) or on a CombiFlash Rf 150 (Teledyne Isco) with pre-packed RediSep Rf silica gel cartridges (automatic column chromatography). Thin-layer chromatography was performed with aluminium-backed sheets with silica gel 60 F₂₅₄ (Merck, ref 1.05554 or Sigma-Aldrich, ref 60805), and spots were visualized with UV light (at 254 or 365 nm) and developed with the following visualising agents: solution of KMnO₄/Δ, anisaldehyde/Δ or vanillin/Δ. Melting points were determined in open capillary tubes with a MFB595010M Gallenkamp melting point apparatus. Infrared (IR) spectra were run on a Perkin-Elmer Spectrum RX I spectrophotometer using the attenuated total reflectance (ATR) technique. Absorption values are expressed as wavenumbers (cm⁻¹); only significant absorption bands are given. ¹H NMR experiments were recorded using a Varian Mercury 400 spectrometer. Chemical shifts (δ) are given in ppm relative to solvent signals (DMSO-d₆: δH = 2.50 ppm; CDCl₃: δH = 7.26 ppm; MeOH-d₄: δH = 3.31 ppm) and coupling constants (*J*) are quoted in Hz. ¹H NMR spectroscopic data are reported as follows: chemical shift (multiplicity, coupling constant, number of protons). High-resolution mass spectrometry (HRMS) analyses were performed with an LC/MSD TOF Agilent Technologies spectrometer.

### Reference example 1: 4-(2-methyl-1H-indol-3-yl)thiazol-2-amine hydrobromide

This compound is used for the synthesis of examples 1, 2, 3, 6, 7, 8, 9, 10, 11, 12, 13, 14, 16, 17, 18, 19, 20, and 21, and has been prepared through the process described below.

A sealed glass tube was charged with a solution of 2-bromo-1-(2-methyl-1*H-*indol-3-yl)ethan-1-one (2.70 g, 10.7 mmol) and thiourea (815 mg, 10.7 mmol) in EtOH (21 mL). The mixture was sonicated at 60-65 °C for 1 h in an ultrasound bath. The resulting mixture was concentrated under reduced pressure, to give 4-(2-methyl-1*H-*indol-3-yl)thiazol-2-amine hydrobromide (3.67 g, quantitative yield) as a bright yellow solid, which was used without further purification; mp 155-157 °C; ¹H NMR (400 MHz, DMSO-d₆) *δ:* 2.49 (s, 3H), 3.51 (br s, 2H), 6.73 (s, 1H), 7.08 (ddd, *J = J' =* 7.6 Hz, *J" =* 0.8 Hz, 1H), 7.13 (ddd, *J = J'* = 7.6 Hz, *J"* = 0.8 Hz, 1H), 7.37 (d, *J =* 7.6 Hz, 1H), 7.54 (d, *J* = 7.6 Hz, 1H), 8.94 (br s, 1H), 11.56 (s, 1H); IR (ATR) *v*. 3018, 1450, 1306, 1254, 1223, 1172, 1099, 1008, 742, 700 cm⁻¹.

### Reference example 2: 5-methyl-4-(2-methyl-1H-indol-3-yl)thiazol-2-amine hydrobromide

This compound is used for the synthesis of example 4.

Prepared from 2-bromo-1-(2-methyl-1*H*-indol-3-yl)propan-1-one (200 mg, 0.79 mmol) and thiourea (57 mg, 0.75 mmol), and following the procedure described in reference example 1, sonicating in an ultrasound bath at 50-70 °C for 30 min, 5-methyl-4-(2-methyl-1*H*-indol-3-yl)thiazol-2-amine hydrobromide (250 mg, quantitative yield) was obtained as a yellowish solid; mp 169-172 °C; ¹H NMR (400 MHz, DMSO-d₆) *δ:* 2.10 (s, 3H), 2.35 (s, 3H), 7.05 (ddd, *J* = *J' =* 7.6 Hz, *J" =* 0.8 Hz, 1H), 7.12 (ddd, *J = J'* = 7.6 Hz, *J"* = 0.8 Hz, 1H), 7.32 (d, *J =* 7.6 Hz, 1H), 7.38 (d, *J =* 7.6 Hz, 1H), 8.94 (br s, 2H), 11.54 (s, 1H); IR (ATR) *v*. 3240, 3072, 1614, 1458, 1418, 1330, 1260, 1016, 983, 743, 582 cm⁻¹.

### Reference example 3: 4-(2-methyl-1H-indol-3-yl)oxazol-2-amine

This compound is used for the synthesis of example 5.

A solution of 2-bromo-1-(2-methyl-1*H*-indol-3-yl)ethan-1-one (200 mg, 0.79 mmol) and urea (1.43 g, 23.8 mmol) in anhydrous CH₃CN (10 mL) was heated at 100 °C for 3 days. The resulting mixture was concentrated under reduced pressure and the residue was taken up in 1 N NaOH (30 mL) and extracted with EtOAc (3 × 30 mL). The combined organic layers were dried over anhydrous Na₂SO₄, filtered, and concentrated in vacuo to yield 4-(2-methyl-1*H*-indol-3-yl)oxazol-2-amine (208 mg, quantitative yield) as a brownish solid. After washing with pentane, the analytical sample of this compound was obtained as a yellowish solid; mp 122-124 °C; ¹H NMR (400 MHz, DMSO-d₆) *δ:* 2.56 (s, 3H), 6.56 (s, 1H), 6.97 (br dd, *J = J' =* 7.6 Hz, 1H), 7.02 (br dd, *J = J' =* 7.6 Hz, 1H), 7.26 (d, *J* = 7.6 Hz, 1H), 7.56 (s, 1H), 7.76 (d, *J* = 7.6 Hz, 1H), 11.04 (s, 1H); IR (ATR) *v*. 3341, 3223, 1713, 1614, 1594, 1457, 1433, 1324, 1250, 1148, 1012, 742, 572 cm⁻¹.

### Reference example 4: 4-(4-bromo-1H-indol-3-yl)thiazol-2-amine

This compound is used for the synthesis of example 22.

To a solution of 2-bromoacetonitrile (0.10 mL, 172 mg, 1.44 mmol) in dry CH₂Cl₂ (4 mL), PhBCl₂ (0.19 mL, 233 mg, 1.46 mmol) was added under argon atmosphere and the mixture was stirred at rt for 15 min. Then, a solution of 4-bromo-1*H*-indole (0.15 mL, 234 mg, 1.20 mmol) in dry CH₂Cl₂ (2 mL) was added dropwise over 30 min. The reaction mixture was stirred at 50 °C for 20 h and was quenched by slow dropwise addition of 0.5 M Na₂CO₃ (20 mL). The organic layer was separated and the aqueous phase was extracted with EtOAc (3×25 mL). The combined organic layers were dried over anhydrous MgSO₄, filtered and concentrated under reduced pressure, to give crude 2-bromo-1-(4-bromo-1*H-*indol-3-yl)ethan-1-one (621 mg), which was used without further purification.

From crude 2-bromo-1-(4-bromo-1*H*-indol-3-yl)ethan-1-one (621 mg) and thiourea (145 mg, 1.90 mmol), following the procedure described in reference example 1, sonicating in an ultrasound bath at 60-65 °C for 45 min, a crude residue was obtained and purified by flash column chromatography [hexane:EtOAc (7:3-3:7)], to provide 4-(4-bromo-1*H*-indol-3-yl)thiazol-2-amine as a brown oil (159 mg, 45% overall); mp 124-127 °C; ¹H NMR (400 MHz, DMSO-d₆) *δ*: 6.44 (s, 1H), 6.78 (br s, 2H), 7.01 (dd, *J = J'* = 8.0 Hz, 1H), 7.21 (dd, *J =* 8.0 Hz, *J'* = 1.0 Hz, 1H), 7.43 (dd, *J* = 8.0 Hz, *J'* = 1.0 Hz, 1H), 7.48 (d, *J =* 2.4 Hz, 1H), 11.50 (br s, 1H); IR (ATR) *v.* 3436, 3361, 3110, 2920, 1600, 1512, 1414, 1331, 1308, 1186, 1005, 916, 811, 778, 743, 718 cm⁻¹; HRMS-ESI+ *m*/*z* calcd for [C₁₁H₈⁷⁹BrN₃S+H⁺]: 293.9695, found: 293.9700.

### Reference example 5: 4-(5-nitro-1H-indol-3-yl)thiazol-2-amine

This compound is used for the synthesis of example 23.

From 2-bromoacetonitrile (83.6 µL, 144 mg, 1.20 mmol), PhBCl₂ (0.19 mL, 233 mg, 1.46 mmol), and 5-nitroindole (162 mg, 1.00 mmol), following the procedure described in reference example 4, with a reaction time of 17 h, 2-bromo-1-(5-nitro-1*H-*indol-3-yl)ethan-1-one (51.0 mg, 18%) was obtained and used without further purification.

From 2-bromo-1-(5-nitro-1*H*-indol-3-yl)ethan-1-one (51.0 mg, 0.18 mmol) and thiourea (13.7 mg, 0.18 mmol), following the procedure described in reference example 1, sonicating in an ultrasound bath at 60-65 °C for 1.5 h, a crude residue was obtained and purified by flash column chromatography [CH₂Cl₂:EtOAc (1:1-1:4)], to provide 4-(5-nitro-1*H*-indol-3-yl)thiazol-2-amine as a light orange powder (32.7 mg, 70%); mp 137-140 °C; ¹H NMR (400 MHz, CDCl₃) *δ*: 4.99 (br s, 2H), 6.76 (s, 1H), 7.45 (d, *J* = 9.0 Hz, 1H), 7.75 (d, *J* = 2.0 Hz, 1H), 8.16 (dd, *J* = 9.0 Hz, *J'* = 2.0 Hz, 1H), 8.52 (br s, 1H), 9.01 (d, *J =* 2.0 Hz, 1H); IR (ATR) *v.* 3356, 3137, 2919, 2850, 1621, 1511, 1466, 1315, 1258, 1225, 1072, 840, 808, 737 cm⁻¹; MS (ESI+): 261.04 [M+H]⁺.

### Reference example 6: 4-(5-methyl-1H-indol-3-yl)thiazol-2-amine

This compound is used for the synthesis of example 24.

From 2-bromoacetonitrile (0.17 mL, 293 mg, 2.44 mmol), PhBCl₂ (0.31 mL, 379 mg, 2.39 mmol), and 5-methyl-1*H*-indole (262 mg, 2.00 mmol), following the procedure described in reference example 4, with a reaction time of 23 h, 2-bromo-1-(5-methyl-1*H-*indol-3-yl)ethan-1-one (197 mg, 39%) was obtained and used without further purification.

From 2-bromo-1-(5-methyl-1*H*-indol-3-yl)ethan-1-one (197 mg, 0.78 mmol) and thiourea (61.1 mg, 0.80 mmol), following the procedure described in reference example 1, sonicating in an ultrasound bath at 60-65 °C for 45 min, a crude residue was obtained and purified by flash column chromatography [hexane:EtOAc (1:4-1:9)], to provide 4-(5-methyl-1*H*-indol-3-yl)thiazol-2-amine (47.0 mg, 26%) as a brown oil; mp 119-121 °C; ¹H NMR (400 MHz, DMSO-d₆) *δ*: 2.41 (s, 3H), 6.65 (s, 1H), 6.89 (br s, 2H), 6.94 (dd, *J* = 8.0 Hz, *J'* = 2.0 Hz, 1H), 7.27 (d, *J =* 8.0 Hz, 1H), 7.55 (d, *J =* 2.0 Hz, 1H), 7.77 (s, 1H), 11.02 (br s, 1H); IR (ATR) *v.* 3366, 3271, 3161, 2920, 1606, 1471, 1410, 1083, 796, 718, 621 cm⁻¹; HRMS-ESI+ *m*/*z* [M+H]⁺ calcd for [C₁₂H₁₁N₃S+H⁺]: 230.0746, found: 230.0750.

### Reference example 7: 4-(5-methoxy-2-methyl-1H-indol-3-yl)thiazol-2-amine

This compound is used for the synthesis of example 25.

From 2-bromoacetonitrile (0.13 mL, 224 mg, 1.87 mmol), PhBCl₂ (0.23 mL, 282 mg, 1.77 mmol), and 5-methoxy-2-methyl-1*H*-indole (241 mg, 1.50 mmol), following the procedure described in reference example 4, with a reaction time of 15 h, 2-bromo-1-(5-methoxy-2-methyl-1*H*-indol-3-yl)ethan-1-one (173 mg, 41%) was obtained and used without further purification.

From 2-bromo-1-(5-methoxy-2-methyl-1*H*-indol-3-yl)ethan-1-one (173 mg, 0.61 mmol) and thiourea (47 mg, 0.62 mmol), following the procedure described in reference example 1, sonicating in an ultrasound bath at 60-65 °C for 1 h, a crude residue was obtained and purified by automatic column chromatography (CH₂Cl₂:MeOH mixtures), to provide 4-(5-methoxy-2-methyl-1*H*-indol-3-yl)thiazol-2-amine (60 mg, 38%) as a brownish solid; mp 152-153 °C; ¹H NMR (400 MHz, DMSO-d₆) *δ:* 2.52 (s, 3H), 3.81 (s, 3H), 6.37 (s, 1H), 6.70 (dd, *J* = 8.8 Hz, *J'* = 2.4 Hz, 1H), 7.15 (dd, *J* = 8.8 Hz, *J' =* 2.5 Hz, 1H), 7.15 (d, *J* = 8.7 Hz, 1H), 7.26 (d, *J* = 2.4 Hz, 1H); IR (ATR) *v.* 3400, 3288, 3100, 2922, 2536, 2287, 1676, 1640, 1620, 1584, 1534, 1478, 1447, 1320, 1269, 1224, 1206, 1165, 1102, 1035, 1018, 967, 898, 845, 812, 774, 733, 700, 669, 619, 591 cm⁻¹; HRMS-ESI+ *m*/*z* [M+H]⁺ calcd for [C₁₃H₁₃N₃OS+H⁺]: 260.0852, found: 260.0851.

### Reference example 8: 4-(6-fluoro-2-methyl-1H-indol-3-yl)thiazol-2-amine hydrobromide

This compound is used for the synthesis of example 26.

From 2-bromoacetonitrile (83.6 µL, 144 mg, 1.20 mmol), PhBCl₂ (156 µL, 191 mg, 1.20 mmol), and 6-fluoro-2-methyl-1*H*-indole (150 mg, 1.01 mmol), following the procedure described in reference example 4, stirring at 50 °C overnight, a crude residue was obtained and purified by automatic column chromatography (hexane:EtOAc mixtures), to provide 2-bromo-1-(6-fluoro-2-methyl-1*H*-indol-3-yl)ethan-1-one (95 mg, 35%) as a yellowish solid; mp 195-196 °C; ¹H NMR (400 MHz, CDCl₃) *δ:* 2.79 (s, 3H), 4.41 (s, 2H), 7.03-7.06 (m, 2H), 7.93 (dd, *J =* 9.6 Hz, *J'* = 4.8 Hz, 1H), 8.44 (br s, 1H); IR (ATR) *v.* 3238, 2917, 2846, 1623, 1600, 1531, 1455, 1424, 1389, 1302, 1274, 1244, 1224, 1163, 1130, 1104, 1041, 985, 957, 896, 842, 814, 786, 748, 715, 692, 647, 596 cm⁻¹; HRMS-ESI+ m/z [M+H]⁺ calcd for [C₁₁H₉⁷⁹BrFNO+H⁺]: 269.9924, found: 269.9921.

From 2-bromo-1-(6-fluoro-2-methyl-1*H*-indol-3-yl)ethan-1-one (95 mg, 0.35 mmol) and thiourea (27 mg, 0.35 mmol), following the procedure described in reference example 1, sonicating in an ultrasound bath at 60-65 °C for 1 h, 4-(6-fluoro-2-methyl-1*H*-indol-3-yl)thiazol-2-amine hydrobromide (105 mg, 91%) was obtained as a yellowish solid; mp 80-81 °C; ¹H NMR (400 MHz, DMSO-d₆) *δ:* 2.47 (s, 3H), 6.71 (s, 1H), 6.93 (ddd, *J=* 9.2 Hz, *J' =* 8.7 Hz, *J"* = 2.4 Hz, 1H), 7.15 (dd, *J =* 9.2 Hz, *J' =* 2.4 Hz, 1H), 7.56 (s, 1H), 8.40-8.90 (br signal, 2H), 11.55 (s, 1H); IR (ATR) *v.* 3177, 2917, 2856, 1607, 1544, 1457, 1312, 1216, 1137, 1107, 1008, 840, 802, 728, 667, 642, 606, 581 cm⁻¹; HRMS-ESI+ m/z [M+H]⁺ calcd for [C₁₂H₁₀FN₃S+H⁺]: 248.0652, found: 248.0650.

### Reference example 9: 4-(2-methyl-5-nitro-1H-indol-3-yl)thiazol-2-amine hydrobromide

This compound is used for the synthesis of example 27.

From 2-bromoacetonitrile (142 µL, 245 mg, 2.04 mmol), PhBCl₂ (332 µL, 406 mg, 2.56 mmol), and 2-methyl-5-nitro-1*H*-indole (300 mg, 1.70 mmol), following the procedure described in reference example 4, stirring at 50 °C for 4.5 h, a crude residue was obtained and purified by automatic column chromatography (hexane:EtOAc mixtures), to provide 2-bromo-1-(2-methyl-5-nitro-1*H*-indol-3-yl)ethan-1-one (280 mg, 55% yield) as a beige solid; mp 255-256 °C; ¹H NMR (400 MHz, DMSO-d₆) *δ:* 2.79 (s, 3H), 4.79 (s, 2H), 7.59 (d, *J =* 8.8 Hz, 1H), 8.09 (dd, *J =* 8.8 Hz, *J' =* 2.4 Hz, 1H), 8.96 (d, *J* = 2.4 Hz, 1H), 12.64 (s, 1H); IR (ATR) *v.* 3273, 3095, 2948, 1645, 1620, 1536, 1465, 1422, 1338, 1305, 1272, 1155, 1084, 969, 875, 842, 830, 738, 667, 563 cm⁻¹; HRMS-ESI+ m/z [M+H]⁺ calcd for [C₁₁H₉⁷⁹BrN₂O₃+H⁺]: 296.9869, found: 296.9869.

From 2-bromo-1-(2-methyl-5-nitro-1*H*-indol-3-yl)ethan-1-one (50 mg, 0.17 mmol) and thiourea (13 mg, 0.17 mmol), following the procedure described in reference example 1, sonicating in an ultrasound bath at 60-65 °C for 2.5 h, 4-(2-methyl-5-nitro-1*H*-indol-3-yl)thiazol-2-amine hydrobromide (66 mg, quantitative yield) was obtained as a brownish solid; mp 198-199 °C; ¹H NMR (400 MHz, DMSO-d₆) *δ:* 2.54 (s, 3H), 3.45 (br s, 2H), 6.85 (s, 1H), 7.55 (d, *J =* 8.8 Hz, 1H), 8.03 (dd, *J* = 8.8 Hz, *J'* = 2.4 Hz, 1H), 8.54 (d, *J =* 2.4 Hz, 1H), 12.25 (s, 1H); IR (ATR) *v.* 3258, 3136, 3100, 1628, 1610, 1562, 1536, 1508, 1468, 1414, 1330, 1310, 1257, 1211, 1211, 1155, 1074, 903, 840, 807, 738, 695, 667 cm⁻¹; HRMS-ESI+ m/z [M+H]⁺ calcd for [C₁₂H₁₀N₄O₂S+H⁺]: 275.0597, found: 275.0595.

### Example 1: 2-(imidazo[2,1-b]thiazol-6-yl)-N-[4-(2-methyl-1H-indol-3-yl)thiazol-2-yl]acetamide

To a solution of imidazo[2,1-*b*][1,3]thiazol-6-ylacetic acid hydrochloride hydrate (1.08 g, 4.56 mmol) and 4-(2-methyl-1*H*-indol-3-yl)thiazol-2-amine hydrobromide (1.42 g, 4.57 mmol) in a mixture of EtOAc (27 mL) and DMF (2.7 mL), DIPEA (3.60 mL, 20.7 mmol) was added, and the solution was stirred at rt for 5 min. Then, HATU (2.61 g, 6.86 mmol) was added portionwise over 10 min and the resulting suspension was stirred for 3 min before the addition of a second portion of DMF (2.7 mL). The reaction mixture was stirred at rt for 4.5 h, concentrated under reduced pressure, taken up in EtOAc (100 mL) and washed with H₂O (5×100 mL). The organic phase was dried over Na₂SO₄, filtered, and concentrated under reduced pressure. The crude residue was purified by flash column chromatography [hexane:EtOAc (2:8-1:9)], providing 2-(imidazo[2,1-*b*]thiazol-6-yl)-*N-*[4-(2-methyl-1*H*-indol-3-yl)thiazol-2-yl]acetamide (1.60 g, 89%) as a beige solid. After washing with pentane, the analytical sample of this compound was obtained as a white solid; mp 210-212 °C; ¹H NMR (400 MHz, DMSO-d₆) *δ:* 2.63 (s, 3H), 3.84 (s, 2H), 7.02 (br dd, *J* = *J' =* 7.6 Hz, 1H), 7.060 (br s, 1H), 7.061 (br dd, *J = J' =* 7.6 Hz, 1H), 7.21 (d, *J =* 4.8 Hz, 1H), 7.31 (dm, *J* = 7.6 Hz, 1H), 7.70 (s, 1H), 7.88 (d, *J* = 4.8 Hz, 1H), 7.97 (br d, *J* = 7.6 Hz, 1H), 11.17 (s, 1H), 12.26 (s, 1H); IR (ATR) *v.* 3219, 3178, 3103, 3067, 2983, 1656, 1548, 1521, 1459, 1330, 1264, 1252, 1240, 1056, 964, 832, 741, 644 cm⁻¹; HRMS-ESI+ m/z [M+H]⁺ calcd for [C₁₉H₁₅N₅OS₂+H⁺]: 394.0791, found: 394.0785.

### Example 2: 2-(imidazo[2,1-b]thiazol-3-yl)-N-[4-(2-methyl-1H-indol-3-yl)thiazol-2-yllacetamide

From 2-(imidazo[2,1-*b*]thiazol-3-yl)acetic acid (50 mg, 0.27 mmol), 4-(2-methyl-1*H*-indol-3-yl)thiazol-2-amine hydrobromide (94 mg, 0.30 mmol), DIPEA (0.17 mL, 0.98 mmol) and HATU (157 mg, 0.41 mmol), and following the procedure described in example 1, 2-(imidazo[2,1-b]thiazol-3-yl)-*N*-[4-(2-methyl-1*H*-indol-3-yl)thiazol-2-yl]acetamide (39 mg, 37%) was obtained as a beige sticky solid. Recrystallization from EtOH, followed by washing with pentane afforded the analytical sample of this compound as a white solid; mp 160-163 °C; ¹H NMR (400 MHz, DMSO-d₆) *δ:* 2.63 (s, 3H), 4.38 (s, 2H), 7.02 (br dd, *J = J'* = 7.2 Hz, 1H), 7.06 (br dd, *J = J' =* 7.2 Hz, 1H), 7.10 (s, 1H), 7.32 (d, *J* = 7.2 Hz, 1H), 7.63 (s, 1H), 7.91 (br s, 1H), 7.96 (d, *J* = 7.2 Hz, 1H), 8.29 (d, *J =* 2.0 Hz, 1H), 11.29 (s, 1H), 12.59 (br s, 1H); IR (ATR) *v.* 3131, 2956, 1671, 1549, 1524, 1476, 1458, 1430, 1350, 1334, 1292, 1269, 1234, 1197, 1165, 1130, 1050, 821, 752, 717, 689 cm⁻¹; HRMS-ESI+ m/z [M+H]⁺ calcd for [C₁₉H₁₅N₅OS₂+H⁺]: 394.0791, found: 394.0788.

### Example 3: 2-(benzo[d]oxazol-2-yl)-N-[4-(2-methyl-1H-indol-3-yl)thiazol-2-yl]acetamide

From sodium 2-(benzo[*d*]oxazol-2-yl)acetate (57 mg, 0.29 mmol), 4-(2-methyl-1*H*-indol-3-yl)thiazol-2-amine hydrobromide (80 mg, 0.26 mmol), DIPEA (0.11 mL, 0.63 mmol) and HATU (147 mg, 0.39 mmol), and following the procedure described in example 1, 2-(benzo[*d*]oxazol-2-yl)-*N*-[4-(2-methyl-1*H*-indol-3-yl)thiazol-2-yl]acetamide (31 mg, 31%) was obtained. After washing with pentane, the analytical sample of this compound was obtained as a beige solid; mp 239-242 °C; ¹H NMR (400 MHz, DMSO-d₆) *δ:* 2.63 (s, 3H), 4.34 (s, 2H), 7.02 (br dd, *J = J'* = 7.6 Hz, 1H), superimposed in part 7.07 (br dd, *J = J' =* 7.6 Hz, 1H), 7.12 (s, 1H), 7.32 (d, *J* = 7.6 Hz, 1H), 7.38 (br dd, *J = J' =* 7.2 Hz, 1H), superimposed in part 7.41 (br dd, *J* = *J' =* 7.2 Hz, 1H), 7.74 (m, 2H), 7.97 (d, *J* = 7.6 Hz, 1H), 11.20 (s, 1H), 12.58 (s, 1H); IR (ATR) *v.* 3244, 3228, 3186, 3116, 3081, 2919, 1654, 1579, 1551, 1510, 1482, 1457, 1370, 1296, 1235, 1148, 1055, 928, 843, 738, 690 cm⁻¹. HRMS-ESI+ m/z [M+H]⁺ calcd for [C₂₁H₁₆N₄O₂S+H⁺]: 389.1067, found: 389.1064.

### Example 4: 2-(imidazo[2,1-b]thiazol-6-yl)-N-[5-methyl-4-(2-methyl-1H-indol-3-yl)thiazol-2-yl]acetamide

From 2-(imidazo[2,1-*b*]thiazol-6-yl)acetic acid hydrochloride (150 mg, 0.69 mmol), 5-methyl-4-(2-methyl-1*H*-indol-3-yl)thiazol-2-amine hydrobromide (222 mg, 0.68 mmol), DIPEA (0.48 mL, 2.76 mmol) and HATU (391 mg, 1.03 mmol), and following the procedure described in example 1, 2-(imidazo[2,1-*b*]thiazol-6-yl)-*N*-[5-methyl-4-(2-methyl-1*H*-indol-3-yl)thiazol-2-yl]acetamide (238 mg, 86%) was obtained. After washing with pentane, the analytical sample of this compound was obtained as a yellowish solid; mp 169-172 °C; ¹H NMR (400 MHz, DMSO-d₆) *δ:* 2.21 (s, 3H), 2.34 (s, 3H), 3.79 (s, 2H), 6.95 (ddd, *J* = *J' =* 7.2 Hz, *J" =* 1.2 Hz, 1H), 7.03 (ddd, *J = J' =* 7.2 Hz, *J"* = 1.2 Hz, 1H), 7.21 (d, *J* = 4.4 Hz, 1H), 7.31 (m, 2H), 7.68 (s, 1H), 7.87 (d, *J* = 4.4 Hz, 1H), 11.14 (s, 1H), 12.13 (s, 1H); IR (ATR) *v.* 3130, 3098, 3043, 2917, 1661, 1549, 1459, 1424, 1275, 1219, 1151, 839, 739, 715, 658, 641 cm⁻¹; HRMS-ESI+ m/z [M+H]⁺ calcd for [C₂₀H₁₇N₅OS₂+H⁺]: 408.0947, found: 408.0937.

### Example 5: 2-(imidazo[2,1-b]thiazol-6-yl)-N-[4-(2-methyl-1H-indol-3-yl)oxazol-2-yl]acetamide

From 2-(imidazo[2,1-*b*]thiazol-6-yl)acetic acid hydrochloride (103 mg, 0.47 mmol), 4-(2-methyl-1*H*-indol-3-yl)oxazol-2-amine (120 mg, 0.56 mmol), DIPEA (0.25 mL, 1.44 mmol) and HATU (267 mg, 0.70 mmol), and following the procedure described in example 1, 2-(imidazo[2,1-*b*]thiazol-6-yl)-*N*-[4-(2-methyl-1*H*-indol-3-yl)oxazol-2-yl]acetamide (53 mg, 30%) was obtained. After washing with pentane, the analytical sample of this compound was obtained as a beige solid; mp 122-124 °C; ¹H NMR (400 MHz, DMSO-d₆) *δ:* 2.59 (s, 3H), 3.78 (br s, 2H), 7.01 (ddd, *J = J' =* 7.2 Hz, *J"* = 1.2 Hz, 1H), 7.06 (ddd, *J* = *J'* = 7.2 Hz, *J"* = 1.2 Hz, 1H), 7.21 (d, *J* = 4.4 Hz, 1H), 7.30 (dm,*J* = 7.2 Hz, 1H), 7.68 (s, 1H), 7.81 (br d, *J = 7.2* Hz, 1H), 7.88 (d, *J =* 4.4 Hz, 1H), 8.07 (s, 1H), 11.20 (s, 1H), 11.34 (s, 1H); IR (ATR) *v.* 3144, 3111, 2924, 1699, 1596, 1557, 1459, 1427, 1386, 1307, 1250, 1192, 1149, 1052, 839, 743, 659 cm⁻¹; HRMS-ESI+ m/z [M+H]⁺ calcd for [C₁₉H₁₅N₅O₂S+H⁺]: 378.1019, found: 378.1020.

### Example 6: 2-(5-methoxy-1H-indol-3-yl)-N-[4-(2-methyl-1H-indol-3-yl)thiazol-2-yl]acetamide

From 2-(5-methoxy-1*H*-indol-3-yl)acetic acid (104 mg, 0.51 mmol), 4-(2-methyl-1*H*-indol-3-yl)thiazol-2-amine (106 mg, 0.46 mmol), DIPEA (0.18 mL, 1.03 mmol) and HATU (264 mg, 0.69 mmol), and following the procedure described in example 1, 2-(5-methoxy-1H-indol-3-yl)-*N*-[4-(2-methyl-1*H*-indol-3-yl)thiazol-2-yl]acetamide (68 mg, 35%) was obtained as a beige solid. After washing with pentane, the analytical sample of this compound was obtained as a grey solid; mp 127-129 °C; ¹H NMR (400 MHz, DMSO-d₆) *δ:* 2.62 (s, 3H), 3.76 (s, 3H), 3.86 (s, 2H), 6.74 (dd, *J* = 8.8 Hz, *J' =* 2.4 Hz, 1H), superimposed in part 7.01 (ddd, *J* = *J*' = 7.2 Hz, *J"* = 1.2 Hz, 1H), 7.03 (s, 1H), 7.06 (ddd, *J = J' =* 7.2 Hz, *J"* = 1.2 Hz, 1H), 7.15 (d, *J =* 2.4 Hz, 1H), 7.24-7.27 (m, 2H), 7.31 (dm, *J =* 7.2 Hz, 1H), 7.96 (br d, *J = 7.2* Hz, 1H), 10.80 (s, 1H), 11.17 (s, 1H), 12.26 (s, 1H); IR (ATR) *v.* 3640, 3571, 3391, 2924, 1668, 1627, 1538, 1486, 1457, 1282, 1215, 1057, 835, 743 cm⁻¹; HRMS-ESI+ m/z [M+H]⁺ calcd for [C₂₃H₂₀N₄O₂S+H⁺]: 417.1380, found: 417.1377.

### Example 7: 2-(1H-indol-2-yl)-N-4-(2-methyl-1H-indol-3-yl)thiazol-2-yl]acetamide

From 2-(1*H*-indol-2-yl)acetic acid (75 mg, 0.43 mmol), 4-(2-methyl-1*H*-indol-3-yl)thiazol-2-amine hydrobromide (133 mg, 0.43 mmol), DIPEA (0.26 mL, 1.49 mmol) and HATU (244 mg, 0.64 mmol), and following the procedure described in example 1, 2-(1*H*-indol-2-yl)-*N*-[4-(2-methyl-1*H*-indol-3-yl)thiazol-2-yl]acetamide (23 mg, 14%) was obtained. After washing with pentane, the analytical sample of this compound was obtained as a brownish solid; mp 166-168 °C; ¹H NMR (400 MHz, DMSO-d₆) *δ:* 2.63 (s, 3H), 4.00 (s, 2H), 6.31 (br s, 1H), 6.95 (ddd, *J = J' =* 7.2 Hz, *J"* = 1.2 Hz, 1H), 6.99-7.07 (m, 3H), 7.08 (s, 1H), 7.31 (br d, *J* = 7.2 Hz, 1H), 7.34 (dm, *J* = 8.0 Hz, 1H), 7.45 (d, *J* = 8.0 Hz, 1H), 7.97 (br d, *J* = 7.2 Hz, 1H), 11.06 (s, 1H), 11.18 (s, 1H), 12.35 (s, 1H); IR (ATR) *v.* 3370, 3055, 2922, 1674, 1619, 1538, 1455, 1418, 1288, 1061, 742, 639 cm⁻¹; HRMS-ESI+ m/z [M+H]⁺ calcd for [C₂₂H₁₈N₄OS+H⁺]: 387.1274, found: 387.1276.

### Example 8: 2-(6-fluoroimidazo[1,2-a]pyridin-2-yl)-N-[4-(2-methyl-1H-indol-3-yl)thiazol-2-yl]acetamide

From 2-(6-fluoroimidazo[1,2-*a*]pyridin-2-yl)acetic acid hydrochloride (125 mg, 0.54 mmol), 4-(2-methyl-1*H*-indol-3-yl)thiazol-2-amine hydrobromide (168 mg, 0.54 mmol), DIPEA (0.42 mL, 2.41 mmol) and HATU (309 mg, 0.81 mmol), and following the procedure described in example 1, 2-(6-fluoroimidazo[1,2-*a*]pyridin-2-yl)-*N*-[4-(2-methyl-1*H*-indol-3-yl)thiazol-2-yl]acetamide (138 mg, 63%) was obtained as a brownish solid. After washing with pentane, the analytical sample of this compound was obtained as a beige solid; mp 114-119 °C; ¹HNMR (400 MHz, DMSO-d₆) *δ:* 2.63 (s, 3H), 3.98 (s, 2H), 7.02 (ddd, *J = J' =* 7.2 Hz, *J"* = 1.2 Hz, 1H), superimposed in part 7.06 (ddd, *J = J'* = 7.2 Hz, *J"* = 1.2 Hz, 1H), 7.07 (s, 1H), 7.27-7.33 (m, 2H), 7.56 (dd, *J* = 10.0 Hz, *J' =* 5.2 Hz, 1H), 7.92 (s, 1H), 7.97 (br d, *J* = 7.2 Hz, 1H), 8.77 (dd, *J* = 5.2 Hz, *J' =* 2.4 Hz, 1H), 11.18 (s, 1H), 12.35 (s, 1H); IR (ATR) *v.* 3130, 3054, 2923, 1673, 1538, 1509, 1457, 1280, 1232, 1163, 1054, 816, 742 cm⁻¹; HRMS-ESI+ m/z [M+H]⁺ calcd for [C₂₁H₁₆FN₃OS+H⁺]: 406.1132, found: 406.1131.

### Example 9: N-[4-(2-methyl-1H-indol-3-yl)thiazol-2-yl]-2-(2-methylimidazo[2,1-b]thiazol-6-yl)acetamide

From 2-(2-methylimidazo[2,1-*b*]thiazol-6-yl)acetic acid (100 mg, 0.51 mmol), 4-(2-methyl-1*H*-indol-3-yl)thiazol-2-amine hydrobromide (158 mg, 0.51 mmol), DIPEA (0.31 mL, 1.78 mmol) and HATU (291 mg, 0.77 mmol), and following the procedure described in example 1, *N*-[4-(2-methyl-1*H*-indol-3-yl)thiazol-2-yl]-2-(2-methylimidazo[2,1-*b*]thiazol-6-yl)acetamide (164 mg, 79%) was obtained as a beige solid. After washing with pentane, the analytical sample of this compound was obtained as a white solid; mp 140-142 °C; ¹H NMR (400 MHz, DMSO-d₆) *δ*: 2.38 (d, *J* = 1.6 Hz, 3H), 2.63 (s, 3H), 3.81 (s, 2H), 7.02 (ddd, *J* = *J' =* 7.2 Hz, *J"* = 1.2 Hz, 1H), 7.059 (s, 1H), superimposed in part 7.060 (ddd, *J* = *J' =* 7.2 Hz, *J"* = 1.2 Hz, 1H), 7.31 (br d, *J* = 7.2 Hz, 1H), 7.60 (s, 1H), 7.63 (q, *J* = 1.6 Hz, 1H), 7.97 (br d, *J* = 7.2 Hz, 1H), 11.18 (s, 1H), 12.24 (s, 1H); IR (ATR) *v.* 3380, 3141, 2918, 1674, 1538, 1460, 1417, 1282, 1231, 1055, 843, 741, 688, 643 cm⁻¹; HRMS-ESI+ m/z [M+H]⁺ calcd for [C₂₀H₁₇N₅OS₂+H⁺]: 408.0947, found: 408.0944.

### Example 10: N-[4-(2-methyl-1H-indol-3-yl)thiazol-2-yl]-2-(3-methylimidazo[2,1-b]thiazol-6-yl)acetamide

From 2-(3-methylimidazo[2,1-*b*]thiazol-6-yl)acetic acid (100 mg, 0.51 mmol), 4-(2-methyl-1*H*-indol-3-yl)thiazol-2-amine hydrobromide (158 mg, 0.51 mmol), DIPEA (0.31 mL, 1.78 mmol) and HATU (291 mg, 0.77 mmol), and following the procedure described in example 1, *N*-[4-(2-methyl-1*H*-indol-3-yl)thiazol-2-yl]-2-(3-methylimidazo[2,1-*b*]thiazol-6-yl)acetamide (142 mg, 68%) was obtained as a beige solid. After washing with pentane, the analytical sample of this compound was obtained as a white solid; mp 145-148 °C; ¹H NMR (400 MHz, DMSO-d₆) *δ*: 2.39 (d, *J* = 1.2 Hz, 3H), 2.63 (s, 3H), 3.85 (s, 2H), 6.85 (q, *J* = 1.2 Hz, 1H), 7.02 (ddd, *J* = *J' =* 7.2 Hz, *J" =* 1.2 Hz, 1H), superimposed in part 7.061 (ddd, *J* = *J' =* 7.2 Hz, *J"* = 1.2 Hz, 1H), 7.063 (s, 1H), 7.31 (br d, *J* = 7.2 Hz, 1H), 7.64 (s, 1H), 7.97 (br d, *J* = 7.2 Hz, 1H), 11.18 (s, 1H), 12.27 (s, 1H); IR (ATR) *v.* 3370, 3109, 3043, 2914, 1674, 1538, 1461, 1282, 1231, 1054, 841, 741, 686, 645 cm⁻¹; HRMS-ESI+ m/z [M+H]⁺ calcd for [C₂₀H₁₇N₅OS₃+H⁺]: 408.0947, found: 408.0944.

### Example 11: 2-(1-methyl-1H-indol-3-yl)-N-[4-(2-methyl-1H-indol-3-yl)thiazol-2-yl]acetamide

From 2-(1-methyl-1*H*-indol-3-yl)acetic acid (101 mg, 0.53 mmol), 4-(2-methyl-1*H*-indol-3-yl)thiazol-2-amine hydrobromide (150 mg, 0.48 mmol), DIPEA (0.30 mL, 1.72 mmol) and HATU (276 mg, 0.73 mmol), and following the procedure described in example 1, 2-(1-methyl-1*H*-indol-3-yl)-*N*-[4-(2-methyl-1*H*-indol-3-yl)thiazol-2-yl]acetamide (148 mg, 77%) was obtained. After washing with pentane, the analytical sample of this compound was obtained as a grey solid; mp 139-142 °C ; ¹H NMR (400 MHz, DMSO-d₆) *δ:* 2.63 (s, 3H), 3.77 (s, 3H), 3.90 (s, 2H), 7.00-7.09 (m, 4H), 7.15 (ddd, *J* = *J' =* 7.6 Hz, *J"* = 1.2 Hz, 1H), 7.29 (s, 1H), 7.32 (br d, *J* = 7.2 Hz, 1H), 7.40 (br d, *J* = 8.0 Hz, 1H), 7.64 (br d, *J* = 8.0 Hz, 1H), 7.97 (br d, *J =* 7.6 Hz, 1H), 11.17 (s, 1H), 12.29 (s, 1H); IR (ATR) *v.* 3377, 3052, 2915, 1671, 1532, 1457, 1330, 1281, 1233, 1231, 1151, 1136, 1056, 1009, 737, 692, 644 cm⁻¹; HRMS-ESI+ m/z [M+H]⁺ calcd for [C₂₃H₂₀N₄OS+H⁺]: 401.1431, found: 401.1434.

### Example 12: 2-(5-fluoro-1H-indol-3-yl)-N-[4-(2-methyl-1H-indol-3-yl)thiazol-2-yl]acetamide

From 2-(5-fluoro-1*H*-indol-3-yl)acetic acid (103 mg, 0.53 mmol), 4-(2-methyl-1*H*-indol-3-yl)thiazol-2-amine hydrobromide (150 mg, 0.48 mmol), DIPEA (0.30 mL, 1.72 mmol) and HATU (276 mg, 0.73 mmol), and following the procedure described in example 1, 2-(5-fluoro-1*H*-indol-3-yl)-*N*-[4-(2-methyl-1*H*-indol-3-yl)thiazol-2-yl]acetamide (189 mg, 97%) was obtained. After washing with pentane, the analytical sample of this compound was obtained as a brownish solid; mp 230-232 °C; ¹H NMR (400 MHz, DMSO-d₆) *δ:* 2.62 (s, 3H), 3.88 (s, 2H), 6.93 (ddd, *J* = *J' =* 9.2 Hz, *J"* = 2.8 Hz, 1H), superimposed in part 7.02 (ddd, *J = J' =* 7.2 Hz, *J"* = 1.2 Hz, 1H), 7.04 (s, 1H), 7.06 (ddd, *J* = *J' =* 7.2 Hz, *J"* = 1.2 Hz, 1H), 7.31 (br d, *J* = 7.2 Hz, 1H), 7.34-7.41 (m, 3H), 7.96 (br d, *J* = 7.2 Hz, 1H), 11.08 (d, *J* = 2.4 Hz, 1H), 11.17 (s, 1H), 12.28 (s, 1H); IR (ATR) *v.* 3393, 3356, 3153, 3047, 2920, 2883, 1673, 1668, 1581, 1550, 1488, 1456, 1336, 1280, 1233, 1225, 1203, 1056, 936, 840, 742, 650, 559 cm⁻¹; HRMS-ESI+ m/z [M+H]⁺ calcd for [C₂₂H₁₇FN₄OS+H⁺]: 405.1180, found: 405.1187.

### Example 13: 2-(1H-indol-3-yl)-N-[4-(2-methyl-1H-indol-3-yl)thiazol-2-yl]acetamide

From 2-(1*H*-indol-3-yl)acetic acid (49.7 mg, 0.28 mmol), 4-(2-methyl-1*H*-indol-3-yl)thiazol-2-amine hydrobromide (80.0 mg, 0.26 mmol), DIPEA (0.16 mL, 0.92 mmol) and HATU (147 mg, 0.39 mmol), and following the procedure described in example 1, 2-(1*H*-indol-3-yl)-*N*-[4-(2-methyl-1*H*-indol-3-yl)thiazol-2-yl]acetamide (62.0 mg, 62%) was obtained. After washing with Et₂O and recrystallization from EtOH, the analytical sample of this compound was obtained as a white solid; mp 246-248 °C; ¹H NMR (400 MHz, DMSO-d₆) *δ:* 2.63 (s, 3H), 3.91 (s, 2H), 7.01 (ddd, *J = J' =* 7.6 Hz, *J"* = 1.2 Hz, 1H), 7.02 (ddd, *J = J' =* 7.6 Hz, *J" =* 1.2 Hz, 1H), 7.04 (s, 1H), 7.07 (ddd, *J* = *J' =* 7.6 Hz, *J"* = 1.2 Hz, 1H), 8.00 (ddd, *J = J'* = 7.6 Hz, *J"* = 1.2 Hz, 1H), 7.29-7.33 (m, 2H), 7.38 (br d, *J* = 7.6 Hz, 1H), 7.63 (d, *J* = 7.6 Hz, 1H), 7.97 (d, *J* = 7.6 Hz, 1H), 10.97 (br s, 1H), 11.18 (s, 1H), 12.28 (s, 1H); IR (ATR) *v.* 3386, 3151, 3048, 2878, 1678, 1669, 1551, 1458, 1322, 1314, 1282, 1233, 1183, 1054, 837, 737, 644 cm⁻¹; HRMS-ESI+ m/z [M+H]⁺ calcd for [C₂₂H₁₈N₄OS+H⁺]: 387.1274, found: 387.1274.

### Example 14: 3-(1H-indol-3-yl)-N-[4-(2-methyl-1H-indol-3-yl)thiazol-2-yl]propanamide

From 3-(1*H*-indol-3-yl)propanoic acid (53.7 mg, 0.28 mmol), 4-(2-methyl-1*H-*indol-3-yl)thiazol-2-amine hydrobromide (80.0 mg, 0.26 mmol), DIPEA (0.16 mL, 0.92 mmol) and HATU (147 mg, 0.39 mmol), and following the procedure described in example 1, 3-(1*H*-indol-3-yl)-*N*-[4-(2-methyl-1*H*-indol-3-yl)thiazol-2-yl]propanamide (51.0 mg, 49%) was obtained as a light brown solid; mp 133-135 °C; ¹H NMR (400 MHz, DMSO-d₆) *δ:* 2.62 (s, 3H), 2.85 (t, *J =* 7.6 Hz, 2H), 3.07 (t, *J =* 7.6 Hz, 2H), 6.99 (ddd, *J = J' =* 7.6 Hz, *J"* = 1.2 Hz, 1H), 7.01 (ddd, *J = J' =* 7.6 Hz, *J"* = 1.2 Hz, 1H), 7.05 (s, 1H), 7.06 (ddd, *J = J' =* 7.6 Hz, *J"* = 1.2 Hz, 1H), 7.08 (ddd, *J = J' =* 7.6 Hz, *J"* = 1.2 Hz, 1H), 7.15 (br d, *J* = 2.4 Hz, 1H), 7.30 (dm, *J* = 7.6 Hz, 1H), 7.34 (dm, *J* = 7.6 Hz, 1H), 7.60 (d, *J* = 7.6 Hz, 1H), 7.96 (d, *J =* 7.6 Hz, 1H), 10.79 (s, 1H), 11.17 (s, 1H), 12.11 (s, 1H); IR (ATR) *v.* 3392, 3047, 2918, 2852, 1662, 1607, 1537, 1456, 1416, 1284, 1229, 1151, 1058, 739, 644, 580 cm⁻¹; HRMS-ESI+ m/z [M+H]⁺ calcd for [C₂₃H₂₀N₄OS+H⁺]: 401.1431, found: 401.1432.

### Example 15: 2-(imidazo[2,1-b]thiazol-6-yl)-N-methyl-N-[4-(2-methyl-1H-indol-3-yl)thiazol-2-yl]propanamide

To a solution of 2-(imidazo[2,1-*b*]thiazol-6-yl)-*N*-[4-(2-methyl-1*H*-indol-3-yl)thiazol-2-yl]acetamide (72.0 mg, 0.18 mmol) in DMSO (0.7 mL), finely powdered KOH (85% purity reagent, 24.2 mg, 0.37 mmol) and iodomethane (26 µL, 0.42 mmol) were successively added and the resulting mixture was stirred at rt for 2 h. A second portion of iodomethane (13 µL, 0.21 mmol) was added and the reaction mixture was stirred at rt for 1 h. H₂O (2 mL) was added and the resulting precipitate was collected by filtration and washed with H₂O and Et₂O. After drying, 2-(imidazo[2,1-*b*]thiazol-6-yl)-*N*-methyl-*N*-[4-(2-methyl-1*H*-indol-3-yl)thiazol-2-yl]propanamide (54 mg, 71%) was obtained; mp 85-88 °C; ¹H NMR (400 MHz, CDCl₃) *δ:* 1.67 (d, *J* = 6.8 Hz, 3H), 2.68 (s, 3H), 3.94 (s, 3H), 4.48 (q, *J* = 6.8 Hz, 1H), 6.80 (d, *J* = 4.5 Hz, 1H), 6.97 (s, 1H), 7.12-7.17 (m, 2H), 7.28-7.33 (m, 1H), 7.35 (d, *J =* 4.5 Hz, 1H), 7.39 (s, 1H), 7.95-8.01 (m, 2H); IR (ATR) *v*. 3109, 2921, 2851, 1658, 1456, 1418, 1288, 1231, 1119, 1055, 741, 651 cm⁻¹; HRMS-ESI+ m/z [M+H]⁺ calcd for [C₂₁H₁₉N₅OS₂+H⁺]: 422.1104, found: 422.1102.

### Example 16: 2-(6-chloro-1H-indol-3-yl)-N-[4-(2-methyl-1H-indol-3-yl)thiazol-2-yl]acetamide

From 2-(6-chloro-1*H*-indol-3-yl)acetic acid (67.5 mg, 0.32 mmol), 4-(2-methyl-1*H*-indol-3-yl)thiazol-2-amine hydrobromide (100 mg, 0.32 mmol), DIPEA (0.25 mL, 1.44 mmol) and HATU (183 mg, 0.48 mmol), and following the procedure described in example 1, 2-(6-chloro-1*H*-indol-3-yl)-*N*-[4-(2-methyl-1*H*-indol-3-yl)thiazol-2-yl]acetamide (39.2 mg, 29%) was obtained as a beige solid; mp 175-177 °C; ¹H NMR (400 MHz, DMSO-d₆) *δ:* 2.62 (s, 3H), 3.90 (s, 2H), superimposed in part 7.01 (ddd, *J* = *J'* = 7.2 Hz, *J"* = 1.2 Hz, 1H), 7.03 (dd, *J* = 8.4 Hz, *J'* = 2.0 Hz, 1H), 7.04 (s, 1H), superimposed in part 7.06 (ddd, *J = J' =* 7.2 Hz, *J"* = 1.2 Hz, 1H), 7.31 (dm, *J* = 7.2 Hz, 1H), 7.35 (br d, *J* = 2.0 Hz, 1H), 7.41 (dd, *J* = 2.0 Hz, *J'* = 0.4 Hz, 1H), 7.62 (d, *J* = 8.4 Hz, 1H), 7.96 (br d, *J* = 7.2 Hz, 1H), 11.10 (br s, 1H), 11.17 (br s, 1H), 12.28 (br s, 1H); IR (ATR) *v.* 3371, 3062, 2966, 2884, 1681, 1544, 1459, 1335, 1301, 1279, 1233, 1057, 806, 737 cm⁻¹; HRMS-ESI+ m/z [M+H]⁺ calcd for [C₂₂H₁₇ClN₄OS+H⁺]: 421.0884, found: 421.0889.

### Example 17: 2-(1H-imidazol-4-yl)-N-[4-(2-methyl-1H-indol-3-yl)thiazol-2-yl]acetamide

From 2-(1*H*-imidazol-4-yl)acetic acid hydrochloride (78 mg, 0.48 mmol), 4-(2-methyl-1*H*-indol-3-yl)thiazol-2-amine hydrobromide (150 mg, 0.48 mmol), DIPEA (0.38 mL, 2.18 mmol) and HATU (276 mg, 0.73 mmol), and following the procedure described in example 1, 2-(1*H*-imidazol-4-yl)-*N*-[4-(2-methyl-1*H*-indol-3-yl)thiazol-2-yl]acetamide (34 mg, 21%) was obtained as a brownish solid. After washing with pentane, the analytical sample of this compound was obtained as a grey solid; mp 107-112 °C; ¹H NMR (400 MHz, DMSO-d₆) *δ:* 2.62 (s, 3H), 3.75 (s, 2H), 6.99 (br s, 1H), 7.01 (ddd, *J* = *J' =* 7.2 Hz, *J"* = 1.2 Hz, 1H), 7.056 (s, 1H), 7.058 (ddd, *J = J' =* 7.2 Hz, *J"* = 1.2 Hz, 1H), 7.31 (br d, *J* = *7.2* Hz, 1H), 7.63 (s, 1H), 7.96 (br d, *J* = 7.2 Hz, 1H), 11.17 (s, 1H), 12.20 (s, 1H); IR (ATR) *v.* 3183, 2920, 1673, 1548, 1457, 1288, 1232, 1083, 1056, 827, 742, 694 cm⁻¹; HRMS-ESI+ m/z [M+H]⁺ calcd for [C₁₇H₁₅N₅OS+H⁺]: 338.1070, found: 338.1067.

### Example 18: 2-(benzofuran-2-yl)-N-[4-(2-methyl-1H-indol-3-yl)thiazol-2-yl]acetamide

From 2-(benzofuran-2-yl)acetic acid (100 mg, 0.57 mmol), 4-(2-methyl-1*H-*indol-3-yl)thiazol-2-amine hydrobromide (176 mg, 0.57 mmol), DIPEA (0.35 mL, 2.01 mmol) and HATU (324 mg, 0.85 mmol), and following the procedure described in example 1, 2-(benzofuran-2-yl)-*N*-[4-(2-methyl-1*H*-indol-3-yl)thiazol-2-yl]acetamide (158 mg, 72%) was obtained as a purple solid. After washing with pentane, the analytical sample of this compound was obtained as a pink solid; mp 198-200 °C; ¹H NMR (400 MHz, DMSO-d₆) *δ:* 2.64 (s, 3H), 4.12 (s, 2H), 6.83 (s, 1H), 7.03 (ddd, *J = J' =* 7.2 Hz, *J"* = 1.2 Hz, 1H), 7.07 (ddd, *J = J' =* 7.2 Hz, *J"* = 1.2 Hz, 1H), 7.10 (s, 1H), 7.23 (ddd, *J = J'* = 7.6 Hz, *J"* = 1.2 Hz, 1H), 7.27 (ddd, *J* = *J' =* 7.6 Hz, *J" =* 1.2 Hz, 1H), 7.32 (br d, *J =* 7.2 Hz, 1H), 7.54 (d, *J* = 7.6 Hz, 1H), 7.61 (d, *J* = 7.6 Hz, 1H), 7.98 (br d, *J* = 7.2 Hz, 1H), 11.20 (s, 1H), 12.47 (s, 1H); IR (ATR) *v.* 3409, 3110, 2941, 2896, 1693, 1543, 1506, 1454, 1306, 1288, 1266, 1254, 1232, 1178, 1158, 1058, 843, 758, 751, 738, 645 cm⁻¹; HRMS-ESI+ m/z [M+H]⁺ calcd for [C₂₂H₁₇N₃O₂S+H⁺]: 388.1114, found: 388.1109.

### Example 19: 2-(4-bromothiophen-2-yl)-N-[4-(2-methyl-1H-indol-3-yl)thiazol-2-yl]acetamide

From 2-(4-bromothiophen-2-yl)acetic acid (120 mg, 0.54 mmol), 4-(2-methyl-1*H*-indol-3-yl)thiazol-2-amine hydrobromide (168 mg, 0.54 mmol), DIPEA (0.33 mL, 1.89 mmol) and HATU (310 mg, 0.82 mmol), and following the procedure described in example 1, 2-(4-bromothiophen-2-yl)-*N*-[4-(2-methyl-1*H*-indol-3-yl)thiazol-2-yl]acetamide (165 mg, 71%) was obtained. After washing with pentane, the analytical sample of this compound was obtained as a beige solid; mp 212-214 °C; ¹H NMR (400 MHz, DMSO-d₆) *δ:* 2.62 (s, 3H), 4.07 (d, *J* = 1.2 Hz, 2H), superimposed in part 7.02 (ddd, *J =* 8.0 Hz, *J' =* 7.2 Hz, *J" =* 1.2 Hz, 1H), 7.04 (d, *J* = 1.6 Hz, 1H), 7.06 (ddd, *J* = 8.0 Hz, *J'* = 7.2 Hz, *J"* = 1.2 Hz, 1H), 7.09 (s, 1H), 7.31 (br d, *J* = 8.0 Hz, 1H), 7.56 (d, *J* = 1.6 Hz, 1H), 7.96 (br d, *J* = 8.0 Hz, 1H), 11.19 (s, 1H), 12.39 (s, 1H); IR (ATR) *v.* 3401, 3367, 3053, 2894, 2735, 1687, 1680, 1556, 1506, 1460, 1413, 1315, 1283, 1231, 1198, 1057, 870, 837, 823, 748, 690, 646, 591 cm⁻¹; HRMS-ESI+ m/z[M+H]⁺ calcd for [C₁₈H₁₄⁷⁹BrN₃OS₂+H⁺]: 431.9834, found: 431.9828.

### Example 20: N-[4-(2-methyl-1H-indol-3-yl)thiazol-2-yl]-2-(3-methylbenzo[b]thiophen-2-yl)acetamide

From 2-(3-methylbenzo[b]thiophen-2-yl)acetic acid (100 mg, 0.48 mmol), 4-(2-methyl-1*H*-indol-3-yl)thiazol-2-amine hydrobromide (150 mg, 0.48 mmol), DIPEA (0.30 mL, 1.72 mmol) and HATU (276 mg, 0.73 mmol), and following the procedure described in example 1, *N*-[4-(2-methyl-1*H*-indol-3-yl)thiazol-2-yl]-2-(3-methylbenzo[*b*]thiophen-2-yl)acetamide (121 mg, 60%) was obtained. After washing with pentane, the analytical sample of this compound was obtained as a reddish solid; mp 233-235 °C; ¹H NMR (400 MHz, DMSO-d₆) *δ*: 2.39 (s, 3H), 2.63 (s, 3H), 4.16 (s, 2H), 7.03 (br dd, *J* = *J' =* 7.6 Hz, 1H), 7.05 (br dd, *J = J'* = 7.6 Hz, 1H), 7.08 (s, 1H), 7.32 (br d, *J* = 7.6 Hz, 1H), superimposed in part 7.34 (br dd, *J* = *J' =* 7.6 Hz, 1H), 7.40 (br dd, *J = J' =* 7.6 Hz, 1H), 7.73 (br d, *J* = 7.6 Hz, 1H), 7.89 (d, *J* = 7.6 Hz, 1H), 7.98 (d, *J* = 7.6 Hz, 1H), 11.19 (s, 1H), 12.45 (s, 1H); IR (ATR) *v.* 3372, 3050, 2912, 2854, 1674, 1538, 1458, 1431, 1411, 1281, 1224, 1184, 1157, 1055, 742, 727, 645 cm⁻¹; HRMS-ESI+ m/z [M+H]⁺ calcd for [C₂₃H₁₉N₃OS₂+H⁺]: 418.1042, found: 418.1043.

### Example 21: 2-(5-hydroxy-1H-indol-3-yl)-N-[4-(2-methyl-1H-indol-3-yl)thiazol-2-yl]acetamide

From 2-(5-hydroxy-1*H*-indol-3-yl)acetic acid (100 mg, 0.52 mmol), 4-(2-methyl-1*H*-indol-3-yl)thiazol-2-amine hydrobromide (162 mg, 0.52 mmol), DIPEA (0.09 mL, 0.52 mmol) and HATU (298 mg, 0.78 mmol), and following the procedure described in example 1, 2-(5-hydroxy-1*H*-indol-3-yl)-*N*-[4-(2-methyl-1*H*-indol-3-yl)thiazol-2-yl]acetamide (129 mg, 62%) was obtained. After washing with pentane, the analytical sample of this compound was obtained as a brownish solid; mp 145-149 °C; ¹H NMR (400 MHz, DMSO-d₆) *δ:* 2.62 (s, 3H), 3.80 (s, 2H), 6.60 (dd, *J* = 8.4 Hz, *J' =* 2.0 Hz, 1H), 6.92 (d, *J* = 2.0 Hz, 1H), superimposed in part 7.01 (ddd, *J = J' =* 7.2 Hz, *J"* = 1.2 Hz, 1H), 7.03 (s, 1H), 7.06 (ddd, *J = J'* = 7.2 Hz, *J"* = 1.2 Hz, 1H), 7.15 (d, *J* = 8.4 Hz, 1H), 7.20 (d, *J* = 2.4 Hz, 1H), 7.31 (br d, *J* = 7.2 Hz, 1H), 7.96 (br d, *J* = 7.2 Hz, 1H), 8.62 (s, 1H), 10.63 (br s, 1H), 11.16 (s, 1H), 12.22 (s, 1H); IR (ATR) *v.* 3390, 2923, 1668, 1628, 1532, 1457, 1283, 1231, 1055, 939, 839, 743, 694, 644 cm⁻¹; HRMS-ESI+ m/z [M+H]⁺ calcd for [C₂₂H₁₈N₄O₂S+H⁺]: 403.1223, found: 403.1221.

### Example 22: N-[4-(4-bromo-1H-indol-3-yl)thiazol-2-yl]-2-(imidazo[2,1-b]thiazol-6-yl)acetamide

From imidazo[2,1-*b*][1,3]thiazol-6-ylacetic acid hydrochloride hydrate (95.8 mg, 0.40 mmol), 4-(4-bromo-1*H*-indol-3-yl)thiazol-2-amine (119 mg, 0.40 mmol), DIPEA (0.24 mL, 1.38 mmol) and HATU (228 mg, 0.60 mmol), and following the procedure described in example 1, *N*-[4-(4-bromo-1*H*-indol-3-yl)thiazol-2-yl]-2-(imidazo[2,1-*b*]thiazol-6-yl)acetamide (137 mg, 75%) was obtained as a yellow solid, mp 233-235 °C; ¹H NMR (400 MHz, DMSO-d₆) *δ:* 3.81 (s, 2H), 7.04 (dd, *J* = *J' =* 8.0 Hz, 1H), 7.06 (s, 1H), 7.21 (d, *J =* 4.4 Hz, 1H), 7.23 (dd, *J* = 8.0 Hz, *J' =* 0.8 Hz, 1H), 7.47 (dd, *J =* 8.0 Hz, *J' =* 0.8 Hz, 1H), 7.55 (d, *J =* 2.4 Hz, 1H), 7.69 (s, 1H), 7.88 (d, *J =* 4.4 Hz, 1H), 11.61 (br d, *J* = 2.4 Hz, 1H), 12.25 (s, 1H); IR (ATR) *v.* 3114, 2920, 1676, 1544, 1463, 1355, 1338, 1264, 1187, 1152, 1028, 816, 774, 736, 658 cm⁻¹; HRMS-ESI+ m/z [M+H]⁺ calcd for [C₁₈H₁₂⁷⁹BrN₅OS₂+H⁺]: 457.9739, found: 457.9735.

### Example 23: 2-(imidazo[2,1-b]thiazol-6-yl)-N-[4-(5-nitro-1H-indol-3-yl)thiazol-2-yl]acetamide

From imidazo[2,1-*b*][1,3]thiazol-6-ylacetic acid hydrochloride hydrate (24.5 mg, 0.10 mmol), 4-(5-nitro-1H-indol-3-yl)thiazol-2-amine (26.9 mg, 0.10 mmol), DIPEA (62.8 µL, 0.36 mmol) and HATU (58.7 mg, 0.15 mmol), and following the procedure described in example 1, 2-(imidazo[2,1-*b*]thiazol-6-yl)-*N*-[4-(5-nitro-1H-indol-3-yl)thiazol-2-yl]acetamide (7.7 mg, 18%) was obtained as an orange solid; mp 232-234 °C; ¹H NMR (400 MHz, CDCl₃) *δ:* 3.93 (d, *J =* 0.8 Hz, 2H), 7.12 (d, *J =* 4.4 Hz, 1H), 7.25 (s, 1H), 7.52 (dd, *J =* 9.0 Hz, *J' =* 0.8 Hz, 1H), 7.69 (m, 1H), 7.73 (d, *J =* 4.4 Hz, 1H), 7.88 (s, 1H), 8.09 (dd, *J* = 9.0 Hz, *J'* = 2.4 Hz, 1H), 9.16 (dd, *J =* 2.4 Hz, *J'* = 0.8 Hz, 1H); IR (ATR) *v.* 3317, 3139, 2921, 1702, 1663, 1627, 1574, 1517, 1454, 1324, 1226, 1082, 828, 737, 720, 662, 644 cm⁻¹; HRMS-ESI+ m/z [M+H]⁺ calcd for [C₁₈H₁₂N₆O₃S₂+H⁺]: 425.0485, found 425.0490.

### Example 24: 2-(imidazo[2,1-b]thiazol-6-yl)-N-[4-(5-methyl-1H-indol-3-yl)thiazol-2-yl]acetamide

From imidazo[2,1-*b*][1,3]thiazol-6-ylacetic acid hydrochloride hydrate (48.5 mg, 0.20 mmol), 4-(5-methyl-1*H*-indol-3-yl)thiazol-2-amine (47.0 mg, 0.20 mmol), DIPEA (0.12 mL, 0.69 mmol), HATU (114 mg, 0.30 mmol), and following the procedure described in example 1, 2-(imidazo[2,1-b]thiazol-6-yl)-*N*-[4-(5-methyl-1*H*-indol-3-yl)thiazol-2-yl]acetamide (19.1 mg, 24%) was obtained as a brown solid; mp 160-162 °C; ¹HNMR (400 MHz, MeOH-d₄) *δ:* 2.46 (s, 3H), 3.91 (d, *J=* 0.8 Hz, 2H), 7.00 (dd, *J* = 8.0 Hz, *J'* = 2.0 Hz, 1H), 7.11 (s, 1H), 7.12 (d, *J =* 4.4 Hz, 1H), 7.29 (br d, *J* = 8.0 Hz, 1H), 7.63 (s, 1H), 7.69 (br s, 1H), 7.73 (d, *J* = 4.4 Hz, 1H), 7.82 (m, 1H); IR (ATR) *v.* 3144, 2919, 1676, 1544, 1461, 1272, 1239, 1166, 796, 653, 591 cm⁻¹; HRMS-ESI+ m/z [M+H]⁺ calcd for [C₁₉H₁₅N₅OS₂+H⁺] 394.0791, found 394.0787.

### Example 25: 2-(imidazo[2,1-b]thiazol-6-yl)-N-[4-(5-methoxy-2-methyl-1H-indol-3-yl)thiazol-2-yl]acetamide

From imidazo[2,1-b][1,3]thiazol-6-ylacetic acid hydrochloride hydrate (55 mg, 0.23 mmol), 4-(5-methoxy-2-methyl-1H-indol-3-yl)thiazol-2-amine (60 mg, 0.23 mmol), DIPEA (0.14 mL, 0.80 mmol) and HATU (131 mg, 0.34 mmol), and following the procedure described in example 1, 2-(imidazo[2,1-*b*]thiazol-6-yl)-*N*-[4-(5-methoxy-2-methyl-1*H*-indol-3-yl)thiazol-2-yl]acetamide (21 mg, 22%) was obtained as a beige solid, mp 143-144 °C; ¹H NMR (400 MHz, DMSO-d₆) *δ:* 2.59 (s, 3H), 3.78 (s, 3H), 3.84 (s, 2H), 6.71 (dd, *J* = 8.4 Hz, *J'* = 2.4 Hz, 1H), 7.05 (s, 2H), 7.20 (d, *J =* 8.4 Hz, 1H), 7.21 (d, *J =* 4.8 Hz, 1H), 7.45 (d, *J =* 2.4 Hz, 1H), 7.70 (s, 1H), 7.88 (d, *J* = 4.8 Hz, 1H), 11.03 (s, 1H), 12.23 (s, 1H); IR (ATR) *v.* 3638, 3428, 3148, 2918, 1670, 1627, 1545, 1484, 1461, 1275, 1214, 1142, 1112, 1063, 1030, 831, 726, 662, 555 cm⁻¹; HRMS-ESI+ m/z [M+H]⁺ calcd for [C₂₀H₁₇N₅O₂S₂+H⁺]: 424.0896, found: 424.0902.

### Example 26: N-[4-(6-fluoro-2-methyl-1H-indol-3-yl)thiazol-2-yl]-2-(imidazo[2,1-b]thiazol-6-yl)acetamide

From imidazo[2,1-b][1,3]thiazol-6-ylacetic acid hydrochloride hydrate (62 mg, 0.26 mmol), 4-(6-fluoro-2-methyl-1H-indol-3-yl)thiazol-2-amine hydrobromide (100 mg, 0.30 mmol), DIPEA (0.21 mL, 1.21 mmol) and HATU (150 mg, 0.39 mmol), and following the procedure described in example 1, N-[4-(6-fluoro-2-methyl-1H-indol-3-yl)thiazol-2-yl]-2-(imidazo[2,1-b]thiazol-6-yl)acetamide (37 mg, 35%) was obtained as a beige solid, mp 157-158 °C; ¹H NMR (400 MHz, DMSO-d₆) *δ:* 2.60 (s, 3H), 3.83 (s, 2H), 6.87 (ddd, *J* = 10.0 Hz, *J*' = 8.8 Hz, *J"* = 2.4 Hz, 1H), 7.07 (s, 1H), superimposed in part 7.09 (dd, *J =* 10.0 Hz, *J'* = 2.4 Hz, 1H), 7.21 (d, *J* = 4.4 Hz, 1H), 7.70 (s, 1H), 7.88 (d, *J* = 4.4 Hz, 1H), 7.97 (dd, *J* = 8.8 Hz, *J'* = 6.0 Hz, 1H), 11.27 (s, 1H), 12.28 (s, 1H); IR (ATR) *v*. 3426, 3147, 2918, 1670, 1548, 1487, 1462, 1408, 1342, 1274, 1223, 1137, 1109, 1058, 837, 723, 662, 646, 613, 580, 558 cm⁻¹; HRMS-ESI+ m/z [M+H]⁺ calcd for [C₁₉H₁₄FN₅OS₂+H⁺]: 412.0697, found: 412.0694.

### Example 27: 2-(imidazo[2,1-b]thiazol-6-yl)-N-[4-(2-methyl-5-nitro-1H-indol-3-yl)thiazol-2-yl]acetamide

From imidazo[2,1-b][1,3]thiazol-6-ylacetic acid hydrochloride hydrate (60 mg, 0.25 mmol), 4-(2-methyl-5-nitro-1H-indol-3-yl)thiazol-2-amine hydrobromide (70 mg, 0.20 mmol), DIPEA (0.16 mL, 0.92 mmol) and HATU (145 mg, 0.38 mmol), and following the procedure described in example 1, 2-(imidazo[2,1-b]thiazol-6-yl)-N-[4-(2-methyl-5-nitro-1H-indol-3-yl)thiazol-2-yl]acetamide (36 mg, 41%) was obtained as a yellow solid; mp 150-151 °C; ¹H NMR (400 MHz, DMSO-d₆) *δ:* 2.64 (s, 3H), 3.87 (s, 2H), 7.19 (s, 1H), 7.22 (d, *J =* 4.8 Hz, 1H), 7.50 (d, *J =* 8.8 Hz, 1H), 7.71 (s, 1H), 7.88 (dd, *J =* 4.8 Hz, *J' =* 0.9 Hz, 1H), 7.99 (dd, *J* = 8.8 Hz, *J'* = 2.4 Hz, 1H), 8.94 (d, *J =* 2.4 Hz, 1H), 11.99 (s, 1H), 12.41 (s, 1H); IR (ATR) *v*. 3253, 3146, 3116, 2928, 1673, 1539, 1468, 1452, 1325, 1292, 1254, 1224, 1163, 1063, 898, 847, 741, 669, 642, 555 cm⁻¹; HRMS-ESI+ m/z [M+H]⁺ calcd for [C₁₉H₁₄N₆O₃S₂+H⁺]: 439.0642, found: 439.0649.

### Example 28: N-[4-(5-amino-2-methyl-1H-indol-3-yl)thiazol-2-yl]-2-(imidazo[2,1-b]thiazol-6-yl)acetamide

A solution of 2-(imidazo[2,1-b]thiazol-6-yl)-N-[4-(2-methyl-5-nitro-1H-indol-3-yl)thiazol-2-yl]acetamide (50 mg, 0.11 mmol), Fe (19 mg, 0.34 mmol) and NH₄Cl (30 mg, 0.56 mmol) in a mixture of MeOH (1 mL) and H₂O (0.5 mL) was heated under reflux overnight. The reaction mixture was cooled to rt, filtered through a pad of Celite and washed off with MeOH. The filtrate was evaporated and the residue was purified by automatic column chromatography (CH₂Cl₂:MeOH mixtures), providing *N*-[4-(5-amino-2-methyl-1H-indol-3-yl)thiazol-2-yl]-2-(imidazo[2,1-b]thiazol-6-yl)acetamide (16 mg, 36%) as a brown solid; mp 125-126 °C; ¹H NMR (400 MHz, DMSO-d₆) *δ:* 2.57 (s, 3H), 3.84 (s, 2H), 6.62 (br d, *J =* 8.4 Hz, 1H), 6.95 (s, 1H), 7.12 (d, *J =* 8.4 Hz, 1H), 7.22 (d, *J* = 4.8 Hz, 1H), 7.28 (s, 1H), 7.70 (s, 1H), 7.89 (d, *J =* 4.8 Hz, 1H), 10.94 (s, 1H), 12.27 (s, 1H); IR (ATR) *v*. 3426, 2922, 2851, 1678, 1625, 1544, 1462, 1274, 1132, 1061, 1020, 835, 733, 667, 611, 555 cm⁻¹; HRMS-ESI+ m/z [M+H]⁺ calcd for [C₁₉H₁₆N₆OS₂+H⁺]: 409.0900, found: 409.0896.

### Biological evaluation. Materials and methods

### TET2. Protein Expression and Purification

The human methylcytosine dioxygenase TET2 construct used (1129 - 2002 with residues 1471 - 1843 replaced by a 15-residue GS-linker) was expressed in a pET-28a(+)psumo vector with kanamycin resistance and an N-terminal His6 tag, and cloned in Rosetta D3 *Escherichia coli* competent cells. Transformed cells were cultured overnight in 10 mL of LB media supplied with kanamycin (50 µg/mL), then scaled to 1 L LB medium supplied with kanamycin (50 µg/mL) and grown at 37 °C. Once the optical density (OD600) achieved 0.8, protein expression was induced with 0.4 mM isopropyl-β-thiogalactopyranoside (IPTG) overnight at 18 °C. Cells were harvested by centrifugation (8,000 rpm, 4 °C, 30 min) and the pellet obtained was resuspended in 15 mL of buffer A (50 mM Hepes, 150 mM NaCl, 30 mM imidazole, 2 mM β-mercaptoethanol (pH 8.0) supplemented with Pierce protease inhibitor cocktail (ThermoFisher Scientific)) and lysed by sonication (1 min 40 s: 10 cycles x 10 s ON, 30 s OFF, A=35%, T=19 °C) followed by two consecutive centrifugations (8,000 rpm, 4 °C, 30 min) to clarify the lysate.

The TET2 protein was purified using an ÄKTA Start system (GE Healthcare, Uppsala, Sweden) by two-step purification procedure: one His-affinity chromatography followed by a size exclusion chromatography. First, the protein fraction obtained during the lysis (supernatant) was applied to a 5 mL HisTrap HP column (GE Healthcare), washed with buffer A (50 mM Hepes, 150 mM NaCl, 30 mM imidazole, 2 mM β-mercaptoethanol (pH 8.0)) and the bound protein was eluted with buffer B (50 mM Hepes, 150 mM NaCl, 250 mM imidazole, 2 mM β-mercaptoethanol (pH 8.0)). The eluted protein-containing fractions were pooled and concentrated up to 4 mL for the last purification step: a size exclusion chromatography (HiPrep 16/60 Sephacryl S-100 HR) in 50 mM Hepes, 150 mM NaCl, 2 mM β-mercaptoethanol (pH 8.0). Finally, the mass and purity of the protein were verified by SDS-electrophoresis and mass spectrometry.

### Determination of TET2 binding by surface plasmon resonance

The binding of compounds of the examples was determined by Surface Plasmon Resonance (SPR) using a Biacore T200 SPR biosensor instrument (GE Healthcare, Uppsala, Sweden) at 25 °C. The human TET2 construct was immobilized on a CM5 sensor chip using a standard covalent immobilization via amine coupling following the activation of the carboxymethyl dextran matrix of the sensor chip (injection of a solution containing 0.1 M N-hydroxysuccinimide and 0.4 M 1-ethyl-3-[3-(dimethylamino)propyl]carbodiimide hydrochloride at a flow rate of 15 µL/min for 7 min). The protein immobilization was achieved after the injection of 5 µg/mL TET2 in 10 mM sodium acetate (pH 4.0) at a flow rate of 5 µL/min. Unreacted activated groups of the dextran matrix were deactivated by injection of 1 M ethanolamine hydrochloride for 7 min at a flow rate of 15 µL/min. The corresponding matrix activation and protein immobilization were performed using as a running buffer a phosphate buffered saline solution (1.05x PBS: 10 mM phosphate, pH 7.4, 150 mM NaCl). The screened compounds were prepared in a 20 mM stock solution in DMSO and diluted with 1.05x PBS to achieve a final 5% (v/v) DMSO concentration. The running buffer of the interaction assays consisted of 1xPBS, 5% (v/v) DMSO. The flow rate used for the screening was 60 µL/min and the ligand association and dissociation times set were 60 s and 120 s, respectively.

For data analysis the software used was the Biacore T200 Evaluation software. Different corrections were applied. First, the nonspecific binding to the chip surface and the baseline drift were corrected subtracting the signals of a reference surface (where the immobilization procedure was carried out without proteins) to the signals obtained on the TET2 surface. Second, a series of solvent standards (solvent correction) were introduced to remove the differences derived from the DMSO. Finally, the background was corrected subtracting blank injections (blank subtraction) to the injected compound signals.

The binding affinity was calculated by fitting the data obtained to a single site interaction model by fixing the R max to the R max expected according to the amount of protein immobilized on the chip surface. The steady state values were extracted from the sensograms recorded and plotted against the concentrations assayed. For determining the dissociation constant (*K*_{D}) of the compounds, an initial 2-fold dilution series starting from 75 µM was analysed by duplicate. From this first result, to better calculate the expected K_{D}, an adjustment of the titration series was conducted and analysed. The calculated K_{D} are shown in Table 1 below:

**Table 1**

| Example | *K*_{D} (µM) |
|---|---|
| 1 | 16 |
| 2 | 15 |
| 3 | 16 |
| 4 | 12 |
| 5 | 21 |
| 6 | 5 |
| 7 | 5 |
| 8 | 10 |
| 9 | 9 |
| 10 | 11 |
| 11 | 2 |
| 12 | 6 |
| 13 | 14 |
| 14 | 14 |
| 15 | 11 |
| 16 | 7 |
| 17 | 11 |
| 18 | 5 |
| 19 | 1 |
| 20 | 5 |
| 21 | 7 |
| 22 | 2 |
| 23 | 15 |
| 24 | 1 |
| 25 | 13 |
| 26 | 16 |
| 27 | 2 |
| 28 | 67 |

The results summarised in Table 1 show that the compounds of the invention have strong binding affinity to TET2.

### Determination of TET2 activity in cell

### 1.- HEK293T transfection

The HEK293T cell line was grown in Dulbecco's Modified Eagle Medium (DMEM) containing 10% (v/v) fetal bovine serum (FBS) and 1% (v/v) penicillin-streptomycin (P/S).

Twenty-four hours prior transfection, 3 x 10⁶ HEK293T cells were seeded in p100 dishes previously coated with 0.001% of poly (l-lysine). One hour before transfection, a 10 µM solution of each compound was prepared in complete medium and 9 mL was added by medium replacement in one p100 dish (by duplicate). A solution of complete medium with DMSO was used as a vehicle control. Then, a transfection mix was prepared diluting 8 µg of pCMV6-TET2 expression vector (ID: RC226438, Origene) with 32 µg of polyethylenimine (PEI 25000, Polysciences, Inc.) in a 150 mM NaCl solution per p100 plate. After 15 min of incubation at rt, 1 mL of the transfection mix was added to each p100 plate. Next day, medium was replaced with a fresh diluted compound solution (10 mL/p100 dish). Forty-eight hours after transfection, cells were trypsinized and pelleted. Each pellet was suspended in 500 µL of DNA lysis buffer (50 mM Tris-HCl pH 8, 10 mM EDTA pH 8, 0.1% Triton X-100, 0.5% Tween-20, 400 µg/mL Proteinase K, 50 µg/mL RNAse), and incubated overnight at 56 °C in a thermal mixer. Next day, genomic DNA (gDNA) was extracted from each sample following a standard phenol-chloroform extraction method.

### 2.- 5-Hydroxymethylcytosine (5-hmC) Dot-blot

For each sample, 2 µg of gDNA was diluted in 50 µL of H₂O. Then, 50 µL of 2X Denature solution (0.8 M NaOH, 20 mM EDTA) was added to each sample and incubated at 95 °C for 10 min. Samples were immediately placed on ice to cool and mixed with 100 µL of cold 2X Neutralize buffer (2 M ammonium acetate pH 7). Then, samples were incubated on ice for 10 min. DNA two-fold serial dilutions were prepared by mixing 100 µL of each sample with 100 µL of H₂O in the next well, and so on. A total of 7 serial dilutions were set up. One hundred microliters of each sample and their respective dilutions were spotted on a positively charged nylon membrane (Amersham Hybond-N+, GE healthcare) in an assembled Bio-Dot apparatus (Bio-Rad). Samples were washed adding 400 µL of 0.4 M NaOH in each well. Then, blotted membrane was removed from the Bio-Dot apparatus and washed with 2X SSC buffer (0.3 M NaCl, 30 mM sodium citrate) for 5 min, air dried for 15 min, and UV-crosslinked (energy 120,000 µJ/cm²) for 1 min. Afterwards, the blotted membrane was blocked with Blocking solution (5% non-fat milk, 0.1% Tween-20 in PBS1X) for 1 h at rt. Then, the membrane was incubated with the 5-hmC (5-hydroxymethylcytosine) primary antibody (#39769, Active Motif) diluted 1/10,000 in Blocking solution for 1 h at rt. Next, membrane was washed three times for 5 min in PBS1X and incubated with the anti-rabbit-HRP secondary antibody diluted 1/5,000 in blocking solution for 1 h at rt. Finally, the membrane was washed three times for 5 min in PBS1X, developed with SuperSignal West Pico Chemiluminescent Substrate (ThermoFisher Scientific), and exposed to autoradiography films (Fujifilm).

### 3.- TET2 activity calculation

Each dot-blot membrane allows the evaluation of up to 6 different conditions (in duplicate) at the same time. Among these 6 different conditions there is always the condition of the vehicle and our compound of example 1, so in the end we evaluated 4 new compounds per membrane. The density intensity of each dot was quantified using the Image J software. The dilution condition in which the difference between the density intensities of the different compounds was highest was selected for calculations. Fold-change was calculated in reference to the vehicle condition and corrected with the example 1 condition for each dot-blot membrane.

The calculated fold changes are shown in Table 2 below:

**Table 2**

| Example | Fold change |
|---|---|
| 4 | 9.2 |
| 12 | 16.0 |
| 13 | 11.6 |
| 19 | 8.4 |
| 21 | 15.3 |

The results summarised in Table 2 show a fold change in the amount of 5-hmC illustrating an increase of the activity of TET2 which catalyzes the conversion of 5-methylcytosine into 5-hmC.

### Antiproliferative activity on HL60 cell line

The human leukaemic cell line HL60 was grown in suspension in RPMI 1640 medium containing 10% (v/v) FBS and 1% (v/v) P/S (Life Technologies). EC₅₀ values for all compounds were determined for growth of HL60 over 72 h in duplicate wells in a 96-multiwell plate. Leukaemia cells were suspended at a density of 2 x 10⁵ cells/mL in complete RPMI 1640 medium and 25 µL were seeded in each well (5 x 10³ final total cells/well). A 10 µM solution of the compounds of examples 1, 12 and 21, and a 25 µM solution of all other compounds were prepared in complete medium, and then serially diluted (1:2) (as specified in Table 3 below). A solution of complete medium with DMSO was used as a vehicle control.

**Table 3**

| **Compound** | **Concentrations range (µM)** |
|---|---|
| Ex. 1, Ex. 12, Ex. 21 | 10, 5, 2.5, 1.25, 0.625, 0.31, 0.156, 0.078, 0.039, 0.019, 0.0097 and 0 |
| All other compounds | 25, 12.5, 6.25, 3.125, 1.56, 0.78, 0.39, and 0. |

**Table 3:** Two-fold serial dilution concentrations according to the compound.

Twenty-five microliters of each serially diluted compound solution were added to the corresponding wells of the plate (50 µL of final volume/well). After 48 h of treatment, 50 µL of fresh 2X serially diluted compound solution was added to their corresponding wells (100 µL of final volume/well). After twenty-four additional hours, an equal volume (100 µL) of CellTiter-Glo Reagent (Promega) was added to each well, plates were shaken during 2 min and incubated at rt for 10 min. Luminescence was recorded using a Spark multimode microplate reader and an integration time of 1 s per well.

### Antiproliferative activity on other tumour cell lines

Other human adherent cancer cell lines were cultured in their corresponding cell medium (as specified in Table 4) supplemented with 10% FBS (v/v) and 1% P/S (v/v). Twenty-four hours prior to treatment with the compounds to be tested (Examples 1, 12 and 21), the cells were seeded in 96-well plates at different densities depending of the cell line (as specified in Table 4).

**Table 4**

| **Cell line** | **Medium** | **N° of cells seeded/well** |
|---|---|---|
| A375 (melanoma) | DMEM | 12,000 |
| T98G (glioblastoma) | DMEM | 12,000 |
| LN229 (glioblastoma) | DMEM | 2,000 |
| Hop62 (lung) | RPMI 1640 | 3,000 |
| MCF7 (breast) | DMEM | 8,000 |
| MDA-MD-468 (breast) | RPMI 1640 | 9,000 |
| HCT116 (colon) | RPMI 1640 | 12,000 |

**Table 4:** Cancer cell lines, mediums and number of cells used to calculate EC₅₀.

Depending on the cell line, a 50, 125 or 250 µM solution of the compounds of examples 1, 12 and 21 were prepared in complete medium, and then serially diluted (1:2) (Table 5). A solution of complete medium with DMSO was used as a vehicle control.

**Table 5**

| **Cell line** | **Concentrations range (µM)** |
|---|---|
| A375 | 50, 25, 12.5, 6.25, 3.125, 1.56, 0.78, 0.39, 0.195, 0.0975, 0.048, 0.024, 0.012, 0.006 and 0 |
| HCT116 | 125, 62.5, 31.25, 15.625, 7.8, 3.9, 1.95, 0.97, 0.49, 0.24, 0.12, 0.06, 0.03, 0.015, 0.007, 0.003 and 0 |
| T98G, LN229, Hop62, MCF7 and MDA-MB-468 | 250, 125, 62.5, 31.25, 15.625, 7.8, 3.9, 1.95, 0.97, 0.49, 0.24 and 0 |

**Table 5:** Two-fold serial dilution concentrations according to the cell lines.

Fifty microliters of each serially diluted compound solution was added by medium replacement in two wells (by duplicate). After 48 h of treatment, 50 µL of fresh 2X serially diluted compound solution was added to the corresponding wells of the plate (100 µL of final volume/well). After twenty-four additional hours (a total of 72 h of treatment), an equal volume (100 µL) of CellTiter-Glo Reagent (Promega) was added to each well, plates were shaken during 2 min and incubated at rt for 10 min. Luminescence was recorded using a Spark multimode microplate reader and an integration time of 1 s per well.

The EC₅₀ (in µM) of the tested examples in the HL60 cell line of human leukaemia are summarised in Table 6 below:

**Table 6**

| Example | EC₅₀ (µM) |
|---|---|
| 1 | 0.113 |
| 2 | 4.3 |
| 3 | 1.3 |
| 4 | 2.3 |
| 5 | 12.1 |
| 6 | 3.4 |
| 7 | 9.3 |
| 8 | 0.072 |
| 10 | 3.3 |
| 11 | 2.2 |
| 12 | 0.497 |
| 13 | 1.4 |
| 14 | 13.4 |
| 15 | 20.2 |
| 17 | 4.9 |
| 18 | 7.5 |
| 19 | 0.809 |
| 21 | 0.063 |
| 22 | 0.266 |
| 24 | 0.912 |

The EC₅₀ (in µM) of some of the compounds were also tested on various cancer cell lines to show that the activity is not restricted to a specific hyperproliferative disorder. The results are summarised in Table 7 below

**Table 7**

| | EC₅₀ (µM) | | | | | | | |
|---|---|---|---|---|---|---|---|---|
| Example | HL60 | A375 | LN229 | T98G | Hop62 | MCF7 | MD-MB-468 | HCT 116 |
| 1 | 0.113 | 1.4 | 3.3 | 73.7 | 40.1 | 14.6 | 16.0 | 12.1 |
| 12 | 0.497 | 2.7 | 5.9 | 23.1 | 12.1 | 10.3 | 10.1 | 6.8 |
| 21 | 0.063 | 1.1 | 3.3 | 11.4 | 81.6 | 12.9 | 9.2 | 16.4 |

The results summarised in Tables 6 and 7 show that the compounds of the invention are capable of slowing growth of many different tumoral cell lines.

## Claims

1. A compound of formula (I) 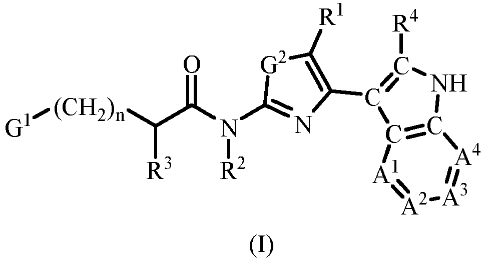 wherein
• n is 0 or 1;
• G¹ is an heteroaromatic ring system comprising a five membered ring attached to the (CH₂)ₙ group, in which the five membered ring is optionally condensed with other rings, wherein said heteroaromatic ring system may comprise 1 to 3 atoms selected from O, S and N in the ring system and said ring system is:
• a) unsubstituted,
• b) C-substituted with 1-3 substituents selected from the group consisting of halogen atoms, C₁₋₄-alkyl, C₁₋₄-alkoxy, C₁₋₄-alkyl-NH-, C₁₋₄-alkyl-S-, C₁₋₄-alkyl-SO-, C₁₋₄-alkyl-SO₂-, C₂₋₄-alkynyl, C₃₋₄-alkynyl-O-, C₃₋₄-alkynyl-NH-, C₃₋₄-alkynyl-S-, -OH, HO-C₁₋₄-alkyl, C₁₋₂-alkyl-O-C₁₋₄-alkyl-, -NH₂, H₂N-C₁₋₄-alkyl, C₁₋₂-alkyl-NH-C₁₋₄-alkyl-, -SH, HS-C₁₋₄-alkyl, C₁₋₂-alkyl-S-C₁₋₄-alkyl-, -CN, NC-C₁₋₄-alkyl, -COOH, HOOC-C₁₋₄-alkyl, -COO-C₁₋₂-alkyl, C₁₋₂-alkyl-OCO-C₁₋₄-alkyl-, -CONH₂, H₂NCO-C₁₋₄-alkyl, -CONH-C₁₋₂-alkyl, C₁₋₂-alkyl-NHCO-C₁₋₄- alkyl-, azido, azido-C₁₋₄-alkyl and -NO₂, or
• c) when the ring system contains nitrogen atoms, said ring system is optionally N-substituted with 1-3 substituents selected from the group consisting of C₁₋₄-alkyl, C₁₋₄-alkyl-SO-, C₁₋₄-alkyl-SO₂-, C₃₋₄-alkynyl, HO-C₂₋₄-alkyl, C₁₋₂-alkyl-O-C₂₋₄-alkyl-, H₂N-C₂₋₄-alkyl, C₁₋₂-alkyl-NH-C₂₋₄-alkyl-, HS-C₂₋₄-alkyl, C₁₋₂-alkyl-S-C₂₋₄-alkyl-, NC-C₁₋₄-alkyl, HOOC-C₁₋₄-alkyl, C₁₋₂-alkyl-OCO-C₁₋₄-alkyl-, H₂NCO-C₁₋₄-alkyl, C₁₋₂-alkyl-NHCO-C₁₋₄-alkyl- and azido-C₂₋₄-alkyl;
wherein when -OH, -NH₂ or -SH substituents are present on the ring system they are not vicinal to a single or double carbon-heteroatom bond;
• G² is selected from the group consisting of O and S;
• R¹ is selected from the group consisting of hydrogen, fluorine and C₁₋₄-alkyl groups
• R² and R³ are independently selected from the group consisting of hydrogen and C₁₋₄-alkyl groups,
• R⁴ is independently selected from the group consisting of hydrogen and methyl
• each one of A¹, A², A³ and A⁴ independently represents any one of N and CR, wherein the ring comprising A¹, A², A³ and A⁴ is aromatic,
• R is selected from the group consisting of hydrogen, halogen atoms, C₁₋₄-alkyl, C₁₋₄-alkoxy, C₁₋₄-alkyl-NH-, C₁₋₄-alkyl-S-, C₁₋₄-alkyl-SO-, C₁₋₄-alkyl-SO₂-, C₂₋₄-alkynyl, C₃₋₄-alkynyl-O-, C₃₋₄-alkynyl-NH-, C₃₋₄-alkynyl-S-, -OH, HO-C₁₋₄-alkyl, C₁₋₂-alkyl-O-C₁₋₄-alkyl-, -NH₂, H₂N-C₁₋₄-alkyl, C₁₋₂-alkyl-NH-C₁₋₄-alkyl-, -SH, HS-C₁₋₄-alkyl, C₁₋₂-alkyl-S-C₁₋₄-alkyl-, -CN, NC-C₁₋₄-alkyl, -COOH, HOOC-C₁₋₄-alkyl, -COO-C₁₋₂-alkyl, C₁₋₂-alkyl-OCO-C₁₋₄-alkyl-, -CONH₂, H₂NCO-C₁₋₄-alkyl, -CONH-C₁₋₂-alkyl, C₁₋₂-alkyl-NHCO-C₁₋₄-alkyl-, azido, azido-C₁₋₄-alkyl and -NO₂,
and pharmaceutically acceptable salts thereof for use in the treatment and/or prevention of hyperproliferative disorders.

2. Compound for use according to claim 1 wherein R¹ is selected from the group consisting of hydrogen and C₁₋₄-alkyl groups.

3. Compound for use according to any one of claims 1 to 2 wherein G¹ is an optionally substituted monocyclic, bicyclic or tricyclic heteroaromatic ring system.

4. Compound for use according to any one of claims 1 to 3 wherein the five-membered ring of G¹ which is attached to the (CH₂)ₙ group contains 1 or 2 heteroatoms selected from N, S and O.

5. Compound for use according to any one of claims 1 to 3 wherein G¹ is selected from the group consisting of 1*H*-indol-3-yl, imidazo[2,1-*b*]thiazol-6-yl, imidazo[1,2-*a*]pyridin-2-yl, 1*H*-indol-2-yl, benzo[*d*]oxazol-2-yl, imidazo[2,1-*b*]thiazol-3-yl, benzo[*b*]thiophen-2-yl, thiophen-2-yl, benzo[*b*]furan-2-yl, 1*H*-imidazol-4-yl, all of which may be optionally substituted as defined in claim 1.

6. Compound for use according to any one of claims 1 to 5 wherein G² is S.

7. Compound for use according to any one of claims 1 to 6 wherein R¹ is selected from hydrogen atom and methyl and R² and R³ are independently selected from hydrogen atom and methyl.

8. Compound for use according to any one of claims 1 to 7 wherein A², A³ and A⁴ are CR.

9. Compound for use according to claim 1 wherein the compound is one of:
• 2-imidazo[2,1-*b*]thiazol-6-yl-*N*-[4-(2-methyl-1*H*-indol-3-yl)thiazol-2-yl]acetamide
• 2-(imidazo[2,1-*b*]thiazol-3-yl)-*N*-[4-(2-methyl-1*H*-indol-3-yl)thiazol-2-yl]acetamide
• 2-(benzo[*d*]oxazol-2-yl)-*N*-[4-(2-methyl-1*H*-indol-3-yl)thiazol-2-yl]acetamide
• 2-(imidazo[2,1-*b*]thiazol-6-yl)-*N*-[5-methyl-4-(2-methyl-1*H*-indol-3-yl)thiazol-2-yl]acetamide
• 2-(imidazo[2,1-*b*]thiazol-6-yl)-*N*-[4-(2-methyl-1*H*-indol-3-yl)oxazol-2-yl]acetamide
• 2-(5-methoxy-1H-indol-3-yl)-N-[4-(2-methyl-1H-indol-3-yl)thiazol-2-yl]acetamide
• 2-(1H-indol-2-yl)-N-[4-(2-methyl-1H-indol-3-yl)thiazol-2-yl]acetamide
• 2-(6-fluoroimidazo[1,2-a]pyridin-2-yl)-N-[4-(2-methyl-1H-indol-3-yl)thiazol-2-yl]acetamide
• N-[4-(2-methyl-1H-indol-3-yl)thiazol-2-yl]-2-(2-methylimidazo[2,1-b]thiazol-6-yl)acetamide
• *N-*[4-(2-methyl-1*H*-indol-3-yl)thiazol-2-yl]-2-(3-methylimidazo[2,1-*b*]thiazol-6-yl)acetamide
• 2-(1-methyl-1*H*-indol-3-yl)-*N*-[4-(2-methyl-1*H*-indol-3-yl)thiazol-2-yl]acetamide
• 2-(5-fluoro-1*H*-indol-3-yl)-*N*-[4-(2-methyl-1*H*-indol-3-yl)thiazol-2-yl]acetamide
• 2-(1*H*-indol-3-yl)-*N*-[4-(2-methyl-1*H*-indol-3-yl)thiazol-2-yl]acetamide
• 3-(1*H*-indol-3-yl)-*N*-[4-(2-methyl-1*H*-indol-3-yl)thiazol-2-yl]propanamide
• 2-(imidazo[2,1-*b*]thiazol-6-yl)-*N*-methyl-*N*-[4-(2-methyl-1*H*-indol-3-yl)thiazol-2-yl]propanamide
• 2-(6-chloro-1*H*-indol-3-yl)-*N*-[4-(2-methyl-1*H*-indol-3-yl)thiazol-2-yl]acetamide
• 2-(1*H*-imidazol-4-yl)-*N*-[4-(2-methyl-1*H*-indol-3-yl)thiazol-2-yl]acetamide
• 2-(benzofuran-2-yl)-*N*-[4-(2-methyl-1*H*-indol-3-yl)thiazol-2-yl]acetamide
• 2-(4-bromothiophen-2-yl)-*N*-[4-(2-methyl-1*H*-indol-3-yl)thiazol-2-yl]acetamide
• *N*-[4-(2-methyl-1*H*-indol-3-yl)thiazol-2-yl]-2-(3-methylbenzo[*b*]thiophen-2-yl)acetamide
• 2-(5-hydroxy-1*H*-indol-3-yl)-*N*-[4-(2-methyl-1*H*-indol-3-yl)thiazol-2-yl]acetamide
• *N*-[4-(4-bromo-1*H*-indol-3-yl)thiazol-2-yl]-2-(imidazo[2,1-*b*]thiazol-6-yl)acetamide
• 2-(imidazo[2,1-*b*]thiazol-6-yl)-*N*-[4-(5-nitro-1*H*-indol-3-yl)thiazol-2-yl]acetamide
• 2-(imidazo[2,1-*b*]thiazol-6-yl)-*N*-[4-(5-methyl-1*H*-indol-3-yl)thiazol-2-yl]acetamide
• 2-(imidazo[2,1-*b*]thiazol-6-yl)*-N*-[4-(5-methoxy-2-methyl-1*H*-indol-3-yl)thiazol-2-yl]acetamide
• *N*-[4-(6-fluoro-2-methyl-1*H*-indol-3-yl)thiazol-2-yl]-2-(imidazo[2,1-*b*]thiazol-6-yl)acetamide
• 2-(imidazo[2,1-*b*]thiazol-6-yl)-*N*-[4-(2-methyl-5-nitro-1*H*-indol-3-yl)thiazol-2-yl]acetamide
• *N*-[4-(5-amino-2-methyl-1*H*-indol-3-yl)thiazol-2-yl]-2-(imidazo[2,1-*b*]thiazol-6-yl)acetamide
• *N*-[5-fluoro-4-(2-methyl-1*H*-indol-3-yl)thiazol-2-yl]-2-(imidazo[2,1-*b*]thiazol-6-yl)acetamide
and pharmaceutically acceptable salts thereof.

10. A compound for use according to any one of claims 1 to 9 wherein the disease is cancer.

11. A compound of formula (I') 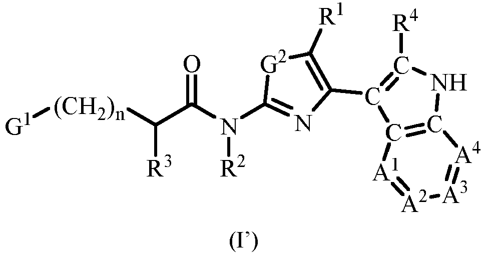 wherein
• n is 0 or 1;
• G¹ is an heteroaromatic ring system selected from the group consisting of benzo[*d*]oxazolyl, imidazo[1,2-*a*]pyridinyl, benzo[*b*]thiophenyl, benzo[*d*]imidazo[2,1-*b*]thiazolyl, 1*H*-imidazol-4-yl, indol-2-yl and benzofuran-2-yl and is said ring system is:
• a) unsubstituted,
• b) C-substituted with 1-3 substituents selected from the group consisting of halogen atoms, C₁₋₄-alkyl, C₁₋₄-alkoxy, C₁₋₄-alkyl-NH-, C₁₋₄-alkyl-S-, C₁₋₄-alkyl-SO-, C₁₋₄-alkyl-SO₂-, C₂₋₄-alkynyl, C₃₋₄-alkynyl-O-, C₃₋₄-alkynyl-NH-, C₃₋₄-alkynyl-S-, -OH, HO-C₁₋₄-alkyl, C₁₋₂-alkyl-O-C₁₋₄-alkyl-, -NH₂, H₂N-C₁₋₄-alkyl, C₁₋₂-alkyl-NH-C₁₋₄-alkyl-, -SH, HS-C₁₋₄-alkyl, C₁₋₂-alkyl-S-C₁₋₄-alkyl-, -CN, NC-C₁₋₄-alkyl, -COOH, HOOC-C₁₋₄-alkyl, -COO-C₁₋₂-alkyl, C₁₋₂-alkyl-OCO-C₁₋₄-alkyl-, -CONH₂, H₂NCO-C₁₋₄-alkyl, -CONH-C₁₋₂-alkyl, C₁₋₂-alkyl-NHCO-C₁₋₄-alkyl-, azido, azido-C₁₋₄-alkyl and -NO₂;
• c) when the ring system contains nitrogen atoms, said ring system is optionally N-substituted with 1 substituent selected from the group consisting of C₁₋₄-alkyl, C₁₋₄-alkyl-SO-, C₁₋₄-alkyl-SO₂-, C₃₋₄-alkynyl, HO-C₂₋₄-alkyl, C₁₋₂-alkyl-O-C₂₋₄-alkyl-, H₂N-C₂₋₄-alkyl, C₁₋₂-alkyl-NH-C₂₋₄-alkyl-, HS-C₂₋₄-alkyl, C₁₋₂-alkyl-S-C₂₋₄-alkyl-, NC-C₁₋₄-alkyl, HOOC-C₁₋₄-alkyl, C₁₋₂-alkyl-OCO-C₁₋₄-alkyl-, H₂NCO-C₁₋₄-alkyl, C₁₋₂-alkyl-NHCO-C₁₋₄-alkyl- and azido-C₂₋₄-alkyl;
wherein when -OH, -NH₂ or -SH substituents are present on the ring system they are not vicinal to a single or double carbon-heteroatom bond;
• G² is selected from the group consisting of O and S;
• R¹ is selected from the group consisting of hydrogen, fluorine and C₁₋₄ alkyl groups
• R² and R³ are independently selected from the group consisting of hydrogen and C₁₋₄ alkyl groups,
• R⁴ is independently selected from the group consisting of hydrogen and methyl
• each one of A¹, A², A³ and A⁴ independently represents any one of N and CR, wherein the ring comprising A¹, A², A³ and A⁴ is aromatic,
• R is selected from the group consisting of hydrogen, halogen atoms, C₁₋₄-alkyl, C₁₋₄-alkoxy, C₁₋₄-alkyl-NH-, C₁₋₄-alkyl-S-, C₁₋₄-alkyl-SO-, C₁₋₄-alkyl-SO₂-, C₂₋₄-alkynyl, C₃₋₄-alkynyl-O-, C₃₋₄-alkynyl-NH-, C₃₋₄-alkynyl-S-, -OH, HO-C₁₋₄-alkyl, C₁₋₂-alkyl-O-C₁₋₄-alkyl-, -NH₂, H₂N-C₁₋₄-alkyl, C₁₋₂-alkyl-NH-C₁₋₄-alkyl-, -SH, HS-C₁₋₄-alkyl, C₁₋₂-alkyl-S-C₁₋₄-alkyl-, -CN, NC-C₁₋₄-alkyl, -COOH, HOOC-C₁₋₄-alkyl, -COO-C₁₋₂-alkyl, C₁₋₂-alkyl-OCO-C₁₋₄-alkyl-, -CONH₂, H₂NCO-C₁₋₄-alkyl, -CONH-C₁₋₂-alkyl, C₁₋₂-alkyl-NHCO-C₁₋₄-alkyl-, azido, azido-C₁₋₄-alkyl and -NO₂,
and pharmaceutically acceptable salts thereof.

12. Compound according to claim 11 wherein R¹ is selected from the group consisting of hydrogen and C₁₋₄ alkyl groups.

13. Compound according to any one claims 11 to 12 wherein G² is S.

14. Compound according to any one of claims 11 to 13 wherein R¹ is selected from hydrogen atom and methyl and R² and R³ are independently selected from hydrogen atom and methyl.

15. Compound according to any one of claims 11 to 14 wherein A², A³ and A⁴ are CR.

16. A compound according to claim 11 which is one of:
• 2-imidazo[2,1-*b*]thiazol-6-yl-*N*-[4-(2-methyl-1*H*-indol-3-yl)thiazol-2-yl]acetamide
• 2-(imidazo[2,1-*b*]thiazol-3-yl)-*N*-[4-(2-methyl-1*H*-indol-3-yl)thiazol-2-yl]acetamide
• 2-(benzo[*d*]oxazol-2-yl)-*N*-[4-(2-methyl-1*H*-indol-3-yl)thiazol-2-yl]acetamide
• 2-(imidazo[2,1-*b*]thiazol-6-yl)-*N*-[5-methyl-4-(2-methyl-1*H*-indol-3-yl)thiazol-2-yl]acetamide
• 2-(imidazo[2,1-*b*]thiazol-6-yl)-*N*-[4-(2-methyl-1*H*-indol-3-yl)oxazol-2-yl]acetamide
• 2-(5-methoxy-1*H*-indol-3-yl)-*N*-[4-(2-methyl-1*H*-indol-3-yl)thiazol-2-yl]acetamide
• 2-(1*H*-indol-2-yl)-*N*-[4-(2-methyl-1*H*-indol-3-yl)thiazol-2-yl acetamide
• 2-(6-fluoroimidazo[1,2-*a*]pyridin-2-yl)-*N*-[4-(2-methyl-1*H*-indol-3-yl)thiazol-2-yl]acetamide
• *N-*[4-(2-methyl-1*H*-indol-3-yl)thiazol-2-yl]-2-(2-methylimidazo[2,1-*b*]thiazol-6-yl)acetamide
• *N-*[4-(2-methyl-1*H*-indol-3-yl)thiazol-2-yl]-2-(3-methylimidazo[2,1-*b*]thiazol-6-yl)acetamide
• 2-(1-methyl-1*H*-indol-3-yl)-*N*-[4-(2-methyl-1*H*-indol-3-yl)thiazol-2-yl]acetamide
• 2-(5-fluoro-1*H*-indol-3-yl)-*N*-[4-(2-methyl-1*H*-indol-3-yl)thiazol-2-yl]acetamide
• 2-(1*H*-indol-3-yl)-*N*-[4-(2-methyl-1*H*-indol-3-yl)thiazol-2-yl]acetamide
• 3-(1*H*-indol-3-yl)-*N*-[4-(2-methyl-1*H*-indol-3-yl)thiazol-2-yl]propanamide
• 2-(imidazo[2,1-*b*]thiazol-6-yl)-*N*-methyl-*N*-[4-(2-methyl-1*H*-indol-3-yl)thiazol-2-yl]propanamide
• 2-(6-chloro-1*H*-indol-3-yl)-*N*-[4-(2-methyl-1*H*-indol-3-yl)thiazol-2-yl]acetamide
• 2-(1*H*-imidazol-4-yl)-*N*-[4-(2-methyl-1*H*-indol-3-yl)thiazol-2-yl]acetamide
• 2-(benzofuran-2-yl)-*N*-[4-(2-methyl-1*H*-indol-3-yl)thiazol-2-yl]acetamide
• 2-(4-bromothiophen-2-yl)-*N*-[4-(2-methyl-1*H*-indol-3-yl)thiazol-2-yl]acetamide
• *N*-[4-(2-methyl-1*H*-indol-3-yl)thiazol-2-yl]-2-(3-methylbenzo[*b*]thiophen-2-yl)acetamide
• 2-(5-hydroxy-1*H*-indol-3-yl)-*N*-[4-(2-methyl-1*H*-indol-3-yl)thiazol-2-yl]acetamide
• *N*-[4-(4-bromo-1*H*-indol-3-yl)thiazol-2-yl]-2-(imidazo[2,1-*b*]thiazol-6-yl)acetamide
• 2-(imidazo[2,1-*b*]thiazol-6-yl)-*N*-[4-(5-nitro-1*H*-indol-3-yl)thiazol-2-yl]acetamide
• 2-(imidazo[2,1-*b*]thiazol-6-yl)-*N*-[4-(5-methyl-1*H*-indol-3-yl)thiazol-2-yl]acetamide
• 2-(imidazo[2,1-*b*]thiazol-6-yl)-*N*-[4-(5-methoxy-2-methyl-1*H*-indol-3-yl)thiazol-2-yl]acetamide
• *N*-[4-(6-fluoro-2-methyl-1*H*-indol-3-yl)thiazol-2-yl]-2-(imidazo[2,1-*b*]thiazol-6-yl)acetamide
• 2-(imidazo[2,1-*b*]thiazol-6-yl)-*N*-[4-(2-methyl-5-nitro-1*H*-indol-3-yl)thiazol-2-yl]acetamide
• *N*-[4-(5-amino-2-methyl-1*H*-indol-3-yl)thiazol-2-yl]-2-(imidazo[2,1-*b*]thiazol-6-yl)acetamide
• *N*-[5-fluoro-4-(2-methyl-1*H*-indol-3-yl)thiazol-2-yl]-2-(imidazo[2,1-*b*]thiazol-6-yl)acetamide
and pharmaceutically acceptable salts thereof.

17. A pharmaceutical composition comprising a compound as defined in any one of claims 11 to 16 and at least one pharmaceutically acceptable excipient.

## Patentansprüche

1. Eine Verbindung der Formel (I) 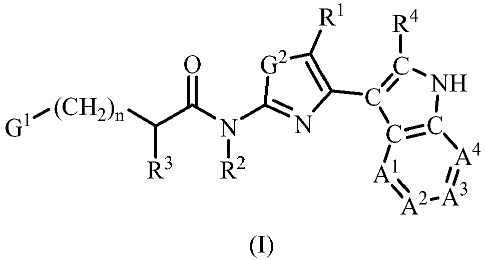 wobei
• n ist 0 oder 1;
• G¹ ist ein heteroaromatisches Ringsystem, das einen an die Gruppe (CH₂)ₙ gebundenen fünfgliedrigen Ring umfasst, wobei der fünfgliedrige Ring gegebenenfalls mit anderen Ringen kondensiert ist, wobei das heteroaromatische Ringsystem 1 bis 3 Atome, ausgewählt aus O, S und N, im Ringsystem umfassen kann und das Ringsystem ist:
• a) unsubstituiert,
• b) C-substituiert mit 1-3 Substituenten, ausgewählt aus der Gruppe bestehend aus Halogenatomen, C₁₋₄-Alkyl, C₁₋₄-Alkoxy, C₁₋₄-Alkyl-NH-, C₁₋₄-Alkyl-S-, C₁₋₄-Alkyl-SO-, C₁₋₄-Alkyl-SO₂ -, C₂₋₄-Alkinyl, C₃₋₄-Alkinyl-O-, C₃₋₄-Alkinyl-NH-, C₃₋₄-Alkinyl-S-, -OH, HO-C₁₋₄-Alkyl, C₁₋₂-Alkyl-O-C₁₋₄-Alkyl-, -NH₂, H₂N-C₁₋₄-Alkyl, C₁₋₂-Alkyl-NH-C₁₋₄-Alkyl-, -SH, HS-C₁₋₄-Alkyl, C₁₋₂-Alkyl-S-C₁₋₄-Alkyl-, -CN, NC-C₁₋₄-Alkyl, -COOH, HOOC-C₁₋₄-Alkyl, -COO-C₁₋₂-Alkyl, C₁₋₂-Alkyl-OCO-C₁₋₄-Alkyl-, -CONH₂-, H₂NCO-C₁₋₄-Alkyl, -CONH-C₁₋₂-Alkyl, C₁₋₂ -Alkyl-NHCO-C₁₋₄ -Alkyl-, Azido, Azido-C₁₋₄ -Alkyl und -NO₂ , oder
• c) wenn das Ringsystem Stickstoffatome enthält, dieses Ringsystem ist optional N-substituiert mit 1-3 Substituenten, ausgewählt aus der Gruppe bestehend aus C₁₋₄-Alkyl, C₁₋₄-Alkyl-SO-, C₁₋₄-Alkyl-SO₂-, C₃₋₄-Alkinyl, HO-C₂₋₄-Alkyl, C₁₋₂-Alkyl-O-C₂₋₄-Alkyl-, H₂N-C₂₋₄-Alkyl, C₁₋₂-Alkyl-NH-C₂₋₄-Alkyl-, HS-C₂₋₄-Alkyl, C₁₋₂-Alkyl-S-C₂₋₄-Alkyl-, NC-C₁₋₄-Alkyl, HOOC-C₁₋₄-Alkyl, C₁₋₂-Alkyl-OCO-C₁₋₄-Alkyl-, H₂NCO-C₁₋₄-Alkyl, C₁₋₂-Alkyl-NHCO-C₁₋₄-Alkyl- und Azido-C₂₋₄-Alkyl;
wobei, wenn -OH-, -NH₂ oder -SH-Substituenten am Ringsystem vorhanden sind, diese nicht in vicinaler Position zu einer Einfach- oder Doppelbindung zwischen Kohlenstoff und einem Heteroatom stehen;
• G² wird aus der Gruppe ausgewählt, bestehend aus O und S;
• R¹ wird aus der Gruppe ausgewählt, bestehend aus Wasserstoff, Fluor und C₁₋₄-Alkylgruppen;
• R² und R³ sind unabhängig voneinander aus der Gruppe ausgewählt, bestehend aus Wasserstoff und C₁₋₄-Alkylgruppen,
• R⁴ wird unabhängig aus der Gruppe ausgewählt, bestehend aus Wasserstoff und Methyl
• jedes der Elemente A¹, A², A³und A⁴ steht unabhängig für N oder CR, wobei der Ring, der aus A¹, A², A³und A⁴ besteht, aromatisch ist,
• R ist ausgewählt aus der Gruppe bestehend aus Wasserstoff, Halogenatomen, C₁₋₄-Alkyl, C₁₋₄-Alkoxy, C₁₋₄-Alkyl-NH-, C₁₋₄-Alkyl-S-, C₁₋₄-Alkyl-SO-, C₁₋₄-Alkyl-SO₂-, C₂₋₄-Alkinyl, C₃₋₄-Alkinyl-O-, C₃₋₄-Alkinyl-NH-, C₃₋₄-Alkinyl-S-, -OH, HO-C₁₋₄-Alkyl, C₁₋₂-Alkyl-O-C₁₋₄-Alkyl-, -NH₂ , H₂N-C₁₋₄-Alkyl, C₁₋₂-Alkyl-NH-C₁₋₄-Alkyl-, -SH, HS-C₁₋₄-Alkyl, C₁₋₂-Alkyl-S-C₁₋₄-Alkyl-, -CN, NC-C₁₋₄-Alkyl, -COOH, HOOC-C₁₋₄-Alkyl, -COO-C₁₋₂-Alkyl, C₁₋₂-Alkyl-OCO-C₁₋₄-Alkyl-, -CONH₂, H₂NCO-C₁₋₄-Alkyl, -CONH-C₁₋₂-Alkyl, C₁₋₂-Alkyl-NHCO-C₁₋₄-Alkyl, Azido, Azido-C₁₋₄-Alkyl und -NO₂ ,
sowie deren pharmazeutisch verträgliche Salze zur Verwendung bei der Behandlung und/oder Vorbeugung von hyperproliferativen Erkrankungen.

2. Verbindung zur Verwendung gemäß Anspruch 1, wobei R¹ aus der Gruppe ausgewählt ist, bestehend aus Wasserstoff und C₁₋₄-Alkylgruppen.

3. Verbindung zur Verwendung gemäß einem der Ansprüche 1 bis 2, wobei G¹ ein gegebenenfalls substituiertes monocyclisches, bicyclisches oder tricyclisches heteroaromatisches Ringsystem ist.

4. Verbindung zur Verwendung gemäß einem der Ansprüche 1 bis 3, wobei der fünfgliedrige Ring von G¹, der an die (CH₂)ₙ-Gruppe gebunden ist, 1 oder 2 Heteroatome enthält, die aus N, S und O ausgewählt sind.

5. Verbindung zur Verwendung gemäß einem der Ansprüche 1 bis 3, wobei G¹ aus der Gruppe ausgewählt ist, bestehend aus 1H-Indol-3-yl, Imidazo[2,1-b]thiazol-6-yl, Imidazo[1,2-a]pyridin-2-yl, 1H-Indol-2-yl, Benzo[d]oxazol-2-yl, Imidazo[2,1-b]thiazol-3-yl, Benzo[b]thiophen-2-yl, Thiophen-2-yl, Benzo[b]furan-2-yl und 1H-Imidazol-4-yl, die alle gegebenenfalls wie in Anspruch 1 definiert substituiert sein können.

6. Verbindung zur Verwendung gemäß einem der Ansprüche 1 bis 5, wobei G² S ist.

7. Verbindung zur Verwendung gemäß einem der Ansprüche 1 bis 6, wobei R¹ aus einem Wasserstoffatom und einer Methylgruppe ausgewählt ist und R² und R³ unabhängig voneinander aus einem Wasserstoffatom und einer Methylgruppe ausgewählt sind.

8. Verbindung zur Verwendung gemäß einem der Ansprüche 1 bis 7, wobei A², A³ und A⁴ CR sind.

9. Verbindung zur Verwendung gemäß Anspruch 1, wobei die Verbindung eine der folgenden ist:
• 2-Imidazo[2,1-*b*]thiazol-6-yl-*N*-[4-(2-methyl-1*H*-indol-3-yl)thiazol-2-yl]acetamid
• 2-(Imidazo[2,1-*b*]thiazol-3-yl)-*N*-[4-(2-methyl-1*H*-indol-3-yl)thiazol-2-yl]acetamid
• 2-(Benzo[*d*]oxazol-2-yl)-*N*-[4-(2-methyl-1*H*-indol-3-yl)thiazol-2-yl]acetamid
• 2-(Imidazo[2,1-*b*]thiazol-6-yl)-*N*-[5-methyl-4-(2-methyl-1*H*-indol-3-yl)thiazol-2-yl]acetamid
• 2-(Imidazo[2,1-*b*]thiazol-6-yl)-*N*-[4-(2-methyl-1*H*-indol-3-yl)oxazol-2-yl]acetamid
• 2-(5-Methoxy-1*H*-indol-3-yl)-*N*-[4-(2-methyl-1*H*-indol-3-yl)thiazol-2-yl]acetamid
• 2-(1*H*-Indol-2-yl)-*N*-[4-(2-methyl-1*H*-indol-3-yl)thiazol-2-yl]acetamid
• 2-(6-Fluorimidazo[1,2-*a*]pyridin-2-yl)-*N*-[4-(2-methyl-1*H*-indol-3-yl)thiazol-2-yl]acetamid
• *N*-[4-(2-Methyl-1*H*-indol-3-yl)thiazol-2-yl]-2-(2-methylimidazo[2,1-*b*]thiazol-6-yl)acetamid
• *N*-[4-(2-Methyl-1*H*-indol-3-yl)thiazol-2-yl]-2-(3-methylimidazo[2,1-*b*]thiazol-6-yl)acetamid
• 2-(1-Methyl-1*H*-indol-3-yl)-*N*-[4-(2-Methyl-1*H*-indol-3-yl)thiazol-2-yl]acetamid
• 2-(5-Fluor-1*H*-indol-3-yl)-*N*-[4-(2-methyl-1*H*-indol-3-yl)thiazol-2-yl]acetamid
• 2-(1*H*-Indol-3-yl)-*N*-[4-(2-methyl-1*H*-indol-3-yl)thiazol-2-yl]acetamid
• 3-(1*H*-Indol-3-yl)-*N*-[4-(2-methyl-1*H*-indol-3-yl)thiazol-2-yl]propanamid
• 2-(Imidazo[2,1-*b*]thiazol-6-yl)-*N*-methyl-*N*-[4-(2-methyl-1*H*-indol-3-yl)thiazol-2-yl]propanamid
• 2-(6-Chlor-1*H*-indol-3-yl)-*N*-[4-(2-methyl-1*H*-indol-3-yl)thiazol-2-yl]acetamid
• 2-(1*H*-Imidazol-4-yl)-*N*-[4-(2-methyl-1*H*-indol-3-yl)thiazol-2-yl]acetamid
• 2-(Benzofuran-2-yl)-*N*-[4-(2-methyl-1H-indol-3-yl)thiazol-2-yl]acetamid
• 2-(4-Bromthiophen-2-yl)-*N*-[4-(2-methyl-1*H*-indol-3-yl)thiazol-2-yl]acetamid
• *N*-[4-(2-Methyl-1*H*-indol-3-yl)thiazol-2-yl]-2-(3-Methylbenzo[*b*]thiophen-2-yl)acetamid
• 2-(5-Hydroxy-1*H*-indol-3-yl)-*N*-[4-(2-methyl-1*H*-indol-3-yl)thiazol-2-yl]acetamid
• *N*-[4-(4-Brom-1*H*-indol-3-yl)thiazol-2-yl]-2-(imidazo[2,1-*b*]thiazol-6-yl)acetamid
• 2-(Imidazo[2,1-*b*]thiazol-6-yl)-*N*-[4-(5-Nitro-1*H*-indol-3-yl)thiazol-2-yl]acetamid
• 2-(Imidazo[2,1-*b*]thiazol-6-yl)-*N*-[4-(5-methyl-1*H*-indol-3-yl)thiazol-2-yl]acetamid
• 2-(Imidazo[2,1-*b*]thiazol-6-yl)-*N*-[4-(5-methoxy-2-methyl-1*H*-indol-3-yl)thiazol-2-yl]acetamid
• *N*-[4-(6-Fluor-2-methyl-1*H*-indol-3-yl)thiazol-2-yl]-2-(imidazo[2,1-*b*]thiazol-6-yl)acetamid
• 2-(Imidazo[2,1-b]thiazol-6-yl)-N-[4-(2-methyl-5-nitro-1*H*-indol-3-yl)thiazol-2-yl]acetamid
• *N*-[4-(5-Amino-2-methyl-1*H*-indol-3-yl)thiazol-2-yl]-2-(imidazo[2,1-*b*]thiazol-6-yl)acetamid
• *N*-[5-Fluor-4-(2-methyl-1*H*-indol-3-yl)thiazol-2-yl]-2-(imidazo[2,1*-b*]thiazol-6-yl)acetamid
sowie pharmazeutisch verträgliche Salze davon.

10. Eine Verbindung zur Verwendung gemäß einem der Ansprüche 1 bis 9, wobei es sich bei der Erkrankung um Krebs handelt.

11. Eine Verbindung der Formel (I') 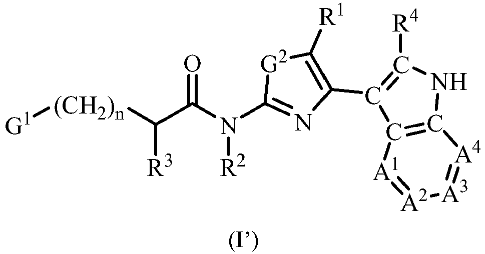 wobei
• n 0 oder 1 ist;
• G¹ ein heteroaromatisches Ringsystem ist, ausgewählt aus der Gruppe bestehend aus Benzo[*d*]oxazolyl, Imidazo[1,2-*a*]pyridinyl, Benzo[*b*]thiophenyl, Benzo[*d*]imidazo[2,1-*b*]thiazolyl, 1*H*-Imidazol-4-yl, Indol-2-yl und Benzofuran-2-yl ausgewählt ist und wobei das Ringsystem ist:
• a) unsubstituiert,
• b) C-substituiert mit 1-3 Substituenten, ausgewählt aus der Gruppe bestehend aus Halogenatomen, C₁₋₄-Alkyl, C₁₋₄-Alkoxy, C₁₋₄-Alkyl-NH-, C₁₋₄-Alkyl-S-, C₁₋₄-Alkyl-SO-, C₁₋₄-Alkyl-SO₂ -, C₂₋₄-Alkinyl, C₃₋₄-Alkinyl-O-, C₃₋₄-Alkinyl-NH-, C₃₋₄-Alkinyl-S-, -OH, HO-C₁₋₄-Alkyl, C₁₋₂-Alkyl-O-C₁₋₄-Alkyl-, -NH₂, H₂N-C₁₋₄-Alkyl, C₁₋₂-Alkyl-NH-C₁₋₄-Alkyl-, -SH, HS-C₁₋₄-Alkyl, C₁₋₂-Alkyl-S-C₁₋₄-Alkyl-, -CN, NC-C₁₋₄-Alkyl, -COOH, HOOC-C₁₋₄-Alkyl, -COO-C₁₋₂-Alkyl, C₁₋₂-Alkyl-OCO-C₁₋₄-Alkyl-, -CONH₂-, H₂NCO-C₁₋₄-Alkyl, -CONH-C₁₋₂-Alkyl, C₁₋₂ -Alkyl-NHCO-C₁₋₄ -Alkyl-, Azido, Azido-C₁₋₄ -Alkyl und -NO₂ ;
• c) wenn das Ringsystem Stickstoffatome enthält, ist dieses Ringsystem optional N-substituiert mit einem Substituenten, der aus der Gruppe ausgewählt ist, bestehend aus C₁₋₄-Alkyl, C₁₋₄-Alkyl-SO-, C₁₋₄-Alkyl-SO₂-, C₃₋₄-Alkinyl, HO-C₂₋₄-Alkyl, C₁₋₂-Alkyl-O-C₂₋₄-Alkyl-, H₂N-C₂₋₄-Alkyl, C₁₋₂-Alkyl-NH-C₂₋₄-Alkyl-, HS-C₂₋₄-Alkyl, C₁₋₂-Alkyl-S-C₂₋₄-Alkyl-, NC-C₁₋₄-Alkyl, HOOC-C₁₋₄-Alkyl, C₁₋₂-Alkyl-OCO-C₁₋₄-Alkyl-, H₂NCO-C₁₋₄-Alkyl, C₁₋₂-Alkyl-NHCO-C₁₋₄-Alkyl- und Azido-C₂₋₄-Alkyl;
wobei, wenn -OH-, -NH₂ oder -SH-Substituenten am Ringsystem vorhanden sind, diese nicht in vicinaler Position zu einer Einfach- oder Doppelbindung zwischen Kohlenstoff und einem Heteroatom stehen;
• G² wird aus der Gruppe ausgewählt, die aus O und S besteht;
• R¹ ist ausgewählt aus der Gruppe bestehend aus Wasserstoff, Fluor und C₁₋₄-Alkylgruppen
• R² und R³ sind unabhängig voneinander aus der Gruppe ausgewählt, bestehend aus Wasserstoff und C₁₋₄-Alkylgruppen,
• R⁴ wird unabhängig aus der Gruppe ausgewählt, bestehend aus Wasserstoff und Methyl,
• jedes der Elemente A¹, A², A³und A⁴ steht unabhängig für N oder CR, wobei der Ring, der aus A¹, A², A³ und A⁴ besteht, aromatisch ist,
• R ist ausgewählt aus der Gruppe bestehend aus Wasserstoff, Halogenatomen, C₁₋₄-Alkyl, C₁₋₄-Alkoxy, C₁₋₄-Alkyl-NH-, C₁₋₄-Alkyl-S-, C₁₋₄-Alkyl-SO-, C₁₋₄-Alkyl-SO₂-, C₂₋₄-Alkinyl, C₃₋₄-Alkinyl-O-, C₃₋₄-Alkinyl-NH-, C₃₋₄-Alkinyl-S-, -OH, HO-C₁₋₄-Alkyl, C₁₋₂-Alkyl-O-C₁₋₄-Alkyl-, -NH₂ , H₂N-C₁₋₄-Alkyl, C₁₋₂-Alkyl-NH-C₁₋₄-Alkyl-, -SH, HS-C₁₋₄ -Alkyl, C₁₋₂-Alkyl-S-C₁₋₄-Alkyl-, -CN, NC-C₁₋₄-Alkyl, -COOH, HOOC-C₁₋₄-Alkyl, -COO-C₁₋₂-Alkyl, C₁₋₂-Alkyl-OCO-C₁₋₄-Alkyl-, -CONH₂, H₂NCO-C₁₋₄-Alkyl, -CONH-C₁₋₂-Alkyl, C₁₋₂-Alkyl-NHCO-C₁₋₄-Alkyl, Azido, Azido-C₁₋₄-Alkyl und -NO₂ ,
sowie pharmazeutisch verträgliche Salze davon.

12. Verbindung nach Anspruch 11, wobei R¹ aus der Gruppe ausgewählt ist, bestehend aus Wasserstoff und C₁₋₄-Alkylgruppen.

13. Verbindung nach einem der Ansprüche 11 bis 12, wobei G² S ist.

14. Verbindung nach einem der Ansprüche 11 bis 13, wobei R¹ aus einem Wasserstoffatom und einer Methylgruppe ausgewählt ist und R² und R³ unabhängig voneinander aus einem Wasserstoffatom und einer Methylgruppe ausgewählt sind.

15. Verbindung nach einem der Ansprüche 11 bis 14, wobei A², A³ und A⁴ CR sind.

16. Verbindung nach Anspruch 11, die eine der folgenden ist:
• 2-Imidazo[2,1-b]thiazol-6-yl-N-[4-(2-methyl-1H-indol-3-yl)thiazol-2-yl]acetamid
• 2-(Imidazo[2,1-b]thiazol-3-yl)-N-[4-(2-methyl-1H-indol-3-yl)thiazol-2-yl]acetamid
• 2-(Benzo[d]oxazol-2-yl)-N-[4-(2-methyl-1H-indol-3-yl)thiazol-2-yl]acetamid
• 2-(Imidazo[2,1-b]thiazol-6-yl)-N-[5-methyl-4-(2-methyl-1*H*-indol-3-yl)thiazol-2-yl]acetamid
• 2-(Imidazo[2,1-b]thiazol-6-yl)-N-[4-(2-methyl-1H-indol-3-yl)oxazol-2-yl]acetamid
• 2-(5-Methoxy-1H-indol-3-yl)-N-[4-(2-methyl-1H-indol-3-yl)thiazol-2-yl]acetamid
• 2-(1H-Indol-2-yl)-N-[4-(2-methyl-1H-indol-3-yl)thiazol-2-yl]acetamid
• 2-(6-Fluorimidazo[1,2-a]pyridin-2-yl)-N-[4-(2-methyl-1H-indol-3-yl)thiazol-2-yl]acetamid
• N-[4-(2-Methyl-1H-indol-3-yl)thiazol-2-yl]-2-(2-methylimidazo[2,1-b]thiazol-6-yl)acetamid
• N-[4-(2-Methyl-1H-indol-3-yl)thiazol-2-yl]-2-(3-methylimidazo[2,1-b]thiazol-6-yl)acetamid
• 2-(1-Methyl-1H-indol-3-yl)-N-[4-(2-Methyl-1H-indol-3-yl)thiazol-2-yl]acetamid
• 2-(5-Fluor-1H-indol-3-yl)-N-[4-(2-methyl-1H-indol-3-yl)thiazol-2-yl]acetamid
• 2-(1H-Indol-3-yl)-N-[4-(2-methyl-1H-indol-3-yl)thiazol-2-yl]acetamid
• 3-(1H-Indol-3-yl)-N-[4-(2-methyl-1H-indol-3-yl)thiazol-2-yl]propanamid
• 2-(Imidazo[2,1-b]thiazol-6-yl)-N-methyl-N-[4-(2-methyl-1H-indol-3-yl)thiazol-2-yl]propanamid
• 2-(6-Chlor-1H-indol-3-yl)-N-[4-(2-methyl-1H-indol-3-yl)thiazol-2-yl]acetamid
• 2-(1H-Imidazol-4-yl)-N-[4-(2-methyl-1H-indol-3-yl)thiazol-2-yl]acetamid
• 2-(Benzofuran-2-yl)-N-[4-(2-methyl-1H-indol-3-yl)thiazol-2-yl]acetamid
• 2-(4-Bromthiophen-2-yl)-N-[4-(2-methyl-1H-indol-3-yl)thiazol-2-yl]acetamid
• N-[4-(2-Methyl-1H-indol-3-yl)thiazol-2-yl]-2-(3-Methylbenzo[b]thiophen-2-yl)acetamid
• 2-(5-Hydroxy-1H-indol-3-yl)-N-[4-(2-methyl-1H-indol-3-yl)thiazol-2-yl]acetamid
• N-[4-(4-Brom-1H-indol-3-yl)thiazol-2-yl]-2-(imidazo[2,1-b]thiazol-6-yl)acetamid
• 2-(Imidazo[2,1-b]thiazol-6-yl)-N-[4-(5-Nitro-1H-indol-3-yl)thiazol-2-yl]acetamid
• 2-(Imidazo[2,1-b]thiazol-6-yl)-N-[4-(5-methyl-1H-indol-3-yl)thiazol-2-yl]acetamid
• 2-(Imidazo[2,1-b]thiazol-6-yl)-N-[4-(5-methoxy-2-methyl-1H-indol-3-yl)thiazol-2-yl]acetamid
• N-[4-(6-Fluor-2-methyl-1H-indol-3-yl)thiazol-2-yl]-2-(imidazo[2,1-b]thiazol-6-yl)acetamid
• 2-(Imidazo[2,1-b]thiazol-6-yl)-N-[4-(2-methyl-5-nitro-1H-indol-3-yl)thiazol-2-yl]acetamid
• N-[4-(5-Amino-2-methyl-1H-indol-3-yl)thiazol-2-yl]-2-(imidazo[2,1-b]thiazol-6-yl)acetamid
• N-[5-Fluor-4-(2-methyl-1H-indol-3-yl)thiazol-2-yl]-2-(imidazo[2,1-b]thiazol-6-yl)acetamid
sowie pharmazeutisch verträgliche Salze davon.

17. Pharmazeutische Zusammensetzung, umfassend eine Verbindung gemäß einem der Ansprüche 11 bis 16 und mindestens einen pharmazeutisch verträglichen Hilfsstoff.

## Revendications

1. Un composé de formule (I) 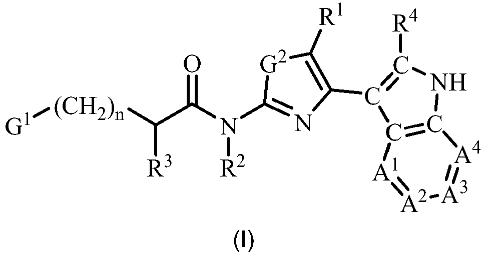 dans lequel
• n vaut 0 ou 1 ;
• G¹ est un système cyclique hétéroaromatique comprenant un cycle à cinq chaînons lié au groupe (CH₂)ₙ, dans lequel le cycle à cinq chaînons est éventuellement condensé avec d'autres cycles, ledit système cyclique hétéroaromatique pouvant comprendre 1 à 3 atomes choisis parmi O, S et N dans le système cyclique et ledit système cyclique est :
• a) non substitué,
• b) C-substitué par 1 à 3 substituants choisis dans le groupe constitué par les atomes d'halogène, un alkyle en C₁₋₄, un alcoxy en C₁₋₄, un alkyle en C₁₋₄-NH-, un alkyle en C₁₋₄-S-, un alkyle en C₁₋₄-SO-, un alkyle en C₁₋₄-SO₂-, un alcynyle en C₂₋₄, un alcynyle en C₃₋₄-O-, un alcynyle en C₃₋₄-NH-, un alcynyle en C₃₋₄-S-, -OH, HO-alkyle en C₁₋₄, un alkyle en C₁₋₂-O-alkyle en C₁₋₄-, -NH₂, H₂N-alkyle en C₁₋₄, un alkyle en C₁₋₂-NH-alkyle en C₁₋₄-, -SH, HS-alkyle en C₁₋₄, un alkyle en C₁₋₂-S-alkyle en C₁₋₄-, -CN, NC-alkyle en C₁₋₄, -COOH, HOOC-alkyle en C₁₋₄, -COO-alkyle en C₁₋₂, un alkyle en C₁₋₂-OCO-alkyle en C₁₋₄-, -CONH₂, H₂NCO-alkyle en C₁₋₄, -CONH-alkyle en C₁₋₂, un alkyle en C₁₋₂-NHCO-alkyle en C₁₋₄-, un azido, un azido-alkyle en C₁₋₄ et -NO₂, ou
• c) lorsque le système cyclique contient des atomes d'azote, ledit système cyclique est éventuellement N-substitué par 1 à 3 substituants choisis dans le groupe constitué par un alkyle en C₁₋₄, un alkyle en C₁₋₄-SO-, un alkyle en C₁₋₄-SO₂-, un alcynyle en C₃₋₄, HO-alkyle en C₂₋₄, un alkyle en C₁₋₂-O-alkyle en C₂₋₄-, H₂N-alkyle en C₂₋₄, un alkyle en C₁₋₂-NH-alkyle en C₂₋₄-, HS-alkyle en C₂₋₄, un alkyle en C₁₋₂-S-alkyle en C₂₋₄-, NC-alkyle en C₁₋₄, HOOC-alkyle en C₁₋₄, un alkyle en C₁₋₂-OCO-alkyle en C₁₋₄-, H₂NCO-alkyle en C₁₋₄, un alkyle en C₁₋₂-NHCO-alkyle en C₁₋₄- et un azido-alkyle en C₂₋₄ ;
dans lequel lorsque des substituants -OH, -NH₂ ou -SH sont présents sur le système cyclique, ils ne sont pas en position vicinale d'une liaison simple ou double carbone-hétéroatome ;
• G² est choisi dans le groupe constitué par O et S ;
• R¹ est choisi dans le groupe constitué par l'hydrogène, le fluor et les groupes alkyle en C₁₋₄
• R² et R³ sont choisis indépendamment dans le groupe constitué par l'hydrogène et les groupes alkyle en C₁₋₄,
• R⁴ est choisi indépendamment dans le groupe constitué par l'hydrogène et le méthyle
• chacun de A¹, A², A³ et A⁴ représente indépendamment l'un quelconque de N et CR, le cycle comprenant A¹, A², A³ et A⁴ étant aromatique,
• R est choisi dans le groupe constitué par l'hydrogène, les atomes d'halogène, un alkyle en C₁₋₄, un alcoxy en C₁₋₄, un alkyle en C₁₋₄-NH-, un alkyle en C₁₋₄-S-, un alkyle en C₁₋₄-SO-, un alkyle en C₁₋₄-SO₂-, un alcynyle en C₂₋₄, un alcynyle en C₃₋₄-O-, un alcynyle en C₃₋₄-NH-, un alcynyle en C₃₋₄-S-, -OH, HO-alkyle en C₁₋₄, un alkyle en C₁₋₂-O-alkyle en C₁₋₄-, -NH₂, H₂N-alkyle en C₁₋₄, un alkyle en C₁₋₂-NH-alkyle en C₁₋₄-, -SH, HS-alkyle en C₁₋₄, un alkyle en C₁₋₂-S-alkyle en C₁₋₄-, -CN, NC-alkyle en C₁₋₄, -COOH, HOOC-alkyle en C₁₋₄, -COO-alkyle en C₁₋₂, un alkyle en C₁₋₂-OCO-alkyle en C₁₋₄-, -CONH₂, H₂NCO-alkyle en C₁₋₄, -CONH-alkyle en C₁₋₂, un alkyle en C₁₋₂-NHCO-alkyle en C₁₋₄-, un azido, un azido-alkyle en C₁₋₄ et -NO₂,
et leurs sels pharmaceutiquement acceptables pour utilisation dans le traitement et/ou la prévention de troubles hyperprolifératifs.

2. Composé pour une utilisation selon la revendication 1 dans lequel R¹ est choisi dans le groupe constitué par l'hydrogène et des groupes alkyle en C₁₋₄.

3. Composé pour une utilisation selon l'une quelconque des revendications 1 à 2 dans lequel G¹ est un système cyclique hétéroaromatique monocyclique, bicyclique ou tricyclique éventuellement substitué.

4. Composé pour une utilisation selon l'une quelconque des revendications 1 à 3, dans lequel le cycle à cinq chaînons de G¹ qui est rattaché au groupe (CH₂)ₙ contient 1 ou 2 hétéroatomes choisis parmi N, S et O.

5. Composé pour une utilisation selon l'une quelconque des revendications 1 à 3 dans lequel G¹ est choisi dans le groupe constitué par le 1*H*-indol-3-yle, l'imidazo[2,1-b]thiazol-6-yle, l'imidazo[1,2-a]pyridin-2-yle, le 1H-indol-2-yle, le benzo[d]oxazol-2-yle, l'imidazo[2,1-b]thiazol-3-yle, le benzo[b]thiophén-2-yle, le thiophén-2-yle, le benzo[b]furan-2-yle, le 1H-imidazol-4-yle, qui peuvent tous être éventuellement substitués comme défini dans la revendication 1.

6. Composé pour une utilisation selon l'une quelconque des revendications 1 à 5 dans lequel G² est S.

7. Composé pour une utilisation selon l'une quelconque des revendications 1 à 6 dans lequel R¹ est choisi parmi un atome d'hydrogène et un méthyle et R² et R³ sont choisis indépendamment parmi un atome d'hydrogène et un méthyle.

8. Composé pour une utilisation selon l'une quelconque des revendications 1 à 7 dans lequel A², A³ et A⁴ sont CR.

9. Composé pour une utilisation selon la revendication 1 dans lequel le composé est l'un de :
• 2-imidazo[2,1-b]thiazol-6-yl-N-[4-(2-méthyl-1H-indol-3-yl)thiazol-2-yl]acétamide
• 2-(imidazo[2,1-b]thiazol-3-yl)-N-[4-(2-méthyl-1H-indol-3-yl)thiazol-2-yl]acétamide
• 2-(benzo[d]oxazol-2-yl)-N-[4-(2-méthyl-1H-indol-3-yl)thiazol-2-yl]acétamide
• 2-(imidazo[2,1-b]thiazol-6-yl)-N-[5-méthyl-4-(2-méthyl-1H-indol-3-yl)thiazol-2-yl]acétamide
• 2-(imidazo[2,1-b]thiazol-6-yl)-N-[4-(2-méthyl-1H-indol-3-yl)oxazol-2-yl]acétamide
• 2-(5-méthoxy-1H-indol-3-yl)-N-[4-(2-méthyl-1H-indol-3-yl)thiazol-2-yl]acétamide
• 2-(1H-indol-2-yl)-N-[4-(2-méthyl-1H-indol-3-yl)thiazol-2-yl]acétamide
• 2-(6-fluoroimidazo[1,2-a]pyridin-2-yl)-N-[4-(2-méthyl-1H-indol-3-yl)thiazol-2-yl]acétamide
• N-[4-(2-méthyl-1H-indol-3-yl)thiazol-2-yl]-2-(2-méthylimidazo[2,1-b]thiazol-6-yl)acétamide
• N-[4-(2-méthyl-1H-indol-3-yl)thiazol-2-yl]-2-(3-méthylimidazo[2,1-b]thiazol-6-yl)acétamide
• 2-(1-méthyl-1H-indol-3-yl)-N-[4-(2-méthyl-1H-indol-3-yl)thiazol-2-yl]acétamide
• 2-(5-fluoro-1H-indol-3-yl)-N-[4-(2-méthyl-1H-indol-3-yl)thiazol-2-yl]acétamide
• 2-(1H-indol-3-yl)-N-[4-(2-méthyl-1H-indol-3-yl)thiazol-2-yl]acétamide
• 3-(1H-indol-3-yl)-N-[4-(2-méthyl-1H-indol-3-yl)thiazol-2-yl]propanamide
• 2-(imidazo[2,1-b]thiazol-6-yl)-N-méthyl-N-[4-(2-méthyl-1H-indol-3-yl)thiazol-2-yl]propanamide
• 2-(6-chloro-1H-indol-3-yl)-N-[4-(2-méthyl-1H-indol-3-yl)thiazol-2-yl]acétamide
• 2-(1H-imidazol-4-yl)-N-[4-(2-méthyl-1H-indol-3-yl)thiazol-2-yl]acétamide
• 2-(benzofuran-2-yl)-N-[4-(2-méthyl-1H-indol-3-yl)thiazol-2-yl]acétamide
• 2-(4-bromothiophén-2-yl)-N-[4-(2-méthyl-1H-indol-3-yl)thiazol-2-yl]acétamide
• N-[4-(2-méthyl-1H-indol-3-yl)thiazol-2-yl]-2-(3-méthylbenzo[b]thiophén-2-yl)acétamide
• 2-(5-hydroxy-1H-indol-3-yl)-N-[4-(2-méthyl-1H-indol-3-yl)thiazol-2-yl]acétamide
• N-[4-(4-bromo-1H-indol-3-yl)thiazol-2-yl]-2-(imidazo[2,1-b]thiazol-6-yl)acétamide
• 2-(imidazo[2,1-b]thiazol-6-yl)-N-[4-(5-nitro-1H-indol-3-yl)thiazol-2-yl]acétamide
• 2-(imidazo[2,1-b]thiazol-6-yl)-N-[4-(5-méthyl-1H-indol-3-yl)thiazol-2-yl]acétamide
• 2-(imidazo[2,1-b]thiazol-6-yl)-N-[4-(5-méthoxy-2-méthyl-1H-indol-3-yl)thiazol-2-yl]acétamide
• N-[4-(6-fluoro-2-méthyl-1H-indol-3-yl)thiazol-2-yl]-2-(imidazo[2,1-b]thiazol-6-yl)acétamide
• 2-(imidazo[2,1-b]thiazol-6-yl)-N-[4-(2-méthyl-5-nitro-1H-indol-3-yl)thiazol-2-yl]acétamide
• N-[4-(5-amino-2-méthyl-1H-indol-3-yl)thiazol-2-yl]-2-(imidazo[2,1-b]thiazol-6-yl)acétamide
• N-[5-fluoro-4-(2-méthyl-1H-indol-3-yl)thiazol-2-yl]-2-(imidazo[2,1-b]thiazol-6-yl)acétamide
et leurs sels pharmaceutiquement acceptables.

10. Composé pour une utilisation selon l'une quelconque des revendications 1 à 9, dans lequel la maladie est un cancer.

11. Composé de formule (I') 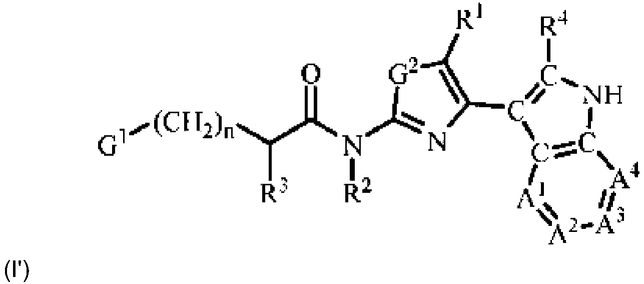 dans lequel
• n vaut 0 ou 1 ;
• G¹ est un système cyclique hétéroaromatique choisi dans le groupe constitué par le benzo[d]oxazolyle, l'imidazo[1,2-a]pyridinyle, le benzo[b]thiophényle, le benzo[d]imidazo[2,1-b]thiazolyle, le 1H-imidazol-4-yle, l'indol-2-yle et le benzofuran-2-yle et ledit système cyclique est :
• a) non substitué,
• b) C-substitué par 1 à 3 substituants choisis dans le groupe constitué par les atomes d'halogène, un alkyle en C₁₋₄, un alcoxy en C₁₋₄, un alkyle en C₁₋₄-NH-, un alkyle en C₁₋₄-S-, un alkyle en C₁₋₄-SO-, un alkyle en C₁₋₄-SO₂-, un alcynyle en C₂₋₄, un alcynyle en C₃₋₄-O-, un alcynyle en C₃₋₄-NH-, un alcynyle en C₃₋₄-S-, -OH, HO-alkyle en C₁₋₄, un alkyle en C₁₋₂-O-alkyle en C₁₋₄-, -NH₂, H₂N-alkyle en C₁₋₄, un alkyle en C₁₋₂-NH-alkyle en C₁₋₄-, -SH, HS-alkyle en C₁₋₄, un alkyle en C₁₋₂-S-alkyle en C₁₋₄-, -CN, NC-alkyle en C₁₋₄, -COOH, HOOC-alkyle en C₁₋₄, -COO-alkyle en C₁₋₂, un alkyle en C₁₋₂-OCO-alkyle en C₁₋₄-, -CONH₂, H₂NCO-alkyle en C₁₋₄, -CONH-alkyle en C₁₋₂, un alkyle en C₁₋₂-NHCO-alkyle en C₁₋₄-, un azido, un azido-alkyle en C₁₋₄ et -NO₂ ;
• c) lorsque le système cyclique contient des atomes d'azote, ledit système cyclique est éventuellement N-substitué par 1 substituant choisi dans le groupe constitué par un alkyle en C₁₋₄, un alkyle en C₁₋₄-SO-, un alkyle en C₁₋₄-SO₂-, un alcynyle en C₃₋₄, HO-alkyle en C₂₋₄, un alkyle en C₁₋₂-O-alkyle en C₂₋₄-, H₂N-alkyle en C₂₋₄, un alkyle en C₁₋₂-NH-alkyle en C₂₋₄-, HS-alkyle en C₂₋₄, un alkyle en C₁₋₂-S-alkyle en C₂₋₄-, NC-alkyle en C₁₋₄, HOOC-alkyle en C₁₋₄, un alkyle en C₁₋₂-OCO-alkyle en C₁₋₄-, H₂NCO-alkyle en C₁₋₄, un alkyle en C₁₋₂-NHCO-alkyle en C₁₋₄- et un azido-alkyle en C₂₋₄ ;
dans lequel lorsque des substituants -OH, -NH₂ ou -SH sont présents sur le système cyclique, ils ne sont pas en position vicinale d'une liaison simple ou double carbone-hétéroatome ;
• G² est choisi dans le groupe constitué par O et S ;
• R¹ est choisi dans le groupe constitué par l'hydrogène, le fluor et les groupes alkyle en C₁₋₄
• R² et R³ sont choisis indépendamment dans le groupe constitué par l'hydrogène et les groupes alkyle en C₁₋₄,
• R⁴ est choisi indépendamment dans le groupe constitué par l'hydrogène et le méthyle
• chacun parmi A¹, A², A³ et A⁴ représente indépendamment l'un quelconque de N et CR, le cycle comprenant A¹, A², A³ et A⁴ étant aromatique,
• R est choisi dans le groupe constitué par l'hydrogène, les atomes d'halogène, un alkyle en C₁₋₄, un alcoxy en C₁₋₄, un alkyle en C₁₋₄-NH-, un alkyle en C₁₋₄-S-, un alkyle en C₁₋₄-SO-, un alkyle en **C₁₋₄-SO₂-,** un alcynyle en C₂₋₄, un alcynyle en C₃₋₄-O-, un alcynyle en C₃₋₄-NH-, un alcynyle en C₃₋₄-S-, -OH, HO-alkyle en C₁₋₄, alkyle en C₁₋₂- O-alkyle en C₁₋₄-, -NH₂, H₂N-alkyle en C₁₋₄, un alkyle en C₁₋₂-NH-alkyle en C₁₋₄-, -SH, HS-alkyle en C₁₋₄, un alkyle en C₁₋₂-S-alkyle en C₁₋₄-, -CN, NC-alkyle en C₁₋₄, -COOH, HOOC-alkyle en C₁₋₄, -COO-alkyle en C₁₋₂, un alkyle en C₁₋₂-OCO-alkyle en C₁₋₄-, -CONH₂, H₂NCO-alkyle en C₁₋₄, -CONH-alkyle en C₁₋₂, un alkyle en C₁₋₂-NHCO-alkyle en C₁₋₄-, un azido, un azido-alkyle en C₁₋₄ et -NO₂,
et leurs sels pharmaceutiquement acceptables.

12. Composé selon la revendication 11 dans lequel R¹ est sélectionné dans le groupe constitué d'hydrogène et de groupes alkyles en C₁₋₄.

13. Composé selon l'une quelconque des revendications 11 à 12 dans lequel G² est S.

14. Composé selon l'une quelconque des revendications 11 à 13 dans lequel R¹ est choisi parmi un atome d'hydrogène et un méthyle et R² et R³ sont indépendamment choisis parmi un atome d'hydrogène et un méthyle.

15. Composé selon l'une quelconque des revendications 11 à 14 dans lequel A², A³ et A⁴ sont CR.

16. Composé selon la revendication 11 qui est l'un parmi :
• 2-imidazo[2,1-*b*]thiazol-6-yl-N-[4-(2-méthyl-1*H*-indol-3-yl)thiazol-2-yl]acétamide
• 2-(imidazo[2,1-*b*]thiazol-3-yl)-N-[4-(2-méthyl-1*H*-indol-3-yl)thiazol-2-yl]acétamide
• 2-(benzo[*d*]oxazol-2-yl)-N-[4-(2-méthyl-1*H*-indol-3-yl)thiazol-2-yl]acétamide
• 2-(imidazo[2,1-*b*]thiazol-6-yl)-N-[5-méthyl-4-(2-méthyl-1*H*-indol-3-yl)thiazol-2-yl]acétamide
• 2-(imidazo[2,1-*b*]thiazol-6-yl)-N-[4-(2-méthyl-1*H*-indol-3-yl)oxazol-2-yl]acétamide
• 2-(5-méthoxy-1*H*-indol-3-yl)-N-[4-(2-méthyl-1*H*-indol-3-yl)thiazol-2-yl]acétamide
• 2-(1*H*-indol-2-yl)-N-[4-(2-méthyl-1*H*-indol-3-yl)thiazol-2-yl]acétamide
• 2-(6-fluoroimidazo[1,2-*a*]pyridin-2-yl)-N-[4-(2-méthyl-1*H*-indol-3-yl)thiazol-2-yl]acétamide
• N-[4-(2-méthyl-1*H*-indol-3-yl)thiazol-2-yl] -2- (2-méthylimidazo[2,1-*b*]thia-zol-6-yl)acétamide
• N-[4-(2-méthyl-1*H*-indol-3-yl)thiazol-2-yl]-2-(3-méthylimidazo[2,1-*b*]thia-zol-6-yl)acétamide
• 2-(1-méthyl-1*H*-indol-3-yl)-N-[4-(2-méthyl-1*H*-indol-3-yl)thiazol-2-yl]acétamide
• 2-(5-fluoro-1*H*-indol-3-yl)-N-[4-(2-méthyl-1*H*-indol-3-yl)thiazol-2-yl]acétamide
• 2-(1*H*-indol-3-yl)-N-[4-(2-méthyl-1*H*-indol-3-yl)thiazol-2-yl]acétamide
• 3-(1*H*-indol-3-yl)-N-[4-(2-méthyl-1*H*-indol-3-yl)thiazol-2-yl]propenamide
• 2-(imidazo[2,1-*b*]thiazol-6-yl)-N-méthyl-N-[4-(2-méthyl-1*H*-indol-3-yl)thi-azol-2-yl]propenamide
• 2-(6-chloro-1*H*-indol-3-yl)-N-[4-(2-méthyl-1*H*-indol-3-yl)thiazol-2-yl]acétamide
• 2-(1*H*-imidazol-4-yl)-N-[4-(2-méthyl-1*H*-indol-3-yl)thiazol-2-yl]acétamide
• 2-(benzofuran-2-yl)-N-[4-(2-méthyl-1*H*-indol-3-yl)thiazol-2-yl]acétamide
• 2-(4-bromothiophen-2-yl)-N-[4-(2-méthyl-1*H*-indol-3-yl)thiazol-2-yl]acétamide
• N-[4-(2-méthyl-1*H*-indol-3-yl)thiazol-2-yl]-2-(3-méthylbenzo[*b*]thiophen-2-yl)acétamide
• 2-(5-hydroxy-1*H*-indol-3-yl)-N-[4-(2-méthyl-1*H*-indol-3-yl)thiazol-2-yl]acétamide
• N-[4-(4-bromo-1*H*-indol-3-yl)thiazol-2-yl]-2-(imidazo[2,1-*b*]thiazol-6-yl)acétamide
• 2-(imidazo[2,1-*b*]thiazol-6-yl)-N-[4-(5-nitro-1*H*-indol-3-yl)thiazol-2-yl]acétamide
• 2-(imidazo[2,1-*b*]thiazol-6-yl)-N-[4-(5-méthyl-1*H*-indol-3-yl)thiazol-2-yl]acétamide
• 2-(imidazo[2,1-*b*]thiazol-6-yl)-N-[4-(5-méthoxy-2-méthyl-1*H*-indol-3-yl) thiazol-2-yl]acétamide
• N-[4-(6-fluoro-2-méthyl-1*H*-indol-3-yl)thiazol-2-yl]-2-(imidazo[2,1-*b*]thi-azol-6-yl)acétamide
• 2-(imidazo[2,1-*b*]thiazol-6-yl)-N-[4-(2-méthyl-5-nitro-1*H*-indol-3-yl)thi-azol-2-yl]acétamide
• N-[4-(5-amino-2-méthyl-1*H*-indol-3-yl)thiazol-2-yl]-2-(imidazo[2,1-*b*]thi-azol-6-yl)acétamide
• N-[5-fluoro-4-(2-méthyl-1*H*-indol-3-yl)thiazol-2-yl]-2-(imidazo[2,1-*b*]thi-azol-6-yl)acétamide
et leurs sels pharmaceutiquement acceptables.

17. Une composition pharmaceutique comprenant un composé tel que défini dans l'une quelconque des revendications 11 à 16 et au moins un excipient pharmaceutiquement acceptable.
